(19) Europäisches Patentamt
European Patent Office
Office européen des brevets

(11) **EP 3 901 272 A1**

(12) **EUROPEAN PATENT APPLICATION**
published in accordance with Art. 153(4) EPC


(43) Date of publication:
**27.10.2021 Bulletin 2021/43**

(21) Application number: **19899302.4**

(22) Date of filing: **17.12.2019**

(51) Int Cl.:
***C12P 19/30*** *(2006.01)* ***C12P 19/32*** *(2006.01)*
***C12P 19/36*** *(2006.01)* ***C12N 1/19*** *(2006.01)*
***C12N 1/21*** *(2006.01)* ***C12N 15/53*** *(2006.01)*
***C12N 15/54*** *(2006.01)* ***C12N 15/56*** *(2006.01)*
***C12N 15/61*** *(2006.01)* ***C12N 15/70*** *(2006.01)*
***C12N 15/81*** *(2006.01)*

(86) International application number:
**PCT/JP2019/049479**

(87) International publication number:
**WO 2020/129997 (25.06.2020 Gazette 2020/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **18.12.2018 JP 2018236634**



(71) Applicants:
- **Teijin Limited**
  **Osaka 530-0005 (JP)**
- **Synart Co., Ltd.**
  **Kobe-shi, Hyogo, 657-8501 (JP)**

(72) Inventors:
- **SHOJI, Shinichiro**
  **Kobe-shi, Hyogo 657-8501 (JP)**
- **ISHII, Jun**
  **Kobe-shi, Hyogo 657-8501 (JP)**
- **KONDO, Akihiko**
  **Kobe-shi, Hyogo 657-8501 (JP)**
- **WATANABE, Hidekazu**
  **Osaka-shi, Osaka 530-0005 (JP)**
- **KANOU, Masanobu**
  **Osaka-shi, Osaka 530-0005 (JP)**
- **NAKAJIMA, Ryota**
  **Osaka-shi, Osaka 530-0005 (JP)**

(74) Representative: **Carpmaels & Ransford LLP**
**One Southampton Row**
**London WC1B 5HA (GB)**


(54) **GENETICALLY MODIFIED MICROORGANISM AND METHOD BOTH FOR PRODUCING NICOTINAMIDE DERIVATIVE, AND VECTOR FOR USE IN SAME**


(57) Provided is a technique for synthesizing a nicotinamide derivative (NAm derivative) such as a nicotinamide mononucleotide (NMN) with high efficiency. A genetically modified microorganism is used, which can express, as nicotinamide phosphoribosylt ransferase (NAMPT), NAMPT having a conversion efficiency of 5-folds or more th at of human NAMPT.


Figure 1

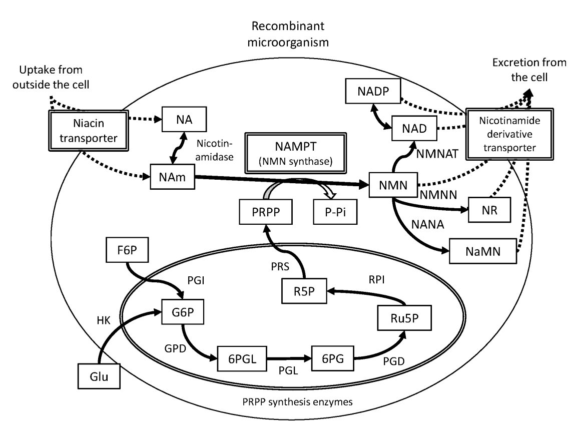


EP 3 901 272 A1

## Description

### TECHNICAL FIELD

The present invention relates to a novel recombinant microorganism for producing a nicotinamide derivative (NAm derivative) such as nicotinamide mononucleotide (NMN) and a novel method for producing a NAm derivative, as well as novel vectors for use in the same.

### BACKGROUND ART

Nicotinamide adenine dinucleotide (NAD) is a nucleotide derived from ribose and nicotinamide. NAD functions as a coenzyme in various redox reactions in vivo and is known to play a central role in aerobic respiration (oxidative phosphorylation). NAD can take two forms in vivo, an oxidized form ($NAD^+$) and a reduced form (NADH). The term "NAD" herein encompasses both the oxidized ($NAD^+$) and reduced (NADH) forms, unless otherwise specified.

There are multiple biosynthetic pathways for NAD, of which the main pathway in mammalian cells is the one that uses nicotinamide (NAm) as a starting material from which NAD is synthesized through a two-step enzymatic reaction. In the first step, NAm taken up into the cell is converted by nicotinamide phosphoribosyl transferase (NAMPT: NMN synthase) in the presence of 5-phosphoribosyl-1 -pyrophosphate (PRPP) into nicotinamide mononucleotide (NMN) and pyrophosphate (P-Pi). In the subsequent second step, NMN obtained in the previous step is converted to NAD by nicotinamide/nicotinate mononucleotide adenylyltransferase (NMNAT) in the presence of adenosine triphosphate (ATP).

NMN, which plays a role as a precursor of NAD in the above biosynthetic pathway, is known to have various functions such as activating mitochondria and sirtuin genes, which are so-called longevity genes. It is considered that especially in vivo, the decrease in NMN production capacity with aging leads to a decrease in NAD, a decrease in mitochondrial activity, and damage to the cell nucleus. NMN has also been reported to involve in aging-related diseases, such as insulin resistance, diabetes, cancer, and Alzheimer's disease. Based on these findings, NMN is attracting attention as a research tool, an intermediate in the synthesis of NAD, and an active pharmaceutical ingredient.

NMN is expected to be used as a synthetic intermediate not only for NAD but also for various nicotinamide derivatives (NAm derivatives), such as nicotinamide riboside (NR) and nicotinate mononucleotide (NaMN).

[Chemical Formula 1]

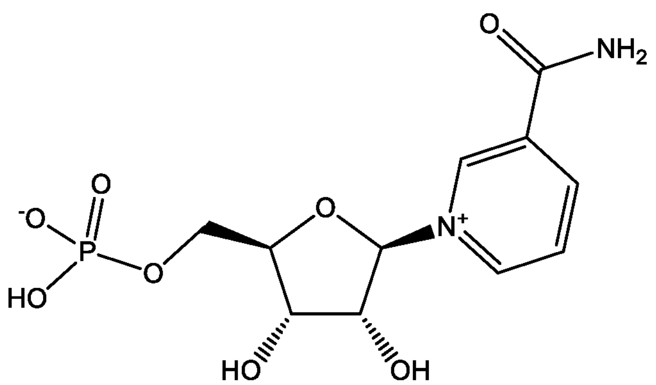

Nicotinamide mononucleotide (NMN)

Conventional methods for the synthesis of NMN include the organic synthesis method, the method based on degradation of NAD, and the synthetic biological method using microorganisms.

According to the organic synthesis method, NMN is synthesized from D-ribose through several steps (Patent Literature 1: US2018/0291054A). This method is time-consuming and costly because it requires several steps of synthesis.

According to the NAD degradation method, NAD biosynthesized by yeast is enzymatically degraded without isolation (Patent Literature 2: WO2017/200050A). This method has a drawback of very poor productivity of NMN per bacterial cell.

According to the synthetic biological method using microorganisms, a host microorganism such as E. coli is genetically engineered to thereby construct a recombinant microorganism that expresses an enzyme similar to biological

enzymes that catalyze the first step in the main biosynthetic system of NAD in mammals, i.e., the step of converting NAm and PRPP to NMN (NAMPT: NMN synthase), and the resulting recombinant microorganism is used for synthesis of NMN (Patent Literature 3: WO2015/069860A; Non-Patent Literature 1: Mariescu et al., Scientific Reports, August 16, 2018, Vol.8, No.1, p.12278). This method cannot achieve sufficient productivity for practical use, since it requires a long time for NMN synthesis but can yield only a small amount of NMN.

## LIST OF CITATIONS

### PATENT LITERATURE

[Patent Literature 1] US2018/0291054A
[Patent Literature 2] WO2017/200050A
[Patent Literature 3] WO2015/069860A

### NON-PATENT LITERATURE

[Non-Patent Literature 1] Mariescu et al., Scientific reports, August 16, 2018, Vol.8, No.1, pp.12278

## SUMMARY OF INVENTION

### PROBLEM TO BE SOLVED BY THE INVENTION

With the above backgrounds, there is a need for efficient synthesis methods of nicotinamide derivatives (NAm derivatives) such as nicotinamide mononucleotide (NMN).

### MEANS TO SOLVE THE PROBLEM

In light of the above issues, the present inventors examined the conventional synthetic biological production system of NMN using microorganisms and, as a result of intensive investigation to improve it, have found that the production efficiency of NMN can be significantly improved by genetically engineering the host microorganism to express a specific enzyme with enhanced activity as a key enzyme (NAMPT) for the biosynthesis of NMN, whereby the production efficiency of NMN can be improved. The present inventors have also found that the production efficiency of NMN can be improved further by genetically engineering the host microorganism to express one or more specific enzymes with enhanced activity as a protein that promotes the uptake of a reactant of NMN synthesis, NAm, or a derivative thereof, NA, into the host microorganism cell (niacin transporter) and/or as a protein that promotes the excretion of NMN or other NAm derivatives out of the host microorganism cell (NAm derivative transporter), whereby the uptake efficiency of NAm into the host microorganism cell can be improved. The present inventors have further found that the production efficiency of NMN can be improved still further by genetically engineering the host microorganism to express one or more specific enzymes with enhanced activity as one or more of a series of enzymes constituting the biosynthetic system of PRPP, another reactant of NMN synthesis, whereby the synthesis efficiency of PRPP can be improved. The present inventors have also found that the thus-improved NMN system can be utilized for producing not only NMN but also other NAm derivatives such as NAD, NR, and NaMN with high efficiency. The present invention has been completed based on these new findings.

The present invention may involve the following aspects.

[1] A recombinant microorganism for producing a nicotinamide derivative, wherein the microorganism has been engineered to express a nicotinamide phosphoribosyl transferase (NAMPT), which converts nicotinamide and phosphoribosyl pyrophosphate into nicotinamide mononucleotide, and/or has been transformed with a vector carrying a nucleic acid encoding the amino acid sequence of the NAMPT, wherein the conversion efficiency of the NAMPT from nicotinamide to nicotinamide mononucleotide is five times or more of the conversion efficiency of a human NAMPT.

[2] The recombinant microorganism according to [1], wherein the NAMPT is composed of a polypeptide with a sequence homology of 80% or more with the amino acid sequence represented by SEQ ID NO:3 or SEQ ID NO:6.

[3] The recombinant microorganism according to [1] or [2], wherein the microorganism has been engineered to express a niacin transporter, which promotes cellular uptake of nicotinic acid and/or nicotinamide, and/or has been transformed with a vector carrying a nucleic acid encoding the amino acid sequence of the niacin transporter, wherein

the niacin transporter increases the intracellular uptake efficiency of nicotinic acid and/or nicotinamide by the host microorganism by 1.1 times or more.

[4] The recombinant microorganism according to [3], wherein the niacin transporter is composed of a polypeptide with a sequence homology of 80% or more with the amino acid sequence represented by SEQ ID NO:9 or SEQ ID NO: 12.

[5] The recombinant microorganism according to any one of [1] to [4], wherein the microorganism has been engineered to express a nicotinamide derivative transporter, which promotes extracellular excretion of a nicotinamide derivative and/or has been transformed with a vector carrying a nucleic acid encoding the amino acid sequence of the nicotinamide derivative transporter, wherein the nicotinamide derivative transporter increases extracellular excretion efficiency of the nicotinamide derivative by the host microorganism by 3 times or more.

[6] The recombinant microorganism according to [5], wherein the nicotinamide derivative transporter is composed of a polypeptide with a sequence homology of 80% or more with the amino acid sequence represented by SEQ ID NO:15.

[7] The recombinant microorganism according to any one of [1] to [6], wherein the microorganism has been engineered to express one or more enzymes which promote a synthetic pathway from glucose-6-phosphoric acid to phosphoribosyl pyrophosphate and/or has been transformed with a vector carrying a nucleic acid encoding the amino acid sequence of the one or more enzymes.

[8] The recombinant microorganism according to [7], wherein the one or more enzymes are selected from the group consisting of phosphoglucose isomerase, glucose-6-phosphate dehydrogenase, 6-phosphogluconolactonase, 6-phosphogluconate dehydrogenase, ribose-5-phosphate isomerase, and phosphoribosyl pyrophosphate synthase.

[9] The recombinant microorganism according to [8], wherein

the phosphoglucose isomerase is a polypeptide with a sequence homology of 80% or more with the amino acid sequence represented by SEQ ID NO:18,
the glucose-6-phosphate dehydrogenase is a polypeptide with a sequence homology of 80% or more with the amino acid sequence represented by SEQ ID NO:21,
the 6-phosphogluconolactonase is a polypeptide with a sequence homology of 80% or more with the amino acid sequence represented by SEQ ID NO:24,
the 6-phosphogluconate dehydrogenase is a polypeptide with a sequence homology of 80% or more with the amino acid sequence represented by SEQ ID NO:27,
the ribose-5-phosphate isomerase is a polypeptide with a sequence homology of 80% or more with the amino acid sequence represented by SEQ ID NO:30 or SEQ ID NO33, and
the phosphoribosyl pyrophosphate synthase is a polypeptide with a sequence homology of 80% or more with the amino acid sequence represented by SEQ ID NO:36.

[10] The recombinant microorganism according to any one of [1] to [9], wherein the nicotinamide derivative is selected from the group consisting of nicotinamide mononucleotide, nicotinamide adenine dinucleotide, nicotinamide riboside, nicotinate mononucleotide, nicotinamide adenine dinucleotide phosphoric acid, and nicotinate adenine dinucleotide.

[11] The recombinant microorganism according to any one of [1] to [10], wherein the recombinant microorganism is E. coli or a yeast.

[12] A method for producing a nicotinamide derivative, comprising:

providing nicotinamide to a recombinant microorganism according to any one of Aspects 1 to 11; and
recovering the nicotinamide derivative produced by the microorganism.

[13] The method according to [12], further comprising purifying the recovered nicotinamide derivative.

[14] A vector carrying a nucleic acid encoding the amino acid sequence of nicotinamide phosphoribosyl transferase (NAMPT), which converts nicotinamide and phosphoribosyl pyrophosphate into nicotinamide mononucleotide, wherein the nucleic acid comprises a base sequence with a sequence identity of 80% or more with the base sequence represented by SEQ ID NO:2 or SEQ ID NO:5.

[15] A vector carrying a nucleic acid encoding the amino acid sequence of a niacin transporter, which promotes cellular uptake of nicotinic acid and/or nicotinamide, wherein the nucleic acid comprises a base sequence with a sequence identity of 80% or more with the base sequence represented by SEQ ID NO:8 or SEQ ID NO:11.

[16] A vector carrying a nucleic acid encoding the amino acid sequence of a nicotinamide derivative transporter, which promotes extracellular excretion of a nicotinamide derivative, wherein the nucleic acid comprises a base sequence with a sequence identity of 80% or more with the base sequence represented by SEQ ID NO:14.

## ADVANTAGEOUS EFFECTS OF INVENTION

The present invention allows for efficient synthesis of NAm derivatives such as NMN.

## BRIEF DESCRIPTION OF DRAWINGS

[Figure 1] Figure 1 is a schematic diagram showing an example of a synthetic biological production system for NAm derivatives.

## DESCRIPTION OF EMBODIMENTS

The present invention will be described in detail in accordance with specific embodiments indicated below. However, the present invention should not be restricted to any of the embodiments disclosed below, but can be implemented in any form to the extent that it does not deviate from the gist of the present invention.

All patent publications, patent application publications, and non-patent documents cited herein are hereby incorporated into this disclosure by reference in their entirety for all purposes.

The term "nucleic acid" used herein encompasses ribonucleic acids, deoxyribonucleic acids, or any modifications of such nucleic acids, and also encompasses both single-stranded and double-stranded nucleic acids. Any nucleic acids (genes) disclosed herein can be prepared by any method known to those skilled in the art, using primers or probes prepared either consulting databases of public organizations known to those skilled in the art or using sequences disclosed herein. Such nucleic acids (genes) can be easily obtained as cDNA of the genes, for example, by using various types of PCR and other DNA amplification techniques known to those skilled in the art. Alternatively, a person skilled in the art can synthesize a nucleic acid using any conventional technique as appropriate based on the sequence information disclosed herein.

The statement that any nucleic acid or gene "encodes" a protein or polypeptide herein means that the nucleic acid or gene expresses the protein or polypeptide with maintaining its activities. The term "encode" herein means encoding a protein disclosed herein either as a continuous structural sequence (exon) or in the form of two or more discrete segments with one or more appropriate intervening sequences (introns).

Gene engineering techniques mentioned herein, such as cloning of nucleic acids or genes, design and preparation of vectors, transformation of cells, and expression of proteins or polypeptides, can be carried out with reference to, e.g., Sambrook, J. et al, Molecular Cloning: A Laboratory Manual, 2nd Ed. Manual, 2nd Ed., Cold Spring Harbor Laboratory Press, Cold Spring Harbor, N.Y., 1989.

### [I. Overview]

This chapter provides an overview of the present invention.

As mentioned above, in an attempt to produce nicotinamide mononucleotide (NMN) via synthetic biological using microorganisms, a method has been proposed which involves constructing a recombinant microorganism expressing enzymes similar to those constituting the main biosynthetic system of nicotinamide adenine dinucleotide (NAD) in mammals by genetically engineering a host microorganism such as E. coli, and using the resulting recombinant microorganism to synthesize NMN (Patent Literature 3: WO2015/069860A; Non-Patent Literature 1: Mariescu et al., Scientific reports, August 16, 2018, Vol.8, No.1, pp.12278). However, this conventional method has a drawback in that it cannot achieve sufficient productivity for practical use, since it requires a long time for NMN synthesis but can yield only a small amount of NMN.

The present invention relates to a NAm derivative synthesis system derived from the conventional synthetic biological production system of NMN using microorganisms, with improved production efficiency of NAm derivatives such as NMN, by genetically engineering a microorganism to express various enzymes and/or transporter proteins involved in the synthesis and/or transport of NAm derivatives.

An exemplary synthetic biological production system for NAm derivatives according to an embodiment of the present invention will be described in more detail using Figure 1. Note that Figure 1 merely illustrates an example, while the present invention should not be limited to the synthesis system shown in Figure 1.

The synthetic biological system of NAm-derivatives shown in Figure 1 includes a NAm-derivative synthesis system consisting of a series of enzymes in a host microorganism cell. As shown in Figure 1, the main reaction pathway of the NMN synthesis system is the reaction pathway that converts NAm and PRPP into NMN and P-Pi.

NAm, one of the two reactants of the main reaction pathway of the NAm derivative synthesis system, is taken into the host microorganism cell from outside, and is interconvertible by nicotinamidase with NAm, which is also taken into the host microorganism cell from outside.

PRPP, the other reactant of the main reaction pathway of the NAm derivative synthesis system, is synthesized

from glucose (Glu) taken into the host microorganism cell from outside, through the following series of reaction steps.

- Phosphorylation of glucose (Glu) to glucose-6-phosphate (G6P) by hexokinase (HK).
- Conversion of fructose-6-phosphate (F6P) to glucose-6-phosphate (G6P) by phosphoglucose isomerase (PGI).
- Conversion of G6P to 6-phosphoglucono-1,5-lactone (6PGL) by glucose-6-phosphate dehydrogenase (GPD).
- Conversion of 6PGL to 6-phosphogluconate (6PG) by 6-phosphogluconolactonase (PGL).
- Conversion of 6PG to ribulose-5-phosphate (Ru5P) by 6-phosphogluconate dehydrogenase (PGD).
- Conversion of Ru5P to ribose-5-phosphate (R5P) by ribose-5-phosphate isomerase (RPI).
- Conversion of R5P to 5-phosphoribosyl-1-pyrophosphate (PRPP) by phosphoribosyl pyrophosphate synthase (PRS).

The NMN produced by the main reaction mentioned above is then converted to NAD by NMNAT, to nicotinate mononucleotide (NaMN) by nicotinamide nucleotide amidase (NANA), and to nicotinamide riboside by nicotinamide mononucleotide-5-nucleotidase (NMNN), which are then excreted from the cell as appropriate.

According to an aspect of the present invention, a host microorganism is genetically engineered to express a specific enzyme with improved activity as a key enzyme for the biosynthesis of NMN (NAMPT: NMN synthase) to enhance the efficiency of NMN synthesis, thereby improving the production efficiency of NAm derivatives.

According to a preferred aspect of the present invention, the NAm derivative synthesis system mentioned above is modified by genetically engineering the host microorganism to express a specific protein with improved activity as a transporter protein (niacin transporter) that promotes the intracellular uptake of NAm and/or NA (niacin) to enhance the efficiency of niacin uptake into the host microorganism cell, thereby further improving the production efficiency of NAm derivatives.

According to another preferred aspect of the present invention, the NAm derivative synthesis system mentioned above is modified by genetically engineering the host microorganism to express a specific enzyme with improved activity as one or more of the series of enzymes (GPI, GPD, PGL, PGD, RPI, and PRS) that constitute the biosynthetic system of PRPP, another reactant of NMN synthesis, to enhance the efficiency of PRPP synthesis, thereby further improving the production efficiency of NAm derivatives.

According to another preferred aspect of the present invention, the NAm derivative synthesis system mentioned above is modified by genetically engineering the host microorganism to express a specific protein with improved activity as a transporter protein (NAm derivative transporter) that promotes the extracellular excretion of NAm derivatives to enhance the efficiency of excretion of the produced NAm derivatives from host microorganism cell, thereby further improving the production efficiency of NAm derivatives.

According to the present invention, the overall production efficiency of the NAm derivative can be significantly improved by introducing a combination of two or more, preferably all, of the specific NAMPT (NMN synthase), niacin transporter, NAm derivative transporter, and PRPP synthesis enzymes (GPI, GPD, PGL, PGD, RPI, and PRS) into the NAm derivative synthesis system of the recombinant microorganism.

NAm derivatives that can be produced by the synthetic system of the present invention aside from NMN include, but are not limited to, NAD, nicotinamide riboside (NR), nicotinate mononucleotide (NaMN), nicotinamide adenine dinucleotide phosphate (NADP), nicotinate adenine dinucleotide, etc.

While the next and subsequent chapters will be given mainly to an embodiment of the synthetic system of the present invention for synthesizing NMN, other embodiments relating to the synthesis of NAm derivatives other than NMN are briefly described below.

For example, synthesis of NAD by the synthetic system of the present invention can be achieved by genetically engineering the host microorganism to express, in addition to the series of genes for NMN synthesis, nicotinamide/nicotinic acid mononucleotide adenylyltransferase (NMNAT), which converts NMN to NAD, according to the same procedure as described above to enhance the conversion efficiency of NMN to NAD.

Synthesis of NADP by the synthetic system of the present invention can be achieved by genetically engineering the host microorganism to express, in addition to the series of genes for NMN synthesis, the NMNAT mentioned above along with a NAD+ kinase, which converts NAD to NADP, according to the same procedure as described above to enhance the conversion efficiency of NMN to NAD and NAD to NADP.

Synthesis of NR by the synthetic system of the present invention can be achieved by genetically engineering the host microorganism to express, in addition to the series of genes for NMN synthesis, nicotinamide mononucleotide-5-nucleotidase (NMNN), which further converts NMN to NR, according to the same procedure as described above to enhance the conversion efficiency of NMN to NR.

Synthesis of NaMN by the synthetic system can also be achieved by genetically engineering the host microorganism to express, in addition to the series of genes for NMN synthesis, nicotinamide nucleotide amidase (NANA), which further converts NMN to NaMN, according to the same procedure as described above to enhance the conversion efficiency of NMN to NaMN.

According to the method using the NAm derivative synthesis system of the present invention with the constitution described above, the production efficiency of NAm derivatives such as NMN can be significantly improved, compared to the conventional synthetic biological production method of NMN. For example, the results shown in the Examples below show that the method using the NAm derivative synthesis system of the present invention can produce more than 10 times the amount of NMN compared to the amount of NMN produced by the conventional synthetic biological methods. The method using the NAm derivative synthesis system of the present invention is also advantageous in that it involves reduced amounts of by-products and has excellent selectivity for NMN and other NAm derivatives.

## [II. Enzymes]

This chapter deals with the enzymes involved in the production of NAm derivatives used in the present invention.
The term "homology" between two amino acid sequences herein means the ratio of identical or similar amino acid residues appearing in each corresponding position when these amino acid sequences are aligned, and the term "identity" between two amino acid sequences herein means the ratio of identical amino acid residues appearing in each corresponding position when these amino acid sequences are aligned.
The "homology" and "identity" between two amino acid sequences can be determined, e.g., with the Needle program of the EMBOSS package (EMBOSS: The European Molecular Biology Open Software Suite, Rice et al., 2000, Trends Genet. 16: 276-277) (preferably version 5.00 or later), using the Needleman-Wunsch algorithm (Needleman and Wunsch, 1970, J. Mol. Biol. 48: 443-453).
Similar amino acids herein include, e.g., amino acids that belong to the same group in the classification based on structures, characteristics, and types of side chains indicated below.

| | |
|---|---|
| - Aromatic amino acids: | F, H, W, Y; |
| - Aliphatic amino acids: | I, L, V; |
| - Hydrophobic amino acids: | A, C, F, H, I, K, L, M, T, V, W, Y; |
| - Charged amino acids: | D, E, H, K, R, etc.; |
| - Positively charged amino acids: | H, K, R; |
| - Negatively charged amino acids: | D, E;. |
| - Polar amino acids: | C, D, E, H, K, N, Q, R, S, T, W, Y; |
| - Small amino acids: | A, C, D, G, N, P, S, T, V, etc.; |
| - Very small amino acids: | A, C, G, S; |
| - Amino acids with aliphatic side chains: | G, A, V, L, I; |
| - Amino acids with aromatic side chains: | F, Y, W; |
| - Amino acids with sulphur-containing side chains: | C, M; |
| - Amino acids with aliphatic hydroxyl side chains: | S, T; |
| - Amino acids with basic side chains: | K, R, H; and |
| - Acidic amino acids and their amide derivatives: | D, E, N, Q. |

### (1) Enzyme that catalyzes the synthesis of NMN from NAm and PRPP (NMN synthase):

Various enzymes derived from various microorganisms have been known as NMN synthases that catalyze the synthesis of NMN from NAm and PRPP (NAMPTs). Such known enzymes can be selectively used as appropriate.
According to the present invention, a specific enzyme with improved activity may preferably be used as NMN synthase (NAMPT). One reason is that an NAMPT with poor activity may decrease the selectivity of NMN production from the substrates, i.e., NAm and PRPP. Another reason is that an NAMPT with poor activity may require a longer time for producing NMN and may lead to a decrease in the production rate due to the degradation and conversion of NMN. Such an NMN synthase with improved activity may be referred to herein as "the NMN synthase of the present invention," although NMN synthases (NAMPT) that can be used in the present invention are not limited to the one explained below.
Specifically, the NMN synthase (NAMPT) of the present invention may preferably have a conversion efficiency of NAm to NMN (NAMPT conversion efficiency) that is typically 5-fold or higher, particularly 7-fold or higher, more particularly 9-fold or higher, compared to the NAMPT conversion efficiency of human NAMPT. As shown in the [EXAMPLES] section below, according to the inventors' investigation, no production of NMN was observed in a bacterium expressing NAMPT with 2-fold or 3-fold conversion efficiency as well as in a bacterium expressing human NAMPT, while a significant increase in NMN production was observed in a bacterium expressing NAMPT with 6-fold conversion efficiency compared to the bacterium expressing human NAMPT.
A specific example of a method for measuring the NAMPT conversion efficiency of NAMPT is as follows:

Escherichia coli (hereinafter referred as E. coli) BL21 (DE3) strain is transformed with a plasmid derived from pRSFDuet-1 via incorporation of a gene for expressing NAMPT to prepare a construct strain, which is then inoculated into a test tube containing 5 mL of LB medium and cultured at 37°C at 200 rpm for 12 hours. The culture is then inoculated into a 500 ml conical flask containing 200 ml of LB medium such that the resulting $OD_{600}$ is 0.03, and incubated at 37°C at 200 rpm. When $OD_{600}$ reached 0.4, isopropyl-β-thiogalactopyranoside is added such that the final concentration becomes 0.1 mM, and the culture is further incubated at 25°C at 200 rpm for 16 hours. 30 mL of the culture medium is then transferred to a 50 mL conical tube, centrifuged at 3000g for 5 minutes, and the bacterial cells are collected. The cells are washed with 1×PBS, the wash fluid is centrifuged at 3000g for 5 minutes, and the bacterial cells are collected. This operation is repeated twice. The recovered bacterial cells are suspended in 15 mL of Cell Lysis Buffer (MBL) to prepare a bacterial lysate solution (lysate) according to a generally recommended method. The bacterial lysate is measured for $OD_{595}$ using Protein Assay Bradford Reagent (Wako Pure Chemical Co., Ltd.), and then diluted with water such that the $OD_{595}$ value becomes 0.1. The resulting diluted solution is used as NAMPT solution and subjected to the measurement of NAMPT activity according to the One-Step Assay Method of CycLexR NAMPT Colorimetric Assay Kit Ver. 2 (MBL). The measurement can be carried out using SpectraMaxR iD3 multimode microplate reader (Molecular Devices Inc.) or other instruments. According to the present invention, the absorbance at 450 nm is measured every 5 minutes at 30°C for up to 60 minutes, the absorbance values at three points where the slope is at its maximum value are selected, and their slope is defined as the NAMPT conversion efficiency.

However, the measurement method of NAMPT conversion efficiency is not limited to the specific example explained above, but may be any other evaluation method so long as it gives equivalent values.

Among them, the NMN synthase of the present invention may preferably be an enzyme comprising a polypeptide having the amino acid sequence represented by SEQ ID NO:3 or SEQ ID NO:6, or a polypeptide having a similar amino acid sequence thereto.

The amino acid sequence of an NAMPT from Sphingopyxis sp. C-I strain is shown in SEQ ID NO:3, and the nucleotide sequence of the naturally-occurring gene encoding the NAMPT of SEQ ID NO:3 is shown in SEQ ID NO:1. The present inventors optimized the nucleotide sequence of the naturally-occurring gene of SEQ ID NO:1 so as to improve its expression and activity in the host microorganism. The resulting optimized nucleotide sequence encoding the NAMPT of SEQ ID NO:3 is shown in SEQ ID NO:2.

The amino acid sequence of an NAMPT from Chitinophaga pinensis is shown in SEQ ID NO:6, and the nucleotide sequence of the naturally-occurring gene encoding the NAMPT of SEQ ID NO:6 is shown in SEQ ID NO:4. The present inventors optimized the nucleotide sequence of the naturally-occurring gene of SEQ ID NO:4 so as to improve its expression and activity in the host microorganism. The resulting optimized nucleotide sequence encoding the NAMPT of SEQ ID NO:6 is shown in SEQ ID NO:5.

Specifically, the NMN synthase of the present invention may preferably have a polypeptide with an amino acid sequence with a homology (preferably identity) of 80% or more, particularly 85% or more, still particularly 90% or more, even still particularly 95% or more, or 96% or more, or 97% or more, or 99% or more, especially 100%, to the amino acid sequence shown in SEQ ID NO:3 or SEQ ID NO:6.

**(2) Transporter protein that promotes the intracellular uptake of niacin (niacin transporter)**:

Various transporter proteins derived from various microorganisms have been known as transporters that promote the intracellular uptake of NAm and/or NA (niacin) (niacin transporters). Such known transporter proteins can be selectively used as appropriate.

According to the present invention, a specific transporter protein with improved activity may preferably be used as a niacin transporter. One reason is that a niacin transporter with poor niacin uptake efficiency may lead to a low intracellular abundance of NAm that reacts with PRPP, thus resulting in a poor selectivity of NMN production from the substrates NAm and PRPP. Another reason is that a niacin transporter with poor niacin uptake efficiency may require a longer time for producing NMN and may lead to a decrease in the production rate due to the degradation and conversion of NMN. Such a transporter protein with improved niacin uptake efficiency may be referred to herein as "the niacin transporter of the present invention," although niacin transporters that can be used in the present invention are not limited to the one explained below.

Specifically, the niacin transporter of the present invention may preferably increase the efficiency of intracellular uptake of nicotinic acid and/or nicotinamide (niacin uptake efficiency) by the host microorganism typically by 1.1-fold or more, particularly by 1.2-fold or more, compared to the niacin uptake efficiency of the host microorganism that does not express the niacin transporter of the present invention. Use of such a niacin transporter with an improved niacin uptake efficiency results in a higher intracellular abundance of NAm in response to its concentration compared to the host microorganism that does not express the niacin transporter of the present invention, resulting in an increase in NMN

production.

A specific example of a method for measuring the niacin uptake efficiency of a niacin transporter is as follows: Escherichia coli (E. Coli) BL21 (DE3) strain is genetically engineered to express NAMPT with a NAMPT conversion efficiency of 200 or higher, and the resulting strain is then transformed with a plasmid derived from pCDFDuet-1 by inserting E. coli (E. Coli) K12-derived genes prs, rpiB, rpiA, gnd, pgl, zwf, and pgi so as to express these genes in this order. The resultant transformant strain is further transformed with another plasmid derived pACYCDuet-1 via incorporation of a gene encoding the niacin transporter to prepare a construct strain, which is then inoculated into a test tube containing 5 mL of LB medium and cultured at 37°C at 200 rpm for 12 hours. The culture is then inoculated into a 500 ml conical flask containing 200 ml of LB medium such that the resulting $OD_{600}$ is 0.03, and incubated at 37°C at 200 rpm. When $OD_{600}$ reached 0.4, isopropyl-β-thiogalactopyranoside is added such that the final concentration becomes 0.1 mM, and the culture is further incubated at 25°C at 200 rpm for 16 hours. The culture medium is then transferred to a 50 mL conical tube, centrifuged at 3000g for 5 minutes, and the bacterial cells are collected. The cells are washed with 1×PBS, the wash fluid is centrifuged at 3000g for 5 minutes, and the bacterial cells are collected. This operation is repeated twice. The collected bacterial cells are suspended in LB medium to obtain an $OD_{600}$ of 10, 10 mL of which suspension is transferred to a 100-mL conical flask, and combined with nicotinamide at 1 g/L, $_D$-glucose at 0.4 g/L, and phosphate buffer (pH 6.2) at 0.005 mol/L, and the reaction is allowed to run at 30°C at 200 rpm. The reaction solution is collected 1 hour and 2 hours from the start of the reaction. The collected solutions are frozen at -30°C, thawed, and centrifuged at 12,000 rpm for 3 minutes to collect the supernatant. These liquid samples are analyzed by HPLC to quantify the amounts of NMN. The obtained NMN quantitative values is used for determining the niacin uptake efficiency of niacin transporter according to Equation (1) below.

[Formula 1]

$$\text{Niacin uptake efficiency of the niacin transporter (\%)} = \{(\text{NMN amount after 1 hour of reaction})/(\text{NMN amount after 2 hour of reaction})\} \times 100 \quad \text{Equation (1)}$$

However, the measurement method of the niacin uptake efficiency of a niacin transporter is not limited to the specific example explained above, but may be any other evaluation method so long as it gives equivalent values.

Among them, the niacin transporter of the present invention may preferably be a protein comprising a polypeptide having the amino acid sequence represented by SEQ ID NO:9 or SEQ ID NO:12, or a polypeptide having a similar amino acid sequence thereto.

The amino acid sequence of the niacin transporter from Burkholderia cenocepacia is shown in SEQ ID NO:9, and the nucleotide sequence of the naturally-occurring gene encoding the niacin transporter of SEQ ID NO:9 is shown in SEQ ID NO:7. The present inventors optimized the nucleotide sequence of the naturally-occurring gene of SEQ ID NO:7 so as to improve its expression and activity in the host microorganism. The resulting optimized nucleotide sequence encoding the niacin transporter of SEQ ID NO:9 is shown in SEQ ID NO:8.

The amino acid sequence of the niacin transporter from Streptococcus pneumoniae is shown in SEQ ID NO: 12, and the nucleotide sequence of the naturally-occurring gene encoding the niacin transporter of SEQ ID NO:12 is shown in SEQ ID NO:10. The present inventors optimized the nucleotide sequence of the naturally-occurring gene of SEQ ID NO:10 so as to improve its expression and activity in the host microorganism. The resulting optimized nucleotide sequence encoding the niacin transporter of SEQ ID NO:12 is shown in SEQ ID NO:11.

Specifically, the niacin transporter of the present invention may preferably have a polypeptide with an amino acid sequence with a homology (preferably identity) of 80% or more, particularly 85% or more, still particularly 90% or more, even still particularly 95% or more, or 96% or more, or 97% or more, or 99% or more, especially 100%, to the amino acid sequence shown in SEQ ID NO:9 or SEQ ID NO:12.

**(3) Transporter protein that promotes the extracellular export of NAm derivatives**

**(NAm derivative transporter):**

Various transporters derived from various microorganisms have been known as transporters that promote the extracellular export of NMN, which is a product of NMN synthesis, and/or NAm derivatives such as NR and NaMN, which are synthesized from NMN (NAm derivative transporters). Such known transporter proteins can be selectively used as

appropriate.

According to the present invention, a specific transporter protein with improved activity may preferably be used as an NAm derivative transporter. One reason is that an NAm derivative transporter with poor NAm derivative excretion efficiency may leave a substantial amount of NMN decomposed and/or converted in the cell, resulting in a decrease in NMN production. Another reason is that an NAm derivative transporter with improved excretion efficiency may serve to accelerate the extracellular excretion of the produced NAm derivatives and thereby facilitate the recovery process of the produced NAm derivatives. Such a transporter protein with improved NAm derivative excretion efficiency may be referred to herein as "the NAm derivative transporter of the present invention," although NAm derivative transporters that can be used in the present invention are not limited to the one explained below.

Specifically, the NAm derivative transporter of the present invention may preferably increase the efficiency of extracellular excretion of nicotinamide derivatives (NAm derivative excretion efficiency) by the host microorganism by usually 3-fold or more, particularly 5-fold or more, even more particularly 7-fold or more, compared to the NAm derivative excretion efficiency of the host microorganism that does not express the NAm derivative transporter of the present invention.

A specific example of a method for measuring the NAm derivative excretion efficiency of a NAm derivative transporter, in the case of a transporter that transports NMN as an NAm derivative (i.e., NMN transporter), is as follows: Escherichia coli (E. Coli) BL21 (DE3) strain is genetically engineered to express NAMPT with a NAMPT conversion efficiency of 200 or higher, and the resulting strain is then transformed with a plasmid derived from pCDFDuet-1 by inserting E. coli (E. Coli) K12-derived genes prs, rpiB, rpiA, gnd, pgl, zwf, and pgi so as to express these genes in this order. The resultant transformant strain is further transformed with another plasmid derived pACYCDuet-1 via incorporation of a gene encoding the NMN transporter to prepare a construct strain, which is then inoculated into a test tube containing 5 mL of LB medium and cultured at 37°C at 200 rpm for 12 hours. The culture is then inoculated into a 500 ml conical flask containing 200 ml of LB medium such that the resulting $OD_{600}$ is 0.03, and incubated at 37°C at 200 rpm. When $OD_{600}$ reached 0.4, isopropyl-β-thiogalactopyranoside is added such that the final concentration becomes 0.1 mM, and the culture is further incubated at 25°C at 200 rpm for 16 hours. The culture medium is then transferred to a 50 mL conical tube, centrifuged at 3000g for 5 minutes, and the bacterial cells are collected. The tube is washed with 1×PBS, the wash fluid is centrifuged at 3000g for 5 minutes, and the bacterial cells are collected. This operation is repeated twice. The collected bacterial cells are suspended in LB medium to obtain an $OD_{600}$ of 10, 10 mL of which suspension is transferred to a 100-mL conical flask, and combined with nicotinamide at 1 g/L, $_{D}$-glucose at 0.4 g/L, and phosphate buffer (pH 6.2) at 0.005 mol/L, and the reaction is allowed to run at 30°C at 200 rpm. The reaction solution is collected 2 hours from the start of the reaction. The collected solution is divided into two, one of which is frozen at -30°C, thawed, and centrifuged at 12,000 rpm for 3 minutes to collect the supernatant, and the other is not frozen but is directly centrifuged at 12,000 rpm for 3 minutes to collect the supernatant. These liquid samples are analyzed by HPLC to quantify the amounts of NMN. The obtained NMN quantitative values is used for determining the NMN excretion efficiency of NMN transporter according to Equation (2) below.

[Formula 2]

$$\text{NMN excretion efficiency of the NMN transporter (\%)} = \{(\text{NMN amount without frozen treatment})/(\text{NMN amount with frozen treatment})\}\times 100 \quad \text{Equation (2)}$$

In the case of NAm derivative transporters that transport NAm derivatives other than NMN, the excretion efficiency of NAm derivatives can be determined in accordance with a method similar to the one explained above.

However, the measurement method of the NAm derivative excretion efficiency of an NAm derivative transporter is not limited to the specific example explained above, but may be any other evaluation method so long as it gives equivalent values.

Among them, the NAm derivative transporter of the present invention may preferably be a protein comprising a polypeptide having the amino acid sequence represented by SEQ ID NO:13, or a polypeptide having a similar amino acid sequence thereto.

The amino acid sequence of the NAm derivative transporter (NMN transporter) from Bacillus mycoides is shown in SEQ ID NO: 15, and the nucleotide sequence of the naturally-occurring gene encoding the NAm derivative transporter of SEQ ID NO:15 is shown in SEQ ID NO:13. The present inventors optimized the nucleotide sequence of the naturally-occurring gene of SEQ ID NO:13 so as to improve its expression and activity in the host microorganism. The resulting

optimized nucleotide sequence encoding the NAm derivative transporter of SEQ ID NO:15 is shown in SEQ ID NO:14.

Specifically, the NAm derivative transporter of the present invention may preferably have a polypeptide with an amino acid sequence with a homology (preferably identity) of 80% or more, particularly 85% or more, still particularly 90% or more, even still particularly 95% or more, or 96% or more, or 97% or more, or 99% or more, especially 100%, to the amino acid sequence shown in SEQ ID NO:15.

**(4) Enzymes involved in the synthesis of PRPP from G6P (PGI, GPD, PGL, PGD, RPI, and PRS):**

As the enzymes involved in the synthesis of PRPP from G6P, namely phosphoglucose isomerase (PGI), glucose 6-phosphate dehydrogenase (GPD), 6-phosphogluconolactonase (PGL), 6-phosphogluconate dehydrogenase (PGD), ribose-5-phosphate isomerase (RPI), and phosphoribosyl pyrophosphate synthase (PRS) (collectively referred to as "PRPP synthesis-related enzymes" as appropriate), various enzymes derived from various microorganisms have been known, and have also been optimized according to various host microorganisms. Such known enzymes can be selectively used as appropriate.

Examples of PRPP synthesis-related enzymes particularly preferred for use in the present invention are listed below. However, PRPP synthesis-related enzymes that can be used in the present invention are not limited to these examples.

Phosphoglucose isomerase (PGI) may be an enzyme comprising a polypeptide having the amino acid sequence shown in SEQ ID NO:18, or a polypeptide having a similar amino acid sequence thereto.

The amino acid sequence of the enzyme pgi from E. coli is shown in SEQ ID NO:18, and the nucleotide sequence of the naturally-occurring gene encoding the enzyme pgi of SEQ ID NO:18 is shown in SEQ ID NO:16. The present inventors optimized the nucleotide sequence of the naturally-occurring gene of SEQ ID NO:16 so as to improve its expression and activity in the host microorganism. The resulting optimized nucleotide sequence encoding the enzyme pgi of SEQ ID NO:18 is shown in SEQ ID NO:17.

Specifically, the enzyme pgi may preferably have a polypeptide with an amino acid sequence with a homology (preferably identity) of 80% or more, particularly 85% or more, still particularly 90% or more, even still particularly 95% or more, or 96% or more, or 97% or more, or 99% or more, especially 100%, to the amino acid sequence shown in SEQ ID NO:18.

Glucose 6-phosphate dehydrogenase (GPD) may be an enzyme comprising a polypeptide having the amino acid sequence shown in SEQ ID NO:21, or a polypeptide having a similar amino acid sequence thereto.

The amino acid sequence of the enzyme zwf from E. coli is shown in SEQ ID NO:21, and the nucleotide sequence of the naturally-occurring gene encoding the enzyme zwf of SEQ ID NO:21 is shown in SEQ ID NO:19. The present inventors optimized the nucleotide sequence of the naturally-occurring gene of SEQ ID NO:19 so as to improve its expression and activity in the host microorganism. The resulting optimized nucleotide sequence encoding the enzyme zwf of SEQ ID NO:21 is shown in SEQ ID NO:20.

Specifically, the enzyme GPD may preferably have a polypeptide with an amino acid sequence with a homology (preferably identity) of 80% or more, particularly 85% or more, still particularly 90% or more, even still particularly 95% or more, or 96% or more, or 97% or more, or 99% or more, especially 100%, to the amino acid sequence shown in SEQ ID NO:21.

6-Phosphogluconolactonase (PGL) may be an enzyme comprising a polypeptide having the amino acid sequence shown in SEQ ID NO:24, or a polypeptide having a similar amino acid sequence thereto.

The amino acid sequence of the enzyme pgl from E. coli is shown in SEQ ID NO:24, and the nucleotide sequence of the naturally-occurring gene encoding the enzyme pgl of SEQ ID NO:24 is shown in SEQ ID NO:22. The present inventors optimized the nucleotide sequence of the naturally-occurring gene of SEQ ID NO:22 so as to improve its expression and activity in the host microorganism. The resulting optimized nucleotide sequence encoding the enzyme pgl of SEQ ID NO:24 is shown in SEQ ID NO:23.

Specifically, the enzyme PGL may preferably have a polypeptide with an amino acid sequence with a homology (preferably identity) of 80% or more, particularly 85% or more, still particularly 90% or more, even still particularly 95% or more, or 96% or more, or 97% or more, or 99% or more, especially 100%, to the amino acid sequence shown in SEQ ID NO:24.

6-Phosphogluconate dehydrogenase (PGD) may be an enzyme comprising a polypeptide having the amino acid sequence shown in SEQ ID NO:27, or a polypeptide having a similar amino acid sequence thereto.

The amino acid sequence of the enzyme gnd from E. coli is shown in SEQ ID NO:27, and the nucleotide sequence of the naturally-occurring gene encoding the enzyme gnd of SEQ ID NO:27 is shown in SEQ ID NO:25. The present inventors optimized the nucleotide sequence of the naturally-occurring gene of SEQ ID NO:25 so as to improve its expression and activity in the host microorganism. The resulting optimized nucleotide sequence encoding the enzyme gnd of SEQ ID NO:27 is shown in SEQ ID NO:26.

Specifically, the enzyme PGD may preferably have a polypeptide with an amino acid sequence with a homology

(preferably identity) of 80% or more, particularly 85% or more, still particularly 90% or more, even still particularly 95% or more, or 96% or more, or 97% or more, or 99% or more, especially 100%, to the amino acid sequence shown in SEQ ID NO:27.

Ribose-5-phosphate isomerase (RPI) may be an enzyme comprising a polypeptide having the amino acid sequence shown in SEQ ID NO:30 or SEQ ID NO:33, or a polypeptide having a similar amino acid sequence thereto.

The amino acid sequence of the enzyme rpiA from E. coli is shown in SEQ ID NO:30, and the nucleotide sequence of the naturally-occurring gene encoding the enzyme rpiA of SEQ ID NO:30 is shown in SEQ ID NO:28. The present inventors optimized the nucleotide sequence of the naturally-occurring gene of SEQ ID NO:28 so as to improve its expression and activity in the host microorganism. The resulting optimized nucleotide sequence encoding the enzyme rpiA of SEQ ID NO:30 is shown in SEQ ID NO:29.

The amino acid sequence of the enzyme rpiB from E. coli is shown in SEQ ID NO:33, and the nucleotide sequence of the naturally-occurring gene encoding the enzyme rpiB of SEQ ID NO:33 is shown in SEQ ID NO:31. The present inventors optimized the nucleotide sequence of the naturally-occurring gene of SEQ ID NO:31 so as to improve its expression and activity in the host microorganism. The resulting optimized nucleotide sequence encoding the enzyme rpiB of SEQ ID NO:33 is shown in SEQ ID NO:32.

Specifically, the enzyme RPI may preferably have a polypeptide with an amino acid sequence with a homology (preferably identity) of 80% or more, particularly 85% or more, still particularly 90% or more, even still particularly 95% or more, or 96% or more, or 97% or more, or 99% or more, especially 100%, to the amino acid sequence shown in SEQ ID NO:30 or SEQ ID NO:33.

Phosphoribosyl pyrophosphate synthase (PRS) may be an enzyme comprising a polypeptide having the amino acid sequence shown in SEQ ID NO:36, or a polypeptide having a similar amino acid sequence thereto.

The amino acid sequence of the enzyme prs from E. coli is shown in SEQ ID NO:36, and the nucleotide sequence of the naturally-occurring gene encoding the enzyme prs of SEQ ID NO:36 is shown in SEQ ID NO:34. The present inventors optimized the nucleotide sequence of the naturally-occurring gene of SEQ ID NO:34 so as to improve its expression and activity in the host microorganism. The resulting optimized nucleotide sequence encoding the enzyme prs of SEQ ID NO:36 is shown in SEQ ID NO:35.

Specifically, the enzyme PRS may preferably have a polypeptide with an amino acid sequence with a homology (preferably identity) of 80% or more, particularly 85% or more, still particularly 90% or more, even still particularly 95% or more, or 96% or more, or 97% or more, or 99% or more, especially 100%, to the amino acid sequence shown in SEQ ID NO:36.

The NMN synthase, niacin transporter, NAm derivative transporter, and/or PRPP synthesis-related enzymes (PGI, GPD, PGL, PGD, RPI, and PRS) can be derived from any source. In other words, each of these enzymes and transporters may be either a gene endogenous to the host organism or a gene derived from any gene exogenous to the host microorganism and artificially modified so as to be expressed in the host microorganism via genetic recombination or any other means.

The enzymes and transporters of the invention with improved activity as mentioned above, especially the NMN synthase of the invention, can be recovered from the host microorganism via general means such as extraction and isolation, and can preferably be used for other applications such as enzymatic reactions as appropriate.

## [III. Vectors]

This chapter deals with the vectors for expressing the enzymes involved in the production of NAm derivatives used in the present invention.

According to an aspect of the invention, a vector carrying a nucleic acid encoding the amino acid sequence(s) of the NMN synthase, niacin transporter, NAm derivative transporter, and/or PRPP synthases (PGI, GPD, PGL, PGD, RPI, and PRS) is produced, and used for introducing the enzyme(s)/transporter(s) into the host microorganism. There is no limitation to the combination of the enzyme(s)/transporter(s) to be carried by the vector. Each enzyme/transporter may be carried by a separate vector, or two or more of the enzyme(s)/transporter(s) may be carried together by a single vector.

An exemplary vector according to the present invention is a vector carrying a nucleic acid encoding the amino acid sequence of the NMN synthase (NAMPT) of the present invention as described above. Such vectors may be referred to as "the NMN synthase vector of the present invention" or as "the NAMPT vector of the present invention" as appropriate.

The NMN synthase vector of the present invention may preferably carry, as the nucleic acid encoding the NMN synthase of the present invention, a nucleic acid having a nucleotide sequence with an identity of 80% or more, particularly 85% or more, more particularly 90% or more, even more particularly 95% or more, or 96% or more, or 97% or more, or 99% or more, especially 100%, to the nucleotide sequence shown in SEQ ID NO:2 or SEQ ID NO:5.

Another exemplary vector according to the present invention is a vector carrying a nucleic acid encoding the amino acid sequence of the niacin transporter of the present invention as described above. Such vectors may be referred

to as "the niacin transporter vector of the present invention" as appropriate.

The niacin transporter vector of the present invention may preferably carry, as the nucleic acid encoding the niacin transporter of the present invention, a nucleic acid having a nucleotide sequence with an identity of 80% or more, particularly 85% or more, more particularly 90% or more, even more particularly 95% or more, or 96% or more, or 97% or more, or 99% or more, especially 100%, to the nucleotide sequence shown in SEQ ID NO:8 or SEQ ID NO:11.

Still another exemplary vector according to the present invention is a vector carrying a nucleic acid encoding the amino acid sequence of the NAm derivative of the present invention as described above. Such vectors may be referred to as "the NAm derivative transporter vector of the present invention" as appropriate.

The NAm derivative transporter vector of the present invention may preferably carry, as the nucleic acid encoding the NAm derivative transporter of the present invention, a nucleic acid having a nucleotide sequence with an identity of 80% or more, particularly 85% or more, more particularly 90% or more, even more particularly 95% or more, or 96% or more, or 97% or more, or 99% or more, especially 100%, to the nucleotide sequence shown in SEQ ID NO:14.

Still another exemplary vector according to the present invention is a vector carrying a nucleic acid encoding the amino acid sequence(s) of one or more of the PRPP synthetases as described above, i.e., PGI, GPD, PGL, PGD, RPI, and PRS. Such vectors may be collectively referred to as "the PRPP synthase vectors of the present invention," and each may also be referred to using the name of the enzyme corresponding to the nucleic acid to be carried, as, e.g., "the GPI enzyme vector of the present invention."

Specifically, the GPI vector of the present invention may preferably carry, as the nucleic acid encoding the GPI of the present invention, a nucleic acid with a nucleotide sequence with an identity of 80% or more, particularly 85% or more, still particularly 90% or more, even still particularly 95% or more, or 96% or more, or 97% or more, or 99% or more, especially 100%, to the nucleotide sequence shown in SEQ ID NO:17.

The GPD vector of the present invention may preferably carry, as the nucleic acid encoding the GPD of the present invention, a nucleic acid with a nucleotide sequence with an identity of 80% or more, particularly 85% or more, still particularly 90% or more, even still particularly 95% or more, or 96% or more, or 97% or more, or 99% or more, especially 100%, to the nucleotide sequence shown in SEQ ID NO:20.

The PGL vector of the present invention may preferably carry, as the nucleic acid encoding the PGL of the present invention, a nucleic acid with a nucleotide sequence with an identity of 80% or more, particularly 85% or more, still particularly 90% or more, even still particularly 95% or more, or 96% or more, or 97% or more, or 99% or more, especially 100%, to the nucleotide sequence shown in SEQ ID NO:23.

The PGD vector of the present invention may preferably carry, as the nucleic acid encoding the PGD of the present invention, a nucleic acid with a nucleotide sequence with an identity of 80% or more, particularly 85% or more, still particularly 90% or more, even still particularly 95% or more, or 96% or more, or 97% or more, or 99% or more, especially 100%, to the nucleotide sequence shown in SEQ ID NO:26.

The RPI vector of the present invention may preferably carry, as the nucleic acid encoding the RPI of the present invention, a nucleic acid with a nucleotide sequence with an identity of 80% or more, particularly 85% or more, still particularly 90% or more, even still particularly 95% or more, or 96% or more, or 97% or more, or 99% or more, especially 100%, to the nucleotide sequence shown in SEQ ID NO:29 or SEQ ID NO:32.

The PRS vector of the present invention may preferably carry, as the nucleic acid encoding the PRS of the present invention, a nucleic acid with a nucleotide sequence with an identity of 80% or more, particularly 85% or more, still particularly 90% or more, even still particularly 95% or more, or 96% or more, or 97% or more, or 99% or more, especially 100%, to the nucleotide sequence shown in SEQ ID NO:35.

In the present disclosure, a vector carrying nucleic acids of two or more enzymes is referred to by the names of the enzyme linked with a slash. For example, the term "GPI/GPD/PGL/PGD/RPI/PRS vector" means a vector carrying nucleic acids encoding the amino acid sequences of GPI, GPD, PGL, PGD, RPI, and PRS.

Each vector mentioned herein may be in any form, as long as it has a nucleic acid region encoding an amino acid sequence of the corresponding enzyme (hereinafter referred to as the "coding region"). For example, it may be either a linear vector or a circular vector. Each DNA to be incorporated into the genome of the host cell may be either carried by a single vector or divided and carried by two or more vectors.

There is no limitation to the replication capacity of each vector mentioned herein. For example, each vector may be an autonomously replicable vector, i.e., a vector that exists outside the chromosomes of the host cell and replicates independently of the chromosome replication. Examples of such autonomously replicable vectors include plasmid vectors, extrachromosomal elements, minichromosomes, and artificial chromosomes. In this case, the vector may usually contain, in addition to the nucleic acid encoding the enzyme mentioned above, functional elements necessary for autonomous replication, such as a replication origin. Examples of replication origins that can be used in E. coli host cells include pUC replication origin, RSF replication origin, p15A replication origin, ColDF13 replication origin, ColE1 replication origin, pBR322 replication origin, pACYC replication origin, pSC101 replication origin, f1 replication origin, M13 replication origin, BAC vector replication origin, PAC vector replication origin, cosmid vector replication origin, etc. Examples of replication origins that can be used in yeast host cells include the 2$\mu$ origins, ARS, etc.

Alternatively, each vector mentioned herein may not be capable of autonomous replication, and may be incorporated into the genome of the host cell when introduced into the host cell, and replicated together with the host genome. In this case, the nucleic acids to be incorporated into the host cell genome may be carried by a single vector or may be divided and carried by two or more vectors. Examples of such vectors lacking autonomous replication ability include virus vectors, phage vectors, cosmid vectors, and fosmid vectors.

Such vectors lacking autonomous replication ability may be configured to be precisely incorporated by homologous recombination into a desired position in a desired chromosome of the host cell. In this case, the nucleic acid to be incorporated into the genome of the host cell may be sandwiched between a pair of flanking sequences having complementary nucleotide sequences on both sides of the desired integration site. The length of each flanking sequence is not restricted, but may be, e.g., 50 bases or more, 100 bases or more, or 200 bases or more. Such recombination can also be achieved using various known recombinases, such as Red recombinase from lambda phage and RecE/RecT recombinase from Rac prophage.

Alternatively, such vectors lacking autonomous replication ability may be configured to be incorporated into the genome of the host cell by non-homologous recombination. In this case, the nucleic acids to be incorporated into the genome of the host cell may be sandwiched by the RB and LB sequences derived from the T-DNA of Agrobacterium, or by various known transposon sequences. Alternatively, the desired nucleic acid may be inserted into the genome of the host cell using genome editing technology.

In addition to the coding region of the enzyme and sequences for autonomous replication and/or for integration into the genome as mentioned above, each vector of the present invention may also contain one or more additional nucleic acid regions having other functions. Examples include regulatory sequences that control the expression of the coding region, selection marker genes, and multi-cloning sites.

Examples of regulatory sequences include promoters, ribosome binding sequences, enhancers, cis-elements, terminators, and the like. Such regulatory sequences may be selected and used based on, e.g., the type of the host cell to be used, the size of the enzyme, etc. Specific examples include, but are not limited to, the following.

Examples of promoters that can be used in E. coli host cells include: trp promoter, lac promoter, PL promoter, PR promoter, tac promoter, T7 promoter, and T5 promoter. Examples of promoters that can be used in yeast host cells include: gal1promoter, gal10 promoter, heat shock protein promoter, MF$\alpha$1 promoter, PHO5 promoter, PGK promoter, GAP promoter, ADH promoter, and AOX1 promoter.

Any known ribosome-binding sequence for use in various host cells can be used so long as it allows mRNA transcribed from DNA to bind to ribosomes in the host cell when the biosynthesis of a protein is initiated.

Examples of terminators that can be used in E. coli host cells include T7 terminator, fd phage terminator, T4 terminator, the terminator of the tetracycline resistance gene, and the terminator of the E. coli trpA gene. Examples of terminators that can be used in yeast host cells include PGK1 terminator, CYC1 terminator, and DIT1 terminator.

The coding region of any of the enzymes mentioned above may be operably linked to such regulatory sequences (e.g., a promoter, a ribosome-binding sequence, and a terminator) in advance such that the enzyme can be expressed from the coding region under the control of these regulatory sequences.

Alternatively, the coding region of any of the enzymes mentioned above may be configured to be operably linked to such regulatory sequences (e.g., promoters, ribosome-binding sequences, and terminators) of the host cell or of the vector upon recombination such that the enzyme can be expressed from the coding region under the control of these regulatory sequences.

A selection marker gene may be used for confirming that the vector has been properly introduced into the host cell and (in the case of vectors lacking the ability of autonomous replication) incorporated into the genome. Any sequence can be selected as the selection marker gene depending on the type of the host cell to be used. Examples of selection markers that can be used in E. coli host cells include, although not limited to: Ampr, Tetr, Cmr, Kmr, Spcr, Smr, Hygr, Gmr, Rifr, Zeocinr, and Blasticidinr. Examples of selectable markers include, although not limited to: URA3, TRP1, SUP4, ADE2, HIS3, LEU2, LYS2, KANMX, AUR1-C, CYH2, CAN1, PDR4, and hphMX.

The selection marker gene may preferably be operably linked to regulatory sequences (e.g., a promoter, a ribosome-binding sequence, and a terminator) and constitute a cassette having the ability to be expressed autonomously, such that it can be expressed in the host cell as appropriate. The regulatory sequences for the expression of the selection marker gene may be prepared independently of the regulatory sequences for the expression of the enzyme(s) as described above, or the selection marker gene may share the same regulatory sequences with the enzyme(s) as described above.

After the host cells are transformed with the vector, the transformed cells are incubated under selection conditions that allow only cells expressing the selection marker to survive, in order to select cells in which the vector has been properly introduced and (in the case of vectors lacking the ability of autonomous replication) incorporated into the genome.

**[IV. Recombinant microonmnism 1**

This chapter deals with the recombinant microorganisms used in the present invention to produce NMN.

An aspect of the present invention relates to a recombinant microorganism expressing the NMN synthases, niacin transporters, NAm derivative transporters, and/or PRPP synthesis-related enzymes (GPI, GPD, PGL, PGD, RPI, and/or PRS) mentioned above. Such microorganismsmay be referred to as "the recombinant microorganisms of the present invention."

The recombinant microorganism of the present invention may be obtained by transforming a host microorganism with the vector of the present invention described above. The biological species of the recombinant microorganism and its host microorganism is not particularly limited, but may preferably be a bacterium or fungus. Examples of bacteria include, although not limited to, those belonging to the genera Escherichia, Staphylococcus, Bacillus, Pseudomonas, Proteus, Corynebacterium, and Actinomyces, among which those belonging to the genus Escherichia (e.g., E. coli) or Corynebacterium may preferably be used. Examples of fungi include, although not limited to, yeasts and filamentous fungi, of which yeasts are preferred. Examples of yeasts include those belonging to the genera Saccharomyces, Candida, Yarrowia, Pichia, and Kluyveromyces.

Some types of host microorganisms may have the ability to express endogenous enzymes corresponding to the NAMPT, the niacin transporter, the NAm derivative transporter, and/or the PRPP synthesis-related enzymes. In such cases, the endogenous NAMPT, niacin transporter, NAm derivative transporter, and/or PRPP synthesis-related enzymes may be used for the biosynthesis of NMN. However, even if the host microorganism is capable of expressing endogenous enzymes corresponding to NAMPT, niacin transporters, NAm derivative transporters, and/or PRPP synthesis-related enzymes, it is preferable to genetically modify the host microorganism to express these enzymes/transporters from the perspective of achieving higher expression of these enzymes/transporters and improved efficiency of final NAm derivative production.

Specifically, according to an aspect of the present invention, the recombinant microorganism may preferably be genetically modified to express at least the NMN synthase of the present invention, and may more preferably be genetically modified to also express the NAm derivative transporter and/or the niacin transporter of the present invention. In addition, the recombinant microorganism of the present invention may preferably be genetically modified to express at least any one of the PRPP synthesis-related enzymes, specifically GPI, GPD, PGL, PGD, RPI, and PRS, and may more preferably be genetically modified to express any two, three, four, or five, an even more preferably all, of GPI, GPD, PGL, PGD, RPI, and PRS.

Any offspring obtained by growing the recombinant microorganism of the present invention also fall under the scope of the recombinant microorganism of the present invention as long as they maintain the ability to express the NMN synthase, niacin transporter, NAm derivative transporter, and/or PRPP synthesis-related enzymes mentioned above.

The recombinant microorganism of the present invention may only have to be capable of expressing the NMN synthase, niacin transporter, NAm derivative transporter, and/or PRPP synthesis-related enzymes mentioned above. However, various modifications may be made thereto in consideration of the production efficiency of the desired NAm derivative.

An example of such modification is genetic recombination causing knockout or knockdown of any of various enzymes which otherwise may lead to a decrease in the production efficiency of the target NAm derivative.

For example, if the NAm derivative to be manufactured is NMN, the enzymes involved in the conversion of NMN to various other NAms mentioned above (specifically, nicotinamide/nicotinate mononucleotide adenylyltransferase (NMNAT), which converts NMN to NAD, nicotinamide nucleotide amidase (NANA), which converts NMN to nicotinic acid mononucleotide (NaMN), nicotinamide mononucleotide-5-nucleotidase (NMNN), which converts NMN to nicotinamide riboside (NR), etc.) are unnecessary; rather, the presence of these enzymes may lead to a decrease in the production efficiency of NMN. It may therefore be preferable to knock-out or knock-down the genes of these enzymes in order to prevent or reduce their expression and to prevent the synthesized NMN from being converted to such other NAm derivatives.

If the NAm derivative to be manufactured is a derivative other than NMN, such as NAD, NaMN, NR, etc., the gene of the enzyme that converts NMN into the desired NAm derivative may preferably be promoted (e.g., by means of external gene transfer, etc.), while the genes of the enzymes that convert NMN into the other derivative may preferably be knocked out or knocked down in order to prevent or reduce their expression, so as not for the synthesized NMN to be converted into other NAm derivatives than the desired NAm derivative. Alternatively, NMN produced by the microorganism of the present invention may further be converted into the desired NAm derivative via enzymatic or chemical reaction.

Various methods of knocking out or knocking down the genes of various enzymes via genetic recombination are known in the art.

Another example of modification is to carry out physical or chemical treatment, or both, on the cell surface of

the recombinant microorganism, instead of or in conjunction with the recombinant expression of niacin transporters and/or NAm derivative transporters, etc., in order to facilitate the intracellular uptake of NAm, a reactant of the NMN synthesis reaction and also to facilitate the extracellular excretion of the produced NMN. Examples of physical treatments include, although not limited to, freezing, drying, and sonication of the microorganism. Examples of chemical treatments include, although not limited to, addition of surfactants such as Triton X-100, Triton X-114, NP-40, Tween-20, Tween-80, and CHAPS; addition of organic solvents such as alcohols and xylene; and $Mn^{2+}$-restricted culture.

## TV. Method of producing NAm derivatives]

This chapter deals with the method of producing NAm derivatives using the recombinant microorganisms mentioned above.

An aspect of the present invention relates to a method for producing a NAm derivative, the method comprising supplying NAm to the recombinant microorganism of the present invention described above and then recovering the NAm derivative produced by the microorganism. This production method may be referred to as "the method of NAm derivative production of the present invention" as appropriate.

The following description will be made on various procedures and conditions of the method of NAm derivative production of the present invention, mainly with reference to an example where the NAm derivative is NMN and the host microorganism is E. coli. However, the method of NAm derivative production of the present invention is not limited to the one using the procedures and conditions of described below, but may be carried out with making various modifications thereto.

The method for feeding NAm to the recombinant microorganism of the present invention is not particularly limited. For example, NAm may be added directly to the culture medium in which the recombinant microorganism of the present invention is being cultured. However, in consideration of the efficiency of production and recovery of the resulting NAm derivative, the medium may preferably be removed via, e.g., centrifugation, and the resulting recombinant microorganism may preferably be added to a reaction solution which has a composition suitable for the reaction. The feeding of the recombinant microorganism may be carried out in bulk, continuously, or intermittently.

The composition of the reaction solution for the NAm derivative production is not limited. For example, it may only contain, as minimum components, the recombinant microorganism of the invention as well as NAm, which is the substrate for the NAm derivative synthesis reaction, in various media used for cultivation, such as an aqueous solution such as phosphate buffer or phosphate-buffered saline (PBS), or water. However, in order to promote the production of the NAm derivative, it may preferably also contain, as nutrient sources for the recombinant microorganism: organic carbon sources such as glucose, glycerol, fructose, starch, and blackstrap molass; and inorganic carbon sources such as carbonates, as well as phosphates such as potassium dihydrogen phosphate and dipotassium hydrogen phosphate as phosphorus components. Other components such as minerals, nitrogen sources, and ATP may be added as appropriate.

Any generally known synthetic or natural culture media can be used as various types of culture media, as long as they do not adversely affect the NAm derivative production.

The composition ratios of the reaction solution are not limited, but may be, for example, as follows:
Although the cell number of the recombinant microorganism is not limited, if the cell number is too low, the reaction may be carried out in such a diluted state that the reaction may not progress sufficiently, while if the cell number is too high, side reactions other than the desired NAm derivative production may occur. In terms of optical density (OD) measured at a wavelength appropriate for the microorganism, the cell number may preferably correspond to an OD of 1 or more, more preferably 5 or more, even more preferably 10 or more, and may preferably correspond to an OD of OD of 500 or less, more preferably 300 or less. In the case of E. coli, the OD may be measured at a wavelength of, e.g., 600 nm.

Although the concentration of NAm is not limited, if the concentration is too low, the amount of NAm taken up by the microorganism may be so low that the desired reaction may not proceed significantly, while if the concentration is too high, it may place a burden on the microorganism. Accordingly, the concentration may preferably be 10 mg/L or more, particularly 100 mg/L or more, more particularly 1000 mg/L or more, and may preferably be 300 g/L or less, particularly 250 g/L or less, more particularly 200 g/L or less.

Although the concentration of carbon source is not limited, if the concentration is too low, metabolism may not sufficiently proceed in the microorganism, while if the concentration is too high, it may place a burden on the microorganism. Accordingly, the concentration may preferably be 10 mg/L or more, particularly 50 mg/L or more, more particularly 100 mg/L or more, and may preferably be 300 g/L or less, particularly 250 g/L or less, more particularly 200 g/L or less.

Although the concentration of phosphorus component is not limited, if the concentration is too low, metabolism may not sufficiently proceed in the microorganism, while if the concentration is too high, it may place a burden on the microorganism. Accordingly, the concentration may preferably be 0.1 mmol/L or more, particularly 0.5 mmol/L or more, more particularly 1 mmol/L or more, and may preferably be 10 mol/L or less, particularly 5 mol/L or less, more particularly 1 mol/L or less.

Other components may also preferably be added in appropriate amounts according to the cell number of the microorganism and the composition ratios of the reaction solution to be used.

These components of the reaction solution may be mixed either simultaneously at once or sequentially in any order. For example, the components other than the recombinant microorganism of the invention and NAm may first be mixed to prepare a reaction solution of basic composition, and then the recombinant microorganism of the invention may be added to the reaction solution. When the recombinant microorganism of the invention starts cellular activity and growth, NAm, the reactant, may be added to start the synthesis reaction of the NAm derivative.

Part of the medium used for the pre-culture of the recombinant microorganism may remain in the reaction solution, so long as it does not interfere with the NAm derivative synthesis reaction.

The pH of the reaction solution may be adjusted as appropriate such that it becomes the optimal pH for the recombinant microorganism of the invention. However, from the viewpoint of the durability of the recombinant microorganism and the stability of the NAm derivative, the pH of the reaction solution may preferably be adjusted at pH 2 or more, especially 3 or more, and for the same reason, it may preferably be adjusted at pH 9 or less, especially 8 or less. The pH may be adjusted using a pH adjusting agent such as calcium carbonate, inorganic or organic acids, alkaline solutions, ammonia, and pH buffers.

The reaction conditions during the NAm derivative production are not limited, but may be, for example, as follows.

The temperature during the reaction may be adjusted as appropriate so long as it is optimal for the recombinant microorganism of the invention, but from the viewpoint of progressing the reaction, the temperature may preferably be 15°C or more, particularly 20°C or more, and from the viewpoint of durability of the recombinant microorganism and stability of the NAm derivative, the temperature may preferably be 50°C or less, particularly 40°C or less.

The pressure during the reaction is also not limited, but may typically be at ambient pressure.

The atmosphere during the reaction may be selected so as to be optimal for the recombinant microorganism of the present invention from, e.g., an ambient atmosphere, an aerobic atmosphere, a hypoxic atmosphere, or an anaerobic atmosphere.

During the reaction, the reaction solution may be shaken or stirred as appropriate.

Although the reaction time depends on, e.g., the type of the recombinant microorganism, the composition ratios of the reaction solution, and the reaction conditions, if the reaction time is too short, the NAm derivative production may not be sufficiently advanced, while if the reaction time is too long, the produced NAm derivatives may be converted or decomposed. For this reason, the reaction time may preferably be 0.1 hours or more, particularly 0.3 hours or more, more particularly 0.5 hours or more, and may preferably be 120 hours or less, particularly 96 hours or less, more particularly 72 hours or less.

The reaction method may be selected from any generally known methods depending on the microorganism used and the reaction conditions. Examples include batch type, continuous batch type, flow microreactor type, loop reactor type, and single-use type.

After the reaction, the NAm derivative produced by the recombinant microorganism of the present invention is recovered. Typically, the produced NAm derivative permeates the cell membrane of the recombinant microorganism of the invention and is secreted into the reaction solution, so the NAm derivative can be isolated and purified from the reaction solution.

Although the methods of isolation and purification are not particularly limited, general methods can be used such as removal of the bacteria, removal of impurities from the fermentation culture supernatant, purification and recovery of the target product. These processes may be used singly, but may preferably be used in combination of any two or more.

The process for removing the bacteria may be selected from any generally known methods. Specific examples include centrifugation, membrane separation, etc.

The process for removing impurities from the fermentation culture supernatant may be selected from any generally known methods. Specific examples include activated carbon treatment, filtration (specifically, including filtration by reverse osmosis membrane, nanofiltration membrane, microfiltration membrane, ultrafiltration membrane, microfiltration membrane, etc.) treatment, ion exchange resin, etc.

The process for purifying and recovering the target product may be selected from any generally known methods. Specific examples include affinity column chromatography, vacuum concentration, membrane concentration, lyophilization, solvent extraction, distillation, separation by column chromatography, separation by ion-exchange column, high-performance liquid chromatography (HPLC) method, and precipitation by recrystallization.

These processes can be used in combination. For example, isolation and purification may preferably be carried out by combining centrifugation, activated carbon treatment, ion exchange resin, nanofiltration membrane treatment, and recrystallization.

The pH during the isolation and purification is not particularly limited, but from the standpoint of the stability of the NAm derivative, the pH range may preferably be pH 2 or more, particularly 3 or more, and may preferably be pH 9 or less, particularly 8 or less. The pH may be adjusted via any method selected from generally known methods. Specific examples include pH adjusting agents such as calcium carbonate, inorganic or organic acids, alkaline solutions, ammonia,

and pH buffers.

The temperature during the isolation and purification is not particularly limited, but the lower limit may preferably be 10°C or more, particularly 15°C or more, more particularly 20°C or more. If the temperature is lower than the lower limit mentioned above, the NAm derivative may precipitate and make it difficult to carry out the desired isolation and purification process. On the other hand, the upper limit may preferably be 50°C or less, particularly 45°C or less, more particularly 40°C or less. If the temperature is higher than the upper limit mentioned above, the NAm derivative may decompose. However, when heating or cooling is performed during various isolation and purification processes, the temperature may temporarily deviate from the aforementioned suitable range.

The pH during recrystallization in the isolation and purification process is not particularly limited, but from the viewpoint of stability and ease of crystallization of the NAm derivative, the pH may preferably be adjusted to within the range of from 2 to 5, more preferably within the range of from 2 to 4. The acid to be used for pH adjustment is not limited, but may be selected from, e.g., hydrochloric acid, phosphoric acid, tartaric acid, malic acid, benzoic acid, acetic acid, succinic acid, and gluconic acid. Among them, hydrochloric acid may be most preferred.

If the NAm derivative remains in the recombinant microorganism cells, the cell membrane may be disrupted by methods such as homogenization, lysozyme, sonication, freeze-thawing, French pressing, or any other chemical, mechanical, or physical cell disruption method to excretion the NAm derivative into the reaction solution before the cells are subject to the isolation and purification of the NAm derivative.

### [VI. Others]

Although the invention has been explained in detail with reference to specific embodiments so far, the present invention should not be limited to the above-mentioned embodiments in any way, but may be implemented in any form so long as it does not deviate from the gist of the present invention.

## EXAMPLES

The invention will be described in further detail with reference to the Examples indicated below. However, the present invention should also not be limited to these Examples in any way, but may be implemented in any form so long as it does not deviate from the gist of the present invention.

### [I. Measurement conditions]

*Quantification of NMN:

| | | |
|---|---|---|
| Instrument: | | LCMS-2020 (Shimadzu Corporation) |
| Detector: | | 254nm |
| Column: | | TSK gel Amide-80, 3 μm, 4.6 mm × 50 mm |
| Column temperature: | | 30°C |
| Injection volume: | | 5 μL |
| Mobile phases: | A: | 0.1% formic acid in water |
| | B: | Acetonitrile/methanol (75/25) containing 0.1% formic acid |

<Condition>

Flow rate: 1 mL/min constant

| | |
|---|---|
| Mobile phase ratio: | 0 -> 2 min (B = 98% constant), |
| | 2 -> 6 min (B = 98 -> 60%), |
| | 6 -> 8 min (B = 60 -> 45%), |
| | 8 -> 12 min (B=45 -> 60%), |
| | 12 -> 15 min (B=60 -> 98%) |
| Measurement time: | 15.1 min. |

Quantification method: NMN standard samples were prepared with water at 0 g/L, 0.01 g/L, 0.05 g/L, 0.1 g/L, 0.25 g/L, 1 g/L, and 2.5 g/L. NMN area values obtained by measuring these samples were used to prepare a calibration curve. The NMN area value obtained by measuring each sample was used to quantify the NMN amount based on the calibration curve. Values less than 0.01 g/L was considered to be below the quantification limit.

*Concentration of bacterial cells (OD):

| | |
|---|---|
| Instrument: | UVmini-1240 (Shimadzu Corporation) |
| Measurement wavelength: | 600nm |
| Cell: | 1.5 mL disposable cell (Material: PS) |

Measurement method: A bacterial solution was diluted with water such that the measurement value was within the range of from 0.05 to 1.0. A cell containing 1 mL of culture medium diluted at the same ratio was set to the instrument to determine the zero point, and then a cell containing 1 mL of the prepared sample solution was set to the instrument and measured for $OD_{600}$.

**[II. Materialsl**

*E. coli:

BL21 (DE3) strain (NEB)

*Vectors:

pRSFDuet-1 (Novagen)
pCDFDuet-1 (Novagen)
pACYCDuet-1 (Novagen)

*Synthetic genes:

Chitinophaga pinensis-derived NAMPT (nicotinamide phosphoribosyl transferase: NMN synthetase)
Sphingopyxis sp. C-1-derived NAMPT
Homo sapiens-derived NAMPT
Burkholderia cenocepacia-derived niaP (niacin transporter)
Streptococcus pneumoniae TIGR4-derived niaX (niacin transporter)
Bacillus mycoides-derived pnuC (nicotinamide mononucleotide transporter)
E. coli K12-derived pgi (phosphoglucose isomerase)
E. coli K12-derived zwf (glucose 6-phosphate dehydrogenase)
E. coli K12-derived pgl (6-phosphogluconolactonase)
E. coli K12-derived gnd (6-phosphogluconate dehydrogenase)
E. coli K12-derived rpiA (ribose-5-phosphate isomerase)
E. coli K12-derived rpiB (ribose-5-phosphate isomerase)
E. coli K12-derived prs (phosphoribosyl pyrophosphate synthase)

SEQ ID NO:39 indicates the amino acid sequence of NAMPT derived from Homo sapiens, and SEQ ID NO:37 indicates the nucleotide sequence of the naturally-occurring gene encoding the NAMPT of SEQ ID NO:39. SEQ ID NO:37 indicates the nucleotide sequence of the naturally-occurring gene encoding the NAMPT of SEQ ID NO:39. SEQ ID NO:38 indicates the nucleotide sequence encoding the NAMPT of SEQ ID NO:39, which was optimized by the present inventors based on the sequence of the naturally-occurring gene such that its expression and activity were improved in the host microorganism.

*Medium components and substrate components:

$_D$-glucose (Nacalai Tesque Co., Ltd.)
Nicotinamide (Tokyo Chemical Industry Co., Ltd.)
PBS (Nippon Gene Co., Ltd.)
Phosphate buffer: prepared by mixing 1 M potassium dihydrogen phosphate (Nacalai Tesque Co., Ltd.) and 1 M dipotassium hydrogen phosphate (Nacalai Tesque Co., Ltd.) to adjust the pH at 6.2, followed by sterilization via autoclaving.
LB medium: prepared by mixing sodium chloride (Nacalai Tesque Co., Ltd.) 10 g/L, tryptone (Nacalai Tesque Co., Ltd.) 10 g/L, and dried yeast extract (Nacalai Tesque Co., Ltd.) 5 g/L, followed by sterilization via autoclaving.
M9 medium: prepared by mixing 48 mM disodium hydrogen phosphate (Nacalai Tesque Co., Ltd.), 22 mM potassium dihydrogen phosphate (Nacalai Tesque Co., Ltd.), 19 mM ammonium chloride (Nacalai Tesque Co., Ltd.), and 8.6 mM sodium chloride (Nacalai Tesque Co., Ltd.), followed by sterilization via autoclaving.

**[III. Construction of vectors]**

*Construction of pRSF-NAMPT CP:

The synthetic gene of NAMPT derived from Chitinophaga pinensis (SEQ ID NO:5), codon-optimized for expression in E. coli, was amplified via PCR using the following primer pair, each containing homologous regions that can be linked to pRSFDuet-1 digested with restriction enzymes NcoI and EcoRI, respectively. The amplified product was then linked to pRSFDuet-1, which had been digested with restriction enzymes NcoI and EcoRI, using the In-Fusion cloning method to thereby produce pRSF-NAMPT CP.

(Primer pair for NAMPT CP)

*Forward (SEQ ID NO:40):
AGGAGATATACCATGACCAAAGAAAACCTGATTCTGCTGGCAGATGCA

*Reverse (SEQ ID NO:41):
GCTCGAATTCGGATCTTAGATGGTTGCGTTTTTACGGATCTGCTCAAA

*Construction of pRSF-NAMPT SSC

The synthetic gene of NAMPT derived from Sphingopyxis sp. C-1 (SEQ ID NO:2), codon-optimized for expression in E. coli, was amplified via PCR using the following primer pair, each containing homologous regions that can be linked to pRSFDuet-1 digested with restriction enzymes NcoI and EcoRI, respectively. The amplified product was then linked to pRSFDuet-1, which had been digested with restriction enzymes NcoI and EcoRI, using the In-Fusion cloning method to thereby produce pRSF-NAMPT SSC.

(Primer pair for NAMPT SSC)

*Forward (SEQ ID NO:42):
AGGAGATATACCATGAAGAATCTGATTCTGGCCACCGATAGCTATAAA

*Reverse (SEQ ID NO:43):
GCTCGAATTCGGATCTTAACGACCTTCGCTACGTTTACGAACTGCATC

*Construction of pRSF-NAMPT HS

The synthetic gene of NAMPT derived from Homo sapiens (SEQ ID NO:38), codon-optimized for expression in E. coli, was amplified via PCR using the following primer pair, each containing homologous regions that can be linked to pRSFDuet-1 digested with restriction enzymes NcoI and EcoRI, respectively. The amplified product was then linked to pRSFDuet-1, which had been digested with restriction enzymes NcoI and EcoRI, using the In-Fusion cloning method to thereby produce pRSF-NAMPT HS.

(Primer pair for NAMPT HS)

*Forward (SEQ ID NO:44):
AGGAGATATACCATGAATCCGGCAGCAGAAGCCGAATTTAACATTCTG
*Reverse (SEQ ID NO:45):
GCTCGAATTCGGATCTTAATGATGTGCTGCTTCCAGTTCAATGTTCAG

*Construction of pCDF-prs->pgi:*

The synthetic genes for pgi, zwf, pgl, gnd, rpiA, rpiB, and prs derived from E. coli K12 (SEQ ID NOs: 17, 20, 23, 26, 29, 32, and 35, respectively), codon-optimized for expression in E. coli, were amplified by PCR using the following primer pairs, each containing homologous regions that can be linked to pRSFDuet-1 and, except for prs, the same RBS regions as that of pCDFDuet-1. First, the fragments of prs, rpiB, rpiA, and gnd were linked to pCDFDuet-1, which had been digested with restriction enzymes NcoI and SacI, using the Gibson Assembly system. The resulting vector was then digested with the restriction enzyme SacI, and linked with the remaining fragments of pgl, zwf, and pgi, using the Gibson Assembly system to produce pCDF-prs->pgi.

(Primer pair for pgi)

*Forward (SEQ ID NO:46):

CGTGATGGTCGTAGCTGGAATGAATTTGAATAAAAGGAGATATACCATGAAGA
ACATTAATCCGACACAG

*Reverse (SEQ ID NO:47):

ACTTAAGCATTATGCGGCCGCAAGCTTGTCGACCTGCAGGCGCGCCGTTAACC
ACGCCAGGCTTTATAAC

(Primer pair for zwf)

*Forward (SEQ ID NO:48):

GTCAGGGTCCGATGTGGGTTGTTGTTAATGCACATTAAAAGGAGATATACCAT
GGCAGTTACCCAGACCG

*Reverse (SEQ ID NO:49):
TTATTCAAATTCATTCCAGCTACG

(Primer pair for pgl)

*Forward (SEQ ID NO:50):

AGAAGGTGTGTTTCATACAGAATGGCTGGACTAAAAGGAGATATACCATGAAA CAGACCGTGTATATTGC

*Reverse (SEQ ID NO:51): TTAATGTGCATTAACAACAACCC

(Primer pair for gnd)

*Forward (SEQ ID NO:52):

TGGTACACCGGATGGTGTTAAAACCATTGTGAAATAAAAGGAGATATACCATG AGCAAACAGCAGATTGG

*Reverse (SEQ ID NO:53):

CATTATGCGGCCGCAAGCTTGTCGACCTGCAGGCGCGCCGAGCTCTTAGTCCA GCCATTCTGTATGAAAC

(Primer pair for rpiA)

*Forward (SEQ ID NO:54):

CAATTACCGCAATTGAACAGCGTCGCAATTAAAAGGAGATATACCATGACCCA GGATGAACTGAAAAAAG

*Reverse (SEQ ID NO:55): TTATTTCACAATGGTTTTAACACCATC

(Primer pair for rpiB)

*Forward (SEQ ID NO:56):

AATGAAGAAAGCATTAGCGCCATGTTTGAACATTAAAAGGAGATATACCATGA AAAAAATCGCCTTTGGC

*Reverse (SEQ ID NO:57): TTAATTGCGACGCTGTTC

(Primer pair for prs)

*Forward (SEQ ID NO:58):

ATTCCCCTGTAGAAATAATTTTGTTTAACTTTAATAAGGAGATATACCGTGCCG GATATGAAACTGTTTG

*Reverse (SEQ ID NO:59):
TTAATGTTCAAACATGGCGC

*Construction of pCDF-pgi->prs:

The synthetic genes pgi, zwf, pgl, gnd, rpiA, rpiB, and prs derived from E. coli K12 (SEQ ID NOs: 17, 20, 23, 26, 29, 32, and 35, respectively), codon-optimized for expression in E. coli, were amplified by PCR using the following primer pairs, each containing homologous regions that can be linked to pRSFDuet-1 and, except for pgi, the same RBS regions as that of pCDFDuet-1. First, the fragments of pgi, zwf, pgl, and gnd were linked to pCDFDuet-1, which had been digested with restriction enzymes NcoI and SacI, using the Gibson Assembly system. The resulting vector was then digested with the restriction enzyme SacI, and linked with the remaining fragments of rpiA, rpiB, and prs, using the Gibson Assembly system to produce pCDF-pgi -> prs.

(Primer pair for pgi)

*Forward (SEQ ID NO:60):

TCCCCTGTAGAAATAATTTTGTTTAACTTTAATAAGGAGATATACCATGAAGAA
CATTAATCCGACACAG

*Reverse (SEQ ID NO:61):
TTAACCACGCCAGGCTTTATAAC

(Primer pair for zwf)

*Forward (SEQ ID NO:62):

ATGGTCTGATTAATCGTTATAAAGCCTGGCGTGGTTAAAAGGAGATATACCAT
GGCAGTTACCCAGACCG

*Reverse (SEQ ID NO:63):
TTATTCAAATTCATTCCAGCTACG

(Primer pair for pgl)

*Forward (SEQ ID NO:64):

CCGTGATGGTCGTAGCTGGAATGAATTTGAATAAAAGGAGATATACCATGAAA
CAGACCGTGTATATTGC

*Reverse (SEQ ID NO:65):
TTAATGTGCATTAACAACAACCC

(Primer pair for gnd)

*Forward (SEQ ID NO:66):

TCAGGGTCCGATGTGGGTTGTTGTTAATGCACATTAAAAGGAGATATACCATG AGCAAACAGCAGATTGG

*Reverse (SEQ ID NO:67):

CATTATGCGGCCGCAAGCTTGTCGACCTGCAGGCGCGCCGAGCTCTTAGTCCA GCCATTCTGTATGAAAC

(Primer pair for rpiA)

*Forward (SEQ ID NO:68):

AAGGTGTGTTTCATACAGAATGGCTGGACTAAAAGGAGATATACCATGACCCA GGATGAACTGAAAAAAG

*Reverse (SEQ ID NO:69):
TTATTTCACAATGGTTTTAACACCATC

(Primer pair for rpiB)

*Forward (SEQ ID NO:70):

GGTACACCGGATGGTGTTAAAACCATTGTGAAATAAAAGGAGATATACCATGA AAAAAATCGCCTTTGGC

*Reverse (SEQ ID NO:71):
TTAATTGCGACGCTGTTC

(Primer pair for prs)

*Forward (SEQ ID NO:72):

AAGCAATTACCGCAATTGAACAGCGTCGCAATTAAAAGGAGATATACCGTGCC GGATATGAAACTGTTTG

*Reverse (SEQ ID NO:73):

TCGACTTAAGCATTATGCGGCCGCAAGCTTGTCGACCTGCAGGCGCGCCGTTA ATGTTCAAACATGGCGC

*Construction of pACYC-pgi->prs :

The synthetic genes pgi, zwf, pgl, gnd, rpiA, rpiB, and prs derived from E. coli K12 (SEQ ID NOs: 17, 20, 23, 26, 29, 32, and 35, respectively), codon-optimized for expression in E. coli, were amplified by PCR using the following primer pairs, each containing homologous regions that can be linked to pRSFDuet-1 and, except for pgi, the same RBS regions as that of pACYCDuet-1. First, the fragments of pgi, zwf, pgl, and gnd were linked to pACYCDuet-1, which had

been digested with restriction enzymes NcoI and SacI, using the Gibson Assembly system. The resulting vector was then digested with the restriction enzyme SacI, and linked with the remaining fragments of rpiA, rpiB, and prs, using the Gibson Assembly system to produce pACYC-pgi->prs.

(Primer pair for pgi)

*Forward (SEQ ID NO:60: same as above)
*Reverse (SEQ ID NO:61: same as above)

(Primer pair for zwf)

*Forward (SEQ ID NO:62: same as above)
*Reverse (SEQ ID NO:63: same as above)

(Primer pair for pgl)

*Forward (SEQ ID NO:64: same as above)
*Reverse (SEQ ID NO:65: same as above)

(Primer pair for gnd)

*Forward (SEQ ID NO:66: same as above)
*Reverse (SEQ ID NO:67: same as above)

(Primer pair for rpiA)

*Forward (SEQ ID NO:68: same as above)
*Reverse (SEQ ID NO:69: same as above)

(Primer pair for rpiB)

*Forward (SEQ ID NO:70: same as above)
*Reverse (SEQ ID NO:71: same as above)

(Primer pair for prs)

*Forward (SEQ ID NO:72: same as above)
*Reverse (SEQ ID NO:73: same as above)

<u>*Construction of pACYC-prs->pgi:</u>

The synthetic genes pgi, zwf, pgl, gnd, rpiA, rpiB, and prs derived from E. coli K12 (SEQ ID NOs: 17, 20, 23, 26, 29, 32, and 35, respectively), codon-optimized for expression in E. coli, were amplified by PCR using the following primer pairs, each containing homologous regions that can be linked to pACYCDuet-1 and, except for prs, the same

RBS regions as that of pACYCDuet-1. First, the fragments of prs, rpiB, rpiA, and gnd were linked to pACYCDuet-1, which had been digested with restriction enzymes NcoI and SacI, using the Gibson Assembly system. The resulting vector was then digested with the restriction enzyme SacI, and linked with the remaining fragments of pgl, zwf, and pgi, using the Gibson Assembly system to produce pACYC-prs->pgi.

(Primer pair for pgi)

*Forward (SEQ ID NO:46: same as above)
*Reverse (SEQ ID NO:47: same as above)

(Primer pair for zwf)

*Forward (SEQ ID NO:48: same as above)
*Reverse (SEQ ID NO:49: same as above)

(Primer pair for pgl)

*Forward (SEQ ID NO:50: same as above)
*Reverse (SEQ ID NO:51: same as above)

(Primer pair for gnd)

*Forward (SEQ ID NO:52: same as above)
*Reverse (SEQ ID NO:53: same as above)

(Primer pair for rpiA)

*Forward (SEQ ID NO:54: same as above)
*Reverse (SEQ ID NO:55: same as above)

(Primer pair for rpiB)

*Forward (SEQ ID NO:56: same as above)
*Reverse (SEQ ID NO:57: same as above)

(Primer pair for prs)

*Forward (SEQ ID NO:58: same as above)
*Reverse (SEQ ID NO:59: same as above)

<u>*Construction of pACYC-niaP BC:</u>

The synthetic gene of niaP derived from Burkholderia cenocepacia (SEQ ID NO: 8), codon-optimized for expression in E. coli, was amplified via PCR using the following primer pair, each containing homologous regions that can

be linked to pACYCDuetl digested with restriction enzymes NcoI and EcoRI, respectively. The amplified product was then linked to pACYCDuet-1, which had been digested with restriction enzymes NcoI and EcoRI, using the In-Fusion cloning method to thereby produce pACYC-niaP BC.

(Primer pair for niaP BC)

*Forward (SEQ ID NO:74):
AGGAGATATACCATGCCTGCAGCAACCGCACC

*Reverse (SEQ ID NO:75):
GCTCGAATTCGGATCTTAGCTTGCTTTATCTGCTGCTGTTGCCGGATAAC

*Construction of pACYC-niaX SPT:

The synthetic gene of niaX derived from Streptococcus pneumoniae TIGR4 (SEQ ID NO: 11), codon-optimized for expression in E. coli, was amplified via PCR using the following primer pair, each containing homologous regions that can be linked to pACYCDuetl digested with restriction enzymes NcoI and EcoRI, respectively. The amplified product was then linked to pACYCDuet-1, which had been digested with restriction enzymes NcoI and EcoRI, using the In-Fusion cloning method to thereby produce pACYC-niaX SPT.

(Primer pair for niaX SPT)

*Forward (SEQ ID NO:76):
AGGAGATATACCTTGAGCGGTCTGCTGTATCACACCAGCGTTTATGCAG
*Reverse (SEQ ID NO:77):
GCTCGAATTCGGATCTTAGCGACGTTTACGCAGAACTTTATAAACTGCC

*Construction of pACYC-pnuC BM:

The synthetic gene of pnuC derived from Bacillus mycoides TIGR4 (SEQ ID NO: 14), codon-optimized for expression in E. coli, was amplified via PCR using the following primer pair, each containing homologous regions that can be linked to pACYCDuetl digested with restriction enzymes NcoI and EcoRI, respectively. The amplified product was then linked to pACYCDuet-1, which had been digested with restriction enzymes NcoI and EcoRI, using the In-Fusion cloning method to thereby produce pACYC-pnuC BM.

(Primer pair for pnuC BM)

*Forward (SEQ ID NO:78):
AGGAGATATACCATGGTTCGTAGTCCGCTGTTTCTGCTGATTAGCAGC

*Reverse (SEQ ID NO:79):
GCTCGAATTCGGATCTTAGATGTAGTTGTTCACGCGTTCACGTTCTTTATG

*Construction of pRSF-NAMPT CP+pnuC BM:

The synthetic gene of pnuC derived from Bacillus mycoides TIGR4 (SEQ ID NO: 14), codon-optimized for expression in E. coli, was amplified via PCR using the following primer pair, each containing homologous regions that can be linked to pRSF-NAMPT CP digested with restriction enzymes NcoI and EcoRI, respectively. The amplified product was then linked to pRSF-NAMPT CP, which had been digested with restriction enzymes NcoI and EcoRI, using the In-Fusion cloning method to thereby produce pRSF-NAMPT CP+pnuC BM.

(Primer pair for pnuC BM part2)

*Forward (SEQ ID NO:80):

TATTAGTTAAGTATAAGAAGGAGATATACAATGGTTCGTAGTCCGCTGTTTCTG CTGATTAGCAGC

*Reverse (SEQ ID NO:81):

ATGCTAGTTATTGCTCAGCGGTGGCAGCAGTTAGATGTAGTTGTTCACGCGTTC ACGTTCTTTATG

*Construction of pCDF-pgi->prs+niP BC:

The synthetic gene of niaP derived from Burkholderia cenocepacia (SEQ ID NO: 8), codon-optimized for expression in E. coli, was amplified via PCR using the following primer pair, each containing homologous regions that can be linked to pCDF-pgi->prs digested with restriction enzymes Bg1II and AvrII, respectively. The amplified product was then linked to pCDF-pgi->prs, which had been digested with restriction enzymes Bg1II and AvrII, using the In-Fusion cloning method to thereby produce pCDF-pgi->prs +pnuC BC.

(Primer pair for niaP BC part2)

*Forward (SEQ ID NO:82):
TATTAGTTAAGTATAAGAAGGAGATATACAATGCCTGCAGCAACCGCACC
*Reverse (SEQ ID NO:83):

ATGCTAGTTATTGCTCAGCGGTGGCAGCAGTTAGCTTGCTTTATCTGCTGCTGT TGCCGGATAAC

*Construction of pRSF-NAMPT HS+pnuC BM:

The synthetic gene of pnuC derived from Bacillus mycoides (SEQ ID NO: 14), codon-optimized for expression in E. coli, was amplified via PCR using the following primer pair, each containing homologous regions that can be linked to pRSF-NAMPT HS digested with restriction enzymes Bg1II and AvrII, respectively. The amplified product was then linked to pRSF-NAMPT HS, which had been digested with restriction enzymes Bg1II and AvrII, using the In-Fusion cloning method to thereby produce pRSF-NAMPT HS+pnuC BM.

(Primer pair for pnuC BM part2)

*Forward (SEQ ID NO:80: same as above)
*Reverse (SEQ ID NO:81: same as above)

**[IV. Establishment of strains for productionl**

*Establishment of BL21/pRSF-NAMPT CP strain (Example 1):

The pRSF-NAMPT CP was introduced into the BL21 (DE3) strain via the heat shock method to establish BL21/pRSF-NAMPT CP strain.

*Establishment of BL21/pRSF-NAMPT SSC strain:

The pRSF-NAMPT SSC was introduced into the BL21 (DE3) strain via the heat shock method to establish BL21/pRSF-NAMPT SSC strain.

*Establishment of BL21/pRSF-NAMPT CP/pCDF-prs->pgi strain (Examples 2 and 7):

The pRSF-NAMPT CP and the pCDF-prs->pgi were introduced into the BL21 (DE3) strain via the heat shock method to establish BL21/pRSF-NAMPT CP/pCDF-prs->pgi strain.

*Establishment of BL21/pRSF-NAMPT CP/pCDF-prs->pgi/pACYC-pgi->prs strain (Example 3):

The pRSF-NAMPT CP, the pCDF-prs->pgi, and the pACYC-pgi->prs were introduced into the BL21 (DE3) strain via the heat shock method to establish BL21/pRSF-NAMPT CP/pCDF-prs->pgi/pACYC-pgi->prs strain.

*Establishment of BL21/pRSF-NAMPT CP/pCDF-prs->pgi/pACYC-niaP BC strain (Examples 4 and 8):

The pRSF-NAMPT CP, the pCDF-prs->pgi, and the pACYC-niaP BC were introduced into the BL21 (DE3) strain via the heat shock method to establish BL21/pRSF-NAMPT CP/pCDF-prs->pgi/pACYC-niaP BC strain.

*Establishment of BL21/pRSF-NAMPT CP/pCDF-prs->pgi/pACYC-niaX SPT strain (Examples 5 and 9):

The pRSF-NAMPT CP, the pCDF-prs->pgi, and the pACYC-niaX SPT were introduced into the BL21 (DE3) strain via the heat shock method to establish BL21/pRSF-NAMPT CP/pCDF-prs->pgi/pACYC-niaX SPT strain.

*Establishment of BL21/pRSF-NAMPT CP/pCDF-prs->pgi/pACYC-pnuC BM strain (Examples 6 and 10):

The pRSF-NAMPT CP, the pCDF-prs->pgi, and the pACYC-pnuC BM were introduced into the BL21 (DE3) strain via the heat shock method to establish BL21/pRSF-NAMPT CP/pCDF-prs->pgi/pACYC-pnuC BM strain.

*Establishment of BL21/pRSF-NAMPT CP+pnuC BM/pCDF-pgi->prs+niaP BC/pACYC-prs->pgi strain (Example 11):

The pRSF-NAMPT CP+pnuC BM, the pCDF-pgi->prs+niaP BC, and the pACYC-prs->pgi were introduced into the BL21 (DE3) strain via the heat shock method to establish BL21/pRSF-NAMPT CP+pnuC BM/pCDF-pgi->prs+niaP BC/pACYC-prs->pgi strain.

*Establishment of BL21/pRSF-NAMPT HS strain (Comparative Example 2):

The pRSF-NAMPT HS was introduced into the BL21 (DE3) strain via the heat shock method to establish BL21/pRSF-NAMPT HS strain.

*Establishment of BL21/pRSF-NAMPT HS+pnuC BM/pCDF-pgi->prs+niaP BC/pACYC-prs->pgi strain (Comparative Example 3):

The pRSF-NAMPT HS+pnuC BM, the pCDF-pgi->prs+niaP BC, and the pACYC-prs->pgi were introduced into the BL21 (DE3) strain via the heat shock method to establish BL21/pRSF-NAMPT HS+pnuC BM/pCDF-pgi->prs+niaP BC/pACYC-prs->pgi strain.

**[v-1. Production of nicotinamide mononucleotide (NMN) 11**

*Example 1 (NMN production using the BL21/pRSF-NAMPT CP strain):

The BL21/pRSF-NAMPT CP strain was inoculated into a test tube containing 5 ml of LB medium and incubated at 37°C with 200 rpm for 12 hours. The culture was then inoculated into a 500 ml conical flask containing 200 ml of LB medium to achieve an OD600 of 0.03, and incubated at 37°C with 200 rpm. When the $OD_{600}$ reached 0.4, isopropyl-β-thiogalactopyranoside (Nakalai Tesque Co. Ltd.) was added to achieve a final concentration of 0.1 mM, and incubated at 25°C with 200 rpm for 16 hours. The culture was then transferred to a 50-mL conical tube and centrifuged at 3000g for 5 minutes to collect the bacterial cells. 1×PBS was added to the tube containing the recovered cells for washing,

and the bacterial cells were collected by centrifugation at 3000g for 5 minutes. This procedure was repeated twice. The collected bacteria were suspended in LB medium to achieve an $OD_{600}$ of 10, and 10 mL of the suspension was transferred into a 100-mL conical flask, to which 1 g/L of nicotinamide, 0.4 g/L of $_D$-glucose, and 0.005 mol/L of phosphate buffer (pH 6.2) were added, and the reaction was allowed to run at 30°C with 200 rpm. After 2 hours, the reaction liquid was collected, frozen at -30°C, thawed, and centrifuged at 12,000 rpm for 3 minutes to collect the supernatant. The collected liquid was subjected to HPLC analysis, which revealed that the amount of NMN was 0.03 g/L.

*Example 2 (NMN production using the BL21/pRSF-NAMPT CP/pCDF-prs->pgi strain):

The reaction procedure was carried out in the same manner as in Example 1 except that the BL21/pRSF-NAMPT CP strain was changed to the BL21/pRSF-NAMPT CP/pCDF-prs->pgi strain. The collected liquid was subjected to HPLC analysis, which showed that the amount of NMN was 0.18 g/L.

*Example 3 (NMN production using the BL21/pRSF-NAMPT CP/pCDF-prs->pgi/ pACYC-pgi->prs strain):

The reaction procedure was carried out in the same manner as in Example 1 except that the BL21/pRSF-NAMPT CP strain was changed to the BL21/pRSF-NAMPT CP/pCDF-prs->pgi/pACYC-pgi->prs strain. The collected liquid was subjected to HPLC analysis, which showed that the amount of NMN was 0.22 g/L.

*Example 4 (NMN production using the BL21/pRSF-NAMPT CP/pCDF-prs->pgi/pACYC-niaP BC strain):

The reaction procedure was carried out in the same manner as in Example 1 except that the BL21/pRSF-NAMPT CP strain was changed to the BL21/pRSF-NAMPT CP/pCDF-prs->pgi/pACYC-niaP BC strain. The collected liquid was subjected to HPLC analysis, which showed that the amount of NMN was 0.21 g/L.

*Example 5 (NMN production using the BL21/pRSF-NAMPT CP/pCDF-prs->pgi/pACYC-niaX SPT strain):

The reaction procedure was carried out in the same manner as in Example 1 except that the BL21/pRSF-NAMPT CP strain was changed to the BL21/pRSF-NAMPT CP/pCDF-prs->pgi/pACYC-niaX SPT strain. The collected liquid was subjected to HPLC analysis, which showed that the amount of NMN was 0.23 g/L.

*Example 6 (NMN production using the BL21/pRSF-NAMPT CP/pCDF-prs->pgi/pACYC-pnuC BM strain):

The reaction procedure was carried out in the same manner as in Example 1 except that the BL21/pRSF-NAMPT CP strain was changed to the BL21/pRSF-NAMPT CP/pCDF-prs->pgi/pACYC-pnuC BM strain. The collected liquid was subjected to HPLC analysis, which showed that the amount of NMN was 0.36 g/L.

*Example 7 (NMN production using the BL21/pRSF-NAMPT CP/pCDF-prs->pgi strain):

The reaction procedure was carried out in the same manner as in Example 2 except that the nicotinamide amount was changed from 1g/L to 2g/L, the D-glucose amount from 0.4g/L to 1.0g/L, and the phosphate buffer (pH 6.2) amount from 0.005mol/L to 0.01mol/L. The collected liquid was subjected to HPLC analysis, which showed that the amount of NMN was 0.20 g/L.

*Example 8 (NMN production using the BL21/pRSF-NAMPT CP/pCDF-prs->pgi/ pACYC-niaP BC strain):

The reaction procedure was carried out in the same manner as in Example 7 except that the BL21/pRSF-NAMPT CP/pCDF-prs->pgi strain was changed to the BL21/pRSF-NAMPT CP/pCDF-prs->pgi/pACYC-niaP BC strain. The collected liquid was subjected to HPLC analysis, which showed that the amount of NMN was 0.31 g/L.

*Example 9 (NMN production using the BL21/pRSF-NAMPT CP/pCDF-prs->pgi/ pACYC-niaX SPT strain):

The reaction procedure was carried out in the same manner as in Example 7 except that the BL21/pRSF-NAMPT CP/pCDF-prs->pgi strain was changed to the BL21/pRSF-NAMPT CP/pCDF-prs->pgi/pACYC-niaX SPT strain. The collected liquid was subjected to HPLC analysis, which showed that the amount of NMN was 0.33 g/L.

*Example 10 (NMN production using the BL21/pRSF-NAMPT CP/pCDF-prs->pgi/ pACYC-pnuC BM strain):

The reaction procedure was carried out in the same manner as in Example 6 except that the collected bacteria was suspended in M9 medium instead of LB medium to achieve an $OD_{600}$ of 10. The collected liquid was subjected to HPLC analysis, which showed that the amount of NMN was 0.12 g/L.

*Comparative Example 1 (NMN production using the BL21 (DE3) strain):

The reaction procedure was carried out in the same manner as in Example 1 except that the BL21/pRSF-NAMPT CP strain was changed to the BL21 (DE3) strain. The collected liquid was subjected to HPLC analysis, which showed that the amount of NMN was below the quantification limit.

*Comparative Example 2 (NMN production using the BL21/pRSF-NAMPT HS strain):

The reaction procedure was carried out in the same manner as in Example 1 except that the BL21/pRSF-NAMPT CP strain was changed to the BL21/pRSF-NAMPT HS strain. The collected liquid was subjected to HPLC analysis, which showed that the amount of NMN was below the quantification limit.

**[V-2. Production of nicotinamide mononucleotide (NMN) 2]**

*Example 11 (NMN production using the BL21/pRSF-NAMPT CP+pnuC BM/pCDF-pgi->prs+niaP BC/pACYC-prs->pgi strain):

The procedure for collecting the bacteria was carried out in the same manner as in Example 1 except that the BL21/pRSF-NAMPT CP strain was changed to the BL21/pRSF-NAMPT CP+pnuC BM/pCDF-pgi->prs+niaP BC/pACYC-prs->pgi strain. The collected cells were suspended in M9 medium to achieve an $OD_{600}$ of 40, and 10 mL of the suspension was added to a 100-mL conical flask to make, to which 7 g/L of nicotinamide, 21 g/L of D-glucose, and 0.05 mol/L of phosphate buffer (pH 6.2) were added. The reaction was allowed to run at 30°C and 200 rpm. After 8 hours, the reaction solution was collected, frozen at -30°C, thawed, and centrifuged at 12,000 rpm for 3 minutes to collect the supernatant. The collected liquid was subjected to HPLC analysis, which showed that the amount of NMN was 6.52g/L.

*Comparative Example 3 (NMN production using the BL21/pRSF-NAMPT HS+pnuC BM/pCDF-pgi->prs+niaP BC/pACYC-prs->pgi strain):

The reaction procedure was carried out in the same manner as in Example 1 except that the BL21/pRSF-NAMPT CP+pnuC BM/pCDF-pgi->prs+niaP BC/pACYC-prs->pgi strain was changed to the BL21/pRSF-NAMPT HS+pnuC BM/pCDF-pgi->prs+niaP BC/pACYC-prs->pgi strain. The collected liquid was subjected to HPLC analysis, which showed that the amount of NMN was 0.04g/L.

**[VI. Purification of nicotinamide mononucleotide (NMN) 1]**

500 mL of pretreated LB medium containing NMN was subjected to membrane concentration by filtering the liquid with a NF membrane (SYNDER, NF-S) for 2 hours with stirring at 400 rpm to achieve a final volume of 50 mL. The resulting concentrate was lyophilized overnight to achieve 6.3 g of NMN-containing crude. The obtained crude was dissolved in 15 mL of miliQ water, and after filtration with a 0.22 μm filter, the filtrate was subjected to preparative HPLC to separate an NMN-containing fraction (purity: 64.56%). The NMN-containing fraction was lyophilized again, and then subjected to preparative HPLC. The resulting high NMN content fraction was adjusted to pH=3 with 1N HCl, and lyophilized to obtain NMN (purity: >99%).
MS(ESI) :m/z 335[M+H]+ $^1$H-NMR ($D_2O$) δ: 9.49 (1H, s), 9.30 (1H, d, J = 6.4 Hz), 9.00 (1H, d, J = 7.8 Hz) 8.31 (1H, dd, J = 7.8, 6.4), 6.32 (1H, d, J = 5.0 Hz), 4.79-4.65 (1H, m), 4.59 (1H, t, J = 5.0 Hz), 4.47-4.45 (1H, m)4.34-4.30 (1H, m), 4.18-4.13 (1H, m).

*Preparation conditions:

| | |
|---|---|
| Instrument: | Agilent Infinity 1200 Preparative HPLC |
| Solvent: | A = 100% $H_2O$ + 0.1% $CH_3COOH$ |
| | B = 95% MeCN/5% $H_2O$ + 0.1% $CH_3COOH$ |
| Column: | zic-HILIC, 21.2 mm I.D. x 150 mm, 5 μm, two columns connected |

(continued)

| | | |
|---|---|---|
| Guard column: | InertSustain Amide, 7.6 mm I.D. x 30 mm | |
| Column temperature: | RT | |
| Flow rate: | 22.0 mL/min | |
| Detection wavelength: 260, 200 nm (PDA) (UV triggered preparative at 260 nm) | | |
| Gradient conditions: | Time (min) | B solv. (%) |
| | 0.00 | 84.00 |
| | 50.0 | 84.00 |
| | 50.1 | 45.00 |
| | 58.0 | 45.00 |
| | 58.1 | 84.00 |
| | 65.0 | STOP |
| Fraction volume: | 8.0 mL | |

*Analysis conditions:

| | | | |
|---|---|---|---|
| Instrument: | Shimadzu IT-TOF/MS | | |
| Solvent: | A = 100% $H_2O$ + 0.1% $CH_3COOH$ | | |
| | B = 95% MeCN/5% $H_2O$ + 0.1% $CH_3COOH$ | | |
| Column: | zic-HILIC, 4.6mmI.D. × 150mm, 5.0μm | | |
| Column temperature: | 25°C | | |
| Flow rate: | 1.2mL/min | | |
| Wavelengths: | 200 nm, 260 nm (PDA) | | |
| Gradient conditions: | Time (min) | B solv. (%) | |
| | 0.00 | 90.00 | |
| | 4.0 | 90.00 | |
| | 21.0 | 50.00 | |
| | 25.0 | 50.00 | |
| | 25.1 | 90.00 | |
| | 30.0 | STOP | |
| Neplaizer gas flow rate: | 1.5 mL/min. | | |
| CDL temperature: | 200°C | | |
| Heat block temperature: | 200°C | | |
| Detector voltage: | 1.65 kV | | |
| MS detection range: | Event1 | MS | 100 to 600 |
| | Event2 | MS/MS | 70 to 500 |

**[VII. Reference evaluations]**

*Evaluation of NAMPT conversion efficiency:

The BL21/pRSF-NAMPT CP strain was inoculated into a test tube containing 5 ml of LB medium and incubated at 37°C with 200 rpm for 12 hours, and the culture was then inoculated into a 500 ml conical flask containing 200 ml of LB medium to achieve an OD600 of 0.03, and incubated at 37°C with 200 rpm. When the $OD_{600}$ reached 0.4, isopropyl-β-thiogalactopyranoside was added to achieve a final concentration of 0.1 mM, and the culture was incubated at 25°C with 200 rpm for 16 hours. 30 mL of the culture was transferred to a 50 mL conical tube, centrifuged at 3000 g for 5 min, and the bacteria were collected. 1×PBS was added to the tube containing the recovered cells for washing, and centrifuged at 3000g for 5 minutes to collect the remaining bacteria. This procedure was repeated twice. The collected bacteria were suspended in 15 mL of Cell Lysis Buffer (MBL Co., Ltd.), and the lysate was prepared according to a generally recommended method. The $OD_{595}$ of the lysate was measured using the Protein Assay Bradford reagent (Wako Pure Chemical Co., Ltd.), and the lysate solution was diluted with water to achieve an $OD_{595}$ of 0.1. This diluted solution was used as NAMPT solution, and the NAMPT conversion efficiency was measured according to the One-Step Assay Method of CycLexR NAMPT Colorimetric Assay Kit Ver. 2 (MBL). A SpectraMaxR iD3 multimode microplate reader (Molecular

Devices) was used for the measurement. The result showed that the NAMPT conversion efficiency was 230.

The NAMPT conversion efficiency was also measured in the same manner as mentioned above except that the BL21/pRSF-NAMPT CP strain was changed to the BL21/pRSF-NAMPT SSC strain. The result showed that the NAMPT conversion efficiency was 170.

As a comparison, the NAMPT conversion efficiency was measured in the same manner as mentioned above except that the BL21/pRSF-NAMPT CP strain was changed to the BL21 (DE3) strain. The result showed that the NAMPT conversion efficiency was 9.

The NAMPT conversion efficiency of human NAMPT (from CycLexR NAMPT Colorimetric Assay Kit Ver. 2 (MBL)) was also measured by diluting the human NAMPT with water to an $OD_{595}$ of 0.1, and the resulting diluted solution was used as NAMPT solution to measure NAMPT conversion efficiency according to the One-Step Assay Method of CycLexR NAMPT Colorimetric Assay Kit Ver. 2 (MBL). The result showed that the NAMPT conversion efficiency was 22.

*Evaluation of nicotinamide uptake efficiency by niaP:

The BL21/pRSF-NAMPT CP/pCDF-prs->pgi/pACYC-niaP BC strain was inoculated into a test tube containing 5 mL of LB medium, and incubated at 37°C with 200 rpm for 12 hours. When the $OD_{600}$ reached 0.4, isopropyl-β-thiogalactopyranoside was added to achieve a final concentration of 0.1 mM, and incubated at 25°C with 200 rpm for 16 hours. The culture was then transferred to a 50-mL conical tube and centrifuged at 3000g for 5 minutes to collect the bacteria. 1×PBS was added to the tube containing the recovered cells for washing, and centrifuged at 3000g for 5 minutes to collect the remaining bacteria. This procedure was repeated twice. The collected bacteria were suspended in LB medium to achieve an $OD_{600}$ of 10, and 10 mL of the suspension was transferred to a 100-mL conical flask, to which 1 g/L of nicotinamide, 0.4 g/L of D-glucose, and 0.005 mol/L of phosphate buffer (pH 6.2) were added. The reaction was allowed to run at 30°C with 200 rpm. The reaction solution was collected after 1 hour and 2 hours of reaction, and the collected solutions were frozen at -30°C, thawed, and centrifuged at 12,000 rpm for 3 minutes to collect the supernatant. These collected liquids were analyzed by HPLC to quantify the amount of NMN. The result showed that the nicotinamide uptake efficiency of niaP was 81%.

The evaluation procedure was carried out in the same manner except that the BL21/pRSF-NAMPT CP/pCDF-prs->pgi strain was used. The result showed that the nicotinamide uptake efficiency of niaP was 66%.

*Evaluation of nicotinamide mononucleotide excretion efficiency by pnuC:

The BL21/pRSF-NAMPT CP/pCDF-prs->pgi/pACYC-pnuC BM strain was inoculated into a test tube containing 5 mL of LB medium and incubated at 37°C with 200 rpm for 12 hours. The culture was inoculated into a 500 ml conical flask containing 200 ml of LB medium to achieve an $OD_{600}$ of 0.03, and incubated at 37°C at 200 rpm. When the $OD_{600}$ reached 0.4, isopropyl-β-thiogalactopyranoside was added to achieve a final concentration of 0.1 mM, and incubation was continued at 25°C with 200 rpm for 16 hours. The culture was then transferred to a 50-mL conical tube and centrifuged at 3000g for 5 minutes to collect the bacteria. 1×PBS was added to the tube containing the recovered cells for washing, and centrifuged at 3000g for 5 minutes to collect the remaining bacteria. This procedure was repeated twice. The collected bacteria were suspended in LB medium to achieve an $OD_{600}$ of 10, and 10 mL of the suspension was transferred to a 100-mL conical flask, to which 1 g/L of nicotinamide, 0.4 g/L of D-glucose, and 0.005 mol/L of phosphate buffer (pH 6.2) were added. The reaction was allowed to run at 30°C with 200 rpm. Two hours after the reaction, two samples of the reaction solution were collected. One was frozen at -30°C, thawed, and centrifuged at 12,000 rpm for 3 minutes to collect the supernatant. The other was not frozen but was directly subjected to centrifugation at 12,000 rpm for 3 minutes to collect the supernatant. These collected liquids were analyzed by HPLC to quantify the amount of NMN. The results showed that the nicotinamide mononucleotide excretion efficiency by pnuC was 81%.

The evaluation procedure was also carried out in the same manner except that the BL21/pRSF-NAMPT CP/pCDF-prs->pgi strain was used. The results showed that the nicotinamide mononucleotide excretion efficiency by pnuC was 11%.

*Production of NAm derivatives other than NMN:

A sample of the reaction solution containing NMN obtained after 8 hours of reaction in Example 11 was combined with adenosine triphosphate (ATP) and nicotinamide mononucleotide adenylyltransferase 1 (NMNAT1) (ATP in CycLexR NAMPT Colorimetric Assay Kit Ver. 2 (MBL) was used), and the reaction was allowed to run at 30°C, whereby the formation of $NAD^+$ was confirmed. To this sample, alcohol dehydrogenase (ADH) and ethanol (using ADH and ethanol from CycLexR NAMPT Colorimetric Assay Kit Ver. 2 (MBL)) were also added, and the reaction was allowed to run at 30°C, whereby the formation of NADH was confirmed.

*Purification of nicotinamide mononucleotide (NMN) 2:

The LB medium containing NMN, from which the bacteria was removed via centrifugation, was treated with activated carbon, and the treated solution was separated from the activated carbon. The treated solution was then subjected to NF membrane filtration to removing macromolecular impurities, and the filtrate was treated with ion exchange resin to further remove impurities. The resulting solution was concentrated with an NF membrane, and further centrifuged to produce an NMN-containing concentrate. 5 mol/L aqueous hydrochloric acid was added to the NMN-containing concentrate to adjust the pH to within the range of from 3 to 4, and the recrystallization was caused by adding an appropriate amount of ethanol. The precipitated solid was collected as NMN crystals in high purity (HPLC purity > 95%).

## INDUSTRIAL APPLICABILITY

The present invention allows for efficient production of NAm derivatives such as NMN, which is especially useful as various research tools, synthetic intermediates of NAD, and even as pharmaceutical ingredients. Therefore, the present invention has high industrial value.

## Claims

1. A recombinant microorganism for producing a nicotinamide derivative, wherein the microorganism has been engineered to express a nicotinamide phosphoribosyl transferase (NAMPT), which converts nicotinamide and phosphoribosyl pyrophosphate into nicotinamide mononucleotide, and/or has been transformed with a vector carrying a nucleic acid encoding the amino acid sequence of the NAMPT, wherein the conversion efficiency of the NAMPT from nicotinamide to nicotinamide mononucleotide is five times or more of the conversion efficiency of a human NAMPT.

2. The recombinant microorganism according to claim 1, wherein the NAMPT is composed of a polypeptide with a sequence homology of 80% or more with the amino acid sequence represented by SEQ ID NO:3 or SEQ ID NO:6.

3. The recombinant microorganism according to claim 1 or 2, wherein the microorganism has been engineered to express a niacin transporter, which promotes cellular uptake of nicotinic acid and/or nicotinamide, and/or has been transformed with a vector carrying a nucleic acid encoding the amino acid sequence of the niacin transporter, wherein the niacin transporter increases the intracellular uptake efficiency of nicotinic acid and/or nicotinamide by the host microorganism by 1.1 times or more.

4. The recombinant microorganism according to claim 3, wherein the niacin transporter is composed of a polypeptide with a sequence homology of 80% or more with the amino acid sequence represented by SEQ ID NO:9 or SEQ ID NO: 12.

5. The recombinant microorganism according to any one of claims 1 to 4, wherein the microorganism has been engineered to express a nicotinamide derivative transporter, which promotes extracellular excretion of a nicotinamide derivative and/or has been transformed with a vector carrying a nucleic acid encoding the amino acid sequence of the nicotinamide derivative transporter, wherein the nicotinamide derivative transporter increases extracellular excretion efficiency of the nicotinamide derivative by the host microorganism by 3 times or more.

6. The recombinant microorganism according to claim 5, wherein the nicotinamide derivative transporter is composed of a polypeptide with a sequence homology of 80% or more with the amino acid sequence represented by SEQ ID NO:15.

7. The recombinant microorganism according to any one of claims 1 to 6, wherein the microorganism has been engineered to express one or more enzymes which promote a synthetic pathway from glucose-6-phosphoric acid to

phosphoribosyl pyrophosphate and/or has been transformed with a vector carrying a nucleic acid encoding the amino acid sequence of the one or more enzymes.

**8.** The recombinant microorganism according to claim 7, wherein the one or more enzymes are selected from the group consisting of phosphoglucose isomerase, glucose-6-phosphate dehydrogenase, 6-phosphogluconolactonase, 6-phosphogluconate dehydrogenase, ribose-5-phosphate isomerase, and phosphoribosyl pyrophosphate synthase.

**9.** The recombinant microorganism according to claim 8, wherein

the phosphoglucose isomerase is a polypeptide with a sequence homology of 80% or more with the amino acid sequence represented by SEQ ID NO:18,
the glucose-6-phosphate dehydrogenase is a polypeptide with a sequence homology of 80% or more with the amino acid sequence represented by SEQ ID NO:21,
the 6-phosphogluconolactonase is a polypeptide with a sequence homology of 80% or more with the amino acid sequence represented by SEQ ID NO:24,
the 6-phosphogluconate dehydrogenase is a polypeptide with a sequence homology of 80% or more with the amino acid sequence represented by SEQ ID NO:27,
the ribose-5-phosphate isomerase is a polypeptide with a sequence homology of 80% or more with the amino acid sequence represented by SEQ ID NO:30 or SEQ ID NO33, and
the phosphoribosyl pyrophosphate synthase is a polypeptide with a sequence homology of 80% or more with the amino acid sequence represented by SEQ ID NO:36.

**10.** The recombinant microorganism according to any one of claims 1 to 9, wherein the nicotinamide derivative is selected from the group consisting of nicotinamide mononucleotide, nicotinamide adenine dinucleotide, nicotinamide riboside, nicotinate mononucleotide, nicotinamide adenine dinucleotide phosphoric acid, and nicotinate adenine dinucleotide.

**11.** The recombinant microorganism according to any one of claims 1 to 10, wherein the microorganism is E. coli or a yeast.

**12.** A method for producing a nicotinamide derivative, comprising:

providing nicotinamide to a recombinant microorganism according to any one of claims 1 to 11; and
recovering the nicotinamide derivative produced by the microorganism.

**13.** The method according to claim 12, further comprising purifying the recovered nicotinamide derivative.

**14.** A vector carrying a nucleic acid encoding the amino acid sequence of nicotinamide phosphoribosyl transferase (NAMPT), which converts nicotinamide and phosphoribosyl pyrophosphate into nicotinamide mononucleotide, wherein the nucleic acid comprises a base sequence with a sequence identity of 80% or more with the base sequence represented by SEQ ID NO:2 or SEQ ID NO:5.

**15.** A vector carrying a nucleic acid encoding the amino acid sequence of a niacin transporter, which promotes cellular uptake of nicotinic acid and/or nicotinamide, wherein the nucleic acid comprises a base sequence with a sequence identity of 80% or more with the base sequence represented by SEQ ID NO:8 or SEQ ID N0:11.

**16.** A vector carrying a nucleic acid encoding the amino acid sequence of a nicotinamide derivative transporter, which promotes extracellular excretion of a nicotinamide derivative, wherein the nucleic acid comprises a base sequence with a sequence identity of 80% or more with the base sequence represented by SEQ ID NO:14.

Figure 1

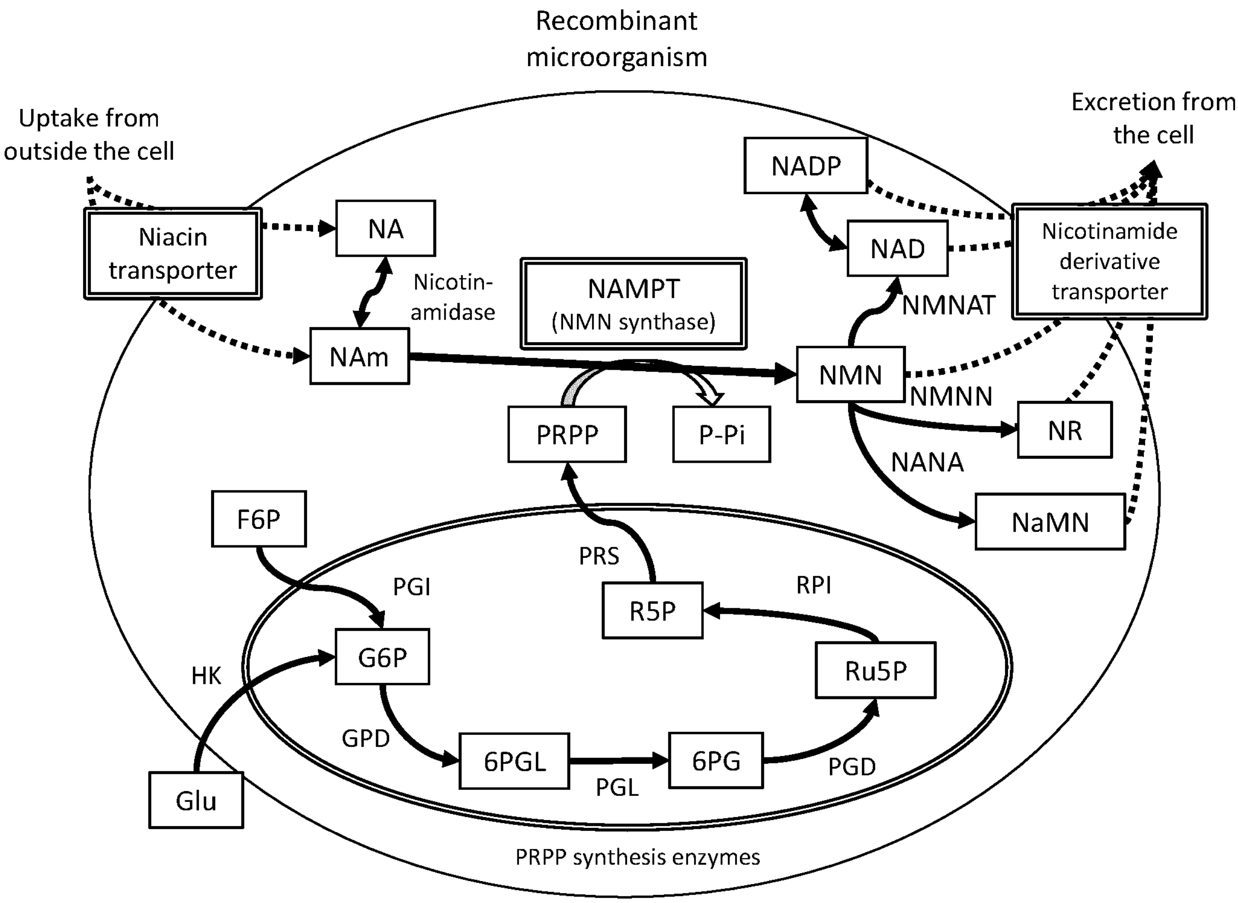
Recombinant
microorganism
Uptake from
outside the cell
Excretion from
the cell
Niacin
transporter
NA
Nicotin-
amidase
NAm
NAMPT
(NMN synthase)
NADP
NAD
NMNAT
Nicotinamide
derivative
transporter
NMN
NMNN
NR
NANA
NaMN
PRPP
P-Pi
F6P
PRS
PGI
RPI
R5P
G6P
Ru5P
HK
GPD
6PGL
6PG
PGD
PGL
Glu
PRPP synthesis enzymes

**INTERNATIONAL SEARCH REPORT**

International application No. PCT/JP2019/049479

A. CLASSIFICATION OF SUBJECT MATTER
C12P 19/30(2006.01)i; C12P 19/32(2006.01)i; C12P 19/36(2006.01)i; C12N 1/19(2006.01)i; C12 N1/21(2006.01)i; C12N 15/53(2006.01)n; C12N 15/54(2006.01)n; C12N 15/56(2006.01)n; C12N 15/61(2006.01)i; C12N 15/70(2006.01)i; C12N 15/81(2006.01)i
FI: C12N15/70 Z ZNA; C12N15/81 Z; C12N15/61; C12N1/21; C12N1/19; C12P19/30; C12P19/32 Z; C12P19/36; C12N15/54; C12N15/53; C12N15/56

According to International Patent Classification (IPC) or to both national classification and IPC

B. FIELDS SEARCHED

Minimum documentation searched (classification system followed by classification symbols)
C12P19/30; C12P19/32; C12P19/36; C12N1/19; C12N1/21; C12N15/53; C12N15/54; C12N15/56; C12N15/61; C12N15/70; C12N15/81

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
JSTPlus/JMEDPlus/JST7580 (JDreamIII);
CAplus/REGSTRY/MEDLINE/EMBASE/BIOSIS/WPIDS (STN);
GenBank/EMBL/DDBJ/GeneSeq/UniProt

C. DOCUMENTS CONSIDERED TO BE RELEVANT

| Category* | Citation of document, with indication, where appropriate, of the relevant passages | Relevant to claim No. |
|---|---|---|
| X<br>A | Database UniProtKB/TrEMBL, [online], Accession No. AOAOM9TZ33, 28 February 2018, [retrieval date 11 March 2020], <https://www.uniprot.org/uniprot/AOAOM9TZ33.txtversion=12>, OKANO, K. et al., Definition: Nicotinamide phosphoribosyltransferase page 1 | 14<br>1-13, 15-16 |
| X<br>A | Database UniProtKB/TrEMBL, [online], Accession No. C7PHC6, 07 November 2018, [retrieval date 11 March 2020], <https://www.uniprot.org/uniprot/C7PHC6.txt?version=47>, LUCAS, S. et al., Definition: Nicotinamide phosphoribosyltransferase pp. 1-2 | 14<br>1-13, 15-16 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search
11 March 2020 (11.03.2020)

Date of mailing of the international search report
24 March 2020 (24.03.2020)

Name and mailing address of the ISA/
Japan Patent Office
3-4-3, Kasumigaseki, Chiyoda-ku,
Tokyo 100-8915, Japan

Authorized officer

Telephone No.

Form PCT/ISA/210 (second sheet) (January 2015)

**INTERNATIONAL SEARCH REPORT**

International application No. PCT/JP2019/049479

C (Continuation). DOCUMENTS CONSIDERED TO BE RELEVANT

| Category* | Citation of document, with indication, where appropriate, of the relevant passages | Relevant to claim No. |
|---|---|---|
| X<br>A | Database GenBank, [online], Accession No. AI045569, 01 December 2014, [retrieval date 11 March 2020], <https://www.ncbi.nlm.nih.gov/protein/AI045569.1/>, DALIGAULT, H. E. et al., Definition: sugar (and other) transporter family protein [Burkholderia cepacia] page 1 | 15<br>1-14, 16 |
| X<br>A | Database GenBank, [online], Accession No. AAK75339, 31 January 2014, [retrieval date 11 March 2020], <https://www.ncbi.nlm.nih.gov/protein/AAK75339.1/>, TETTELIN, H. et al., Definition: hypothetical protein SP_1233 [Streptococcus pneumoniae TIGR4] page 1 | 15<br>1-14, 16 |
| X<br>A | Database UniProtKB/TrEMBL, [online], Accession No. AOAOB5SFH8, 22 November 2017 [retrieval date 11 March 2020], <https://www.uniprot.org/uniprot/AOAOB5SFH8.txt?version=11>, JOHNSON, S. L. et al., Definition: Nicotinamide mononucleotide transporter PnuC family protein page 1 | 16<br>1-15 |
| X<br>A | Database UniProtKB/TrEMBL, [online], Accession No. AOA2BOTGM2, 28 February 2018, [retrieval date 11 March 2020], <https://www.uniprot.org/uniprot/AOA2BOTGM2.txt?version=3>, BLEICH, R. M. et al., Definition: Nicotinamide riboside transporter PnuC page 1 | 16<br>1-15 |
| Y<br>A | MARINESCU, G. C. et al., "ß-nicotinamide mononucleotide (NMN) production in Escherichia coli", SCIENTIFIC REPORTS, 16 August 2018, 8: 12278 abstract, page 2, paragraphs [0003]-[0004], fig. 2, page 5, paragraph [0002] to page 7, paragraph [0003], etc. | 1, 7-13<br>2-6, 14-16 |
| Y<br>A | US 2016/0287621 A1 (PRESIDENT AND FELLOWS OF HARVARD COLLEGE) 06.10.2016 (2016-10-06) paragraphs [0002], [0004]-[0007], [0010], [0039], examples | 1, 7-13<br>2-6, 14-16 |
| X<br>Y<br>A | US 2018/0162895 A1 (BONTAC BIO-ENGINEERING (SHENZHEN) CO., LTD.) 14.01.2018 (2018-01-14) paragraphs [0006]-[0008], [0022]-[0024], [0027], [0035], example 6 | 1, 10-11<br>1, 7-13<br>2-6, 14-16 |
| P, A | WO 2019/065876 A1 (MITSUBISHI CHEMICAL CORPORATION) 04.04.2019 (2019-04-04) claims, paragraphs [0037], [0122], example 5 | 1-16 |

Form PCT/ISA/210 (continuation of second sheet) (January 2015)

**INTERNATIONAL SEARCH REPORT**
Information on patent family members

International application No.
PCT/JP2019/049479

| Patent Documents referred in the Report | Publication Date | Patent Family | Publication Date |
|---|---|---|---|
| US 2016/0287621 A1 | 06 Oct. 2016 | WO 2015/069860 A1 | |
| US 2018/0162895 A1 | 14 Jan. 2018 | WO 2017/185549 A1<br>CN 108026130 A | |
| WO 2019/065876 A1 | 04 Apr. 2019 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20180291054 A
- WO 2017200050 A
- WO 2015069860 A


### Non-patent literature cited in the description


- **MARIESCU et al.** *Scientific Reports,* 16 August 2018, vol. 8 (1), 12278
- **MARIESCU et al.** *Scientific reports,* 16 August 2018, vol. 8 (1), 12278
- **SAMBROOK, J. et al.** Molecular Cloning: A Laboratory Manual. Cold Spring Harbor Laboratory Press, 1989
- **RICE et al.** EMBOSS: The European Molecular Biology Open Software Suite. *Trends Genet.,* 2000, vol. 16, 276-277
- **NEEDLEMAN ; WUNSCH.** *J. Mol. Biol.,* 1970, vol. 48, 443-453

SEQUENCE LISTING

<110> TEIJIN LITITED
SYNART CO., LTD.

<120> RECOMBINANT MICROBES AND METHODS FOR PRODUCING NICOTINAMIDE DERIVATIVES AS WELL AS VECTORS USED THEREFOR

<130> P190716WO

<160> 83

<170> PatentIn version 3.5

<210> 1 Nicotinamide phosphoribosyltransferase SSC
<211> 1386
<212> DNA
<213> Sphingopyxis sp. C-1

<400> 1
atgaaaaacc tgatcctggc gaccgacagc tacaagcaga gccactttct gcaatatccg 60

cccgaggcgc gcgtaatcag cgcctatgtc gaggcgcggc caaacccctt ttccgaagag 120

attgtctttc tgggtctcca gccgctgctg gtcgactatt tcagccagcc gatcaacgcg 180

gcggatatcg acgaggccga ggcgatctgc atcgcgcacg gcgttccgtt caaccgtgcg 240

gggtgggagg cgatcgtcgc cgatcatggc ggttatctgc cgctggagat caaggcgctg 300

cccgaaggcg cgatcgtgcc cgcgggcgtg ccgctggtac agctcgaaaa taccgacccg 360

cgcatgccct ggctgacgac cttcatagag acggcaatgc tgcgtgcgat ctggtatccg 420

acgacggtcg cgacgctgag ctggaagtgc aaacaggtga tccgcgcggg gctcgaaaag 480

acgtccgacg atgtggaggg ccagcttccc ttcaagctcc acgatttcgg cgcgcgcggg 540

gtttcttccg ccgaaagtgc gggtctgggt gggctcgcac atctcgtcaa tttccagggc 600

accgacacga tggaggcgct ggtcgcggcg cggcgctact atggcgccga catggcgggc 660

ttttctattc ccgccgccga gcacagtaca atgacgagct ggggccgcga ccgtgaagag 720

gatgcctatc gcaacatgct cgaccggttc gaaggcgagg gacgcatcgt cgcggtcgtc 780

tccgacagct atgatctcga tacggcggtc accgacatct ggggcggcag cttgcgcgag 840

aaggtgctgg ggcgcgcggg cacgctggtc gtacggcccg acagcggcga tccgatcgaa 900

acgccgctgc gcacggtgaa aacgctgtgg gaaaagtttg gcggccatgt gaacggcaag 960

ggctatcgcg tcctcgatcc gcatgtccgc gtgatccagg gcgacggaat gaccgtcgac 1020

agcatcggcc ggcttgttca gcggatgatc gaggaaggtt tcgcgatcga caatatcgct 1080

ttcggcatgg gcggcgggat gctgcagcac gtcaaccgcg acacgctgcg cttcgcgatg 1140

aaggcgaatg cgatgctggg cagcgacggc gtgtggcacg atgtcttcaa gatgccgagt 1200

accgatccgg gcaaggcgag caaggccgga cggcaggccg tcgtgctgaa ggacggccgg 1260

atggccgcag cgcggctcga cagcgtcgcg gtgggcgaag atctgctggt gccggtatgg 1320

cggaatggcg aactactcgt ccgccacgac ttcgacgcgg tgcggaagcg gtccgaaggc 1380

aggtga 1386

<210> 2 Nicotinamide phosphoribosyltransferase SSC (optimized)
<211> 1386
<212> DNA
<213> Artificial Sequence

<220>
<223> Nicotinamide phosphoribosyltransferase SSC (optimized)

<400> 2
atgaagaatc tgattctggc caccgatagc tataaacaga gccattttct gcagtatccg 60

cctgaagcac gtgttattag cgcatatgtt gaagcccgtc cgaatccgtt tagcgaagaa 120

attgtttttc tgggtctgca gccgctgctg gttgattatt tcagccagcc gattaatgca 180

gccgatattg atgaagcaga agccatttgt attgcacatg gtgttccgtt taatcgtgca 240

ggttgggaag caattgttgc agatcatggt ggttatctgc cgctggaaat taaagcactg 300

ccggaaggtg caattgttcc ggcaggcgtt ccgctggttc agctggaaaa taccgatccg 360

cgtatgccgt ggctgaccac ctttattgaa accgcaatgc tgcgtgcaat ttggtatccg 420

accaccgttg caaccctgag ctggaaatgc aaacaggtta ttcgtgccgg tctggaaaaa 480

accagtgatg atgttgaagg tcagctgccg tttaaactgc atgattttgg tgcacgtggt 540

gttagcagcg cagaaagcgc aggtttaggt ggtctggcac atctggttaa ttttcagggc 600

accgatacca tggaagcact ggttgcagcc cgtcgttatt atggtgcaga tatggcaggt 660

tttagcattc cggcagcaga acatagcacc atgaccagct ggggtcgtga tcgtgaagaa 720

gatgcatatc gtaatatgct ggatcgcttt gaaggtgaag gtcgtattgt tgccgttgtt 780

agcgatagtt atgatctgga taccgcagtt accgatattt ggggtggtag cctgcgtgaa 840

aaagttctgg gtcgtgcggg tacactggtt gttcgtccgg atagcggtga tccgattgaa 900

acaccgctgc gtaccgttaa aaccctgtgg gaaaaatttg gtggtcatgt gaatggtaaa 960

ggttatcgtg ttctggatcc gcatgttcgt gttattcaag gtgatggtat gaccgttgat 1020

agcattggtc gtctggtgca gcgtatgatt gaagaaggtt ttgccattga taacattgcc 1080

tttggtatgg gtggtggtat gctgcaacat gttaatcgtg ataccctgcg ttttgcaatg 1140

aaagcaaatg caatgctggg tagtgatggt gtttggcatg atgtgtttaa aatgccgagc 1200

accgatccgg gtaaagcaag caaagcaggt cgtcaggcag ttgttctgaa agatggtcgt 1260

atggcagcag cacgtctgga tagcgttgca gttggtgaag atctgctggt tccggtttgg 1320

cgtaatggtg aactgctggt gcgtcacgat tttgatgcag ttcgtaaacg tagcgaaggt 1380

cgttaa 1386

```
<210>  3   Nicotinamide phosphoribosyltransferase SSC
<211>  461
<212>  PRT
<213>  Sphingopyxis sp. C-1

<400>  3

Met Lys Asn Leu Ile Leu Ala Thr Asp Ser Tyr Lys Gln Ser His Phe
1               5                   10                  15

Leu Gln Tyr Pro Pro Glu Ala Arg Val Ile Ser Ala Tyr Val Glu Ala
            20                  25                  30

Arg Pro Asn Pro Phe Ser Glu Glu Ile Val Phe Leu Gly Leu Gln Pro
        35                  40                  45

Leu Leu Val Asp Tyr Phe Ser Gln Pro Ile Asn Ala Ala Asp Ile Asp
    50                  55                  60

Glu Ala Glu Ala Ile Cys Ile Ala His Gly Val Pro Phe Asn Arg Ala
65                  70                  75                  80

Gly Trp Glu Ala Ile Val Ala Asp His Gly Gly Tyr Leu Pro Leu Glu
                85                  90                  95

Ile Lys Ala Leu Pro Glu Gly Ala Ile Val Pro Ala Gly Val Pro Leu
            100                 105                 110

Val Gln Leu Glu Asn Thr Asp Pro Arg Met Pro Trp Leu Thr Thr Phe
        115                 120                 125

Ile Glu Thr Ala Met Leu Arg Ala Ile Trp Tyr Pro Thr Thr Val Ala
    130                 135                 140

Thr Leu Ser Trp Lys Cys Lys Gln Val Ile Arg Ala Gly Leu Glu Lys
145                 150                 155                 160

Thr Ser Asp Asp Val Glu Gly Gln Leu Pro Phe Lys Leu His Asp Phe
                165                 170                 175

Gly Ala Arg Gly Val Ser Ser Ala Glu Ser Ala Gly Leu Gly Gly Leu
            180                 185                 190

Ala His Leu Val Asn Phe Gln Gly Thr Asp Thr Met Glu Ala Leu Val
        195                 200                 205

Ala Ala Arg Arg Tyr Tyr Gly Ala Asp Met Ala Gly Phe Ser Ile Pro
    210                 215                 220
```

Ala Ala Glu His Ser Thr Met Thr Ser Trp Gly Arg Asp Arg Glu Glu
225 230 235 240

Asp Ala Tyr Arg Asn Met Leu Asp Arg Phe Glu Gly Glu Gly Arg Ile
245 250 255

Val Ala Val Val Ser Asp Ser Tyr Asp Leu Asp Thr Ala Val Thr Asp
260 265 270

Ile Trp Gly Gly Ser Leu Arg Glu Lys Val Leu Gly Arg Ala Gly Thr
275 280 285

Leu Val Val Arg Pro Asp Ser Gly Asp Pro Ile Glu Thr Pro Leu Arg
290 295 300

Thr Val Lys Thr Leu Trp Glu Lys Phe Gly Gly His Val Asn Gly Lys
305 310 315 320

Gly Tyr Arg Val Leu Asp Pro His Val Arg Val Ile Gln Gly Asp Gly
325 330 335

Met Thr Val Asp Ser Ile Gly Arg Leu Val Gln Arg Met Ile Glu Glu
340 345 350

Gly Phe Ala Ile Asp Asn Ile Ala Phe Gly Met Gly Gly Gly Met Leu
355 360 365

Gln His Val Asn Arg Asp Thr Leu Arg Phe Ala Met Lys Ala Asn Ala
370 375 380

Met Leu Gly Ser Asp Gly Val Trp His Asp Val Phe Lys Met Pro Ser
385 390 395 400

Thr Asp Pro Gly Lys Ala Ser Lys Ala Gly Arg Gln Ala Val Val Leu
405 410 415

Lys Asp Gly Arg Met Ala Ala Ala Arg Leu Asp Ser Val Ala Val Gly
420 425 430

Glu Asp Leu Leu Val Pro Val Trp Arg Asn Gly Glu Leu Leu Val Arg
435 440 445

His Asp Phe Asp Ala Val Arg Lys Arg Ser Glu Gly Arg
450 455 460

<210> 4 Nicotinamide phosphoribosyltransferase CP
<211> 1416

<212> DNA
<213> Chitinophaga pinensis

<400> 4
atgacaaagg aaaacctcat tttgttagct gatgcctaca aatactccca ccacaaactt 60
tacatccccg gtacagaata tatctattct tatttcgaaa gcagaggcgg taaattcaat 120
gaaaccgtct tctacggact ccagtatttc ctgatggaat acctccaggg tgctgttatc 180
accaaagaaa aacttgacga agcagaagct accttgctgg aagtcttcgg tcgtaacgat 240
gtattcgacc gcacccgctt cgaatatatc atcgagaaac acggaggccg cctccctgta 300
cgtatcaaag cagtaccgga aggaacagta accggcgtac gtaacgtgct gatgaccatt 360
gaaaatacag atcctaattg ttactgggtg accaacttcc tggaaacgct cctgatgcag 420
atatggtatc catgtacagt agccacgctt tccagagaga tcaaaaaaac tgttaaacaa 480
tactataacg agacggccag tgaagcggct ttcgcaggaa ttgatttcgt actgaacgac 540
ttcggtttcc gtggcgccag ctctgtagaa agtgcaggta taggcggtag cgctcacctg 600
atcaacttct ccggtagcga taccctgatc ggttccactt ttgccaaacg ttattaccag 660
gcgccggtag ctcccggtct ctctatcccc gctacagaac actctatcgt gactatgctg 720
ggtgaagaag gagaactgga aatcttccgt cacatactga atgcgttccc taccggtact 780
atcgcctgtg tatctgactc ttacaatatc ctccgtgcct gccgcgaata ctggggtact 840
gaactgaaag aacagatcct cagcagacaa ggtaccctgg tgatccgtcc cgacagcggt 900
gacgccattc agaccctact gaaagtattt gaaattctga tggaaacctt cggttatacc 960
gtcaatgaaa aaggctataa agtattacct ccacaggtga gagtgatcca gggtgatggt 1020
attagctatt cttccattcc tccgatcttc gaagctctta aacaggccgg tatcagcgct 1080
gaaaacctgg tgctgggtat gggaggagcc ttgctgcaac gtgtaaacag agatacacag 1140
gaatacgccc tgaaatgctc ttttgcacag gtgaacggta aagccatcaa cgtacagaaa 1200
aacccgctgg aactggatgc gaacggtaat acccgtgtat ccttcaagaa gtctaaatcc 1260
ggtaaacaga aactggtagt agaaaacggt atttatactt ctctacctga aaatgaagca 1320
cctgcactgg ctgaccagct ggtaactgtc ttcgaagacg gagagatcaa aaaagcatac 1380
tcttttgaac agatcagaaa gaacgcaact atttaa 1416

<210> 5 Nicotinamide phosphoribosyltransferase CP (optimized)
<211> 1416
<212> DNA
<213> Artificial Sequence

<220>
<223> Nicotinamide phosphoribosyltransferase CP (optimized)

<400> 5
atgaccaaag aaaacctgat tctgctggca gatgcataca aatatagcca ccacaaactg 60

tatattccgg gaaccgaata tatctacagc tattttgaaa gccgtggtgg caaatttaac 120

gaaaccgttt tttatggcct gcagtacttc ctgatggaat atctgcaggg tgcagttatc 180

acaaaagaaa aactggatga agcagaagca accctgctgg aagtttttgg tcgtaatgat 240

gtttttgatc gcacccgctt tgaatacatc attgaaaaac atggtggtcg tctgccggtt 300

cgtattaaag cagttccgga aggcaccgtt accggtgttc gtaatgttct gatgaccatt 360

gaaaataccg atccgaattg ttattgggtg accaattttc tggaaacact gctgatgcag 420

atttggtatc cgtgtaccgt tgcaaccctg agccgtgaaa tcaaaaaaac cgttaaacag 480

tattacaatg aaaccgcaag cgaagcagca tttgcaggta ttgattttgt gctgaacgat 540

tttggttttc gtggtgcaag cagcgttgaa agtgccggta ttggtggtag cgcacatctg 600

attaacttta gcggtagcga taccctgatt ggtagcacct ttgcaaaacg ttattatcag 660

gcaccggttg caccgggtct gagcattccg gcaacagaac attcaattgt taccatgctg 720

ggtgaagaag gtgaactgga aatttttcgc catattctga atgcatttcc gaccggcacc 780

attgcatgtg ttagcgatag ctataacatt ctgcgtgcat gtcgtgaata ttggggcacc 840

gaactgaaag agcagattct gagccgtcag ggcaccctgg ttattcgtcc ggatagcggt 900

gatgcaattc agacgctgct gaaagtgttt gaaattctga tggaaacctt tggctatacc 960

gtgaacgaaa aaggctataa agttctgcct ccgcaggttc gtgttattca aggtgatggt 1020

attagctata gcagcattcc gcctattttt gaagcactga aacaggcagg tattagcgca 1080

gaaaatctgg ttttaggtat gggtggtgca ctgctgcagc gtgttaatcg tgatacccaa 1140

gaatatgcac tgaaatgtag ctttgcacag gttaatggca aagccattaa tgtgcagaaa 1200

aatccgctgg aactggatgc aaatggtaat acccgtgtga gtttcaaaaa aagcaaaagc 1260

ggtaaacaga aactggtggt ggaaaatggt atttatacca gcctgccgga aaatgaagca 1320

ccggcactgg cagatcagct ggttaccgtt tttgaagatg gcgaaattaa gaaagcctat 1380

agctttgagc agatccgtaa aaacgcaacc atctaa 1416

<210> 6 Nicotinamide phosphoribosyltransferase CP
<211> 471
<212> PRT
<213> Chitinophaga pinensis

<400> 6

Met Thr Lys Glu Asn Leu Ile Leu Leu Ala Asp Ala Tyr Lys Tyr Ser
1 5 10 15

His His Lys Leu Tyr Ile Pro Gly Thr Glu Tyr Ile Tyr Ser Tyr Phe
20 25 30

Glu Ser Arg Gly Gly Lys Phe Asn Glu Thr Val Phe Tyr Gly Leu Gln
35 40 45

Tyr Phe Leu Met Glu Tyr Leu Gln Gly Ala Val Ile Thr Lys Glu Lys
50 55 60

Leu Asp Glu Ala Glu Ala Thr Leu Leu Glu Val Phe Gly Arg Asn Asp
65 70 75 80

Val Phe Asp Arg Thr Arg Phe Glu Tyr Ile Ile Glu Lys His Gly Gly
85 90 95

Arg Leu Pro Val Arg Ile Lys Ala Val Pro Glu Gly Thr Val Thr Gly
100 105 110

Val Arg Asn Val Leu Met Thr Ile Glu Asn Thr Asp Pro Asn Cys Tyr
115 120 125

Trp Val Thr Asn Phe Leu Glu Thr Leu Leu Met Gln Ile Trp Tyr Pro
130 135 140

Cys Thr Val Ala Thr Leu Ser Arg Glu Ile Lys Lys Thr Val Lys Gln
145 150 155 160

Tyr Tyr Asn Glu Thr Ala Ser Glu Ala Ala Phe Ala Gly Ile Asp Phe
165 170 175

Val Leu Asn Asp Phe Gly Phe Arg Gly Ala Ser Ser Val Glu Ser Ala
180 185 190

Gly Ile Gly Gly Ser Ala His Leu Ile Asn Phe Ser Gly Ser Asp Thr
195 200 205

Leu Ile Gly Ser Thr Phe Ala Lys Arg Tyr Tyr Gln Ala Pro Val Ala
210 215 220

Pro Gly Leu Ser Ile Pro Ala Thr Glu His Ser Ile Val Thr Met Leu
225 230 235 240

Gly Glu Glu Gly Glu Leu Glu Ile Phe Arg His Ile Leu Asn Ala Phe
245 250 255

Pro Thr Gly Thr Ile Ala Cys Val Ser Asp Ser Tyr Asn Ile Leu Arg
260 265 270

Ala Cys Arg Glu Tyr Trp Gly Thr Glu Leu Lys Glu Gln Ile Leu Ser
275 280 285

Arg Gln Gly Thr Leu Val Ile Arg Pro Asp Ser Gly Asp Ala Ile Gln
    290                 295                 300

Thr Leu Leu Lys Val Phe Glu Ile Leu Met Glu Thr Phe Gly Tyr Thr
305                 310                 315                 320

Val Asn Glu Lys Gly Tyr Lys Val Leu Pro Pro Gln Val Arg Val Ile
                325                 330                 335

Gln Gly Asp Gly Ile Ser Tyr Ser Ser Ile Pro Pro Ile Phe Glu Ala
            340                 345                 350

Leu Lys Gln Ala Gly Ile Ser Ala Glu Asn Leu Val Leu Gly Met Gly
        355                 360                 365

Gly Ala Leu Leu Gln Arg Val Asn Arg Asp Thr Gln Glu Tyr Ala Leu
    370                 375                 380

Lys Cys Ser Phe Ala Gln Val Asn Gly Lys Ala Ile Asn Val Gln Lys
385                 390                 395                 400

Asn Pro Leu Glu Leu Asp Ala Asn Gly Asn Thr Arg Val Ser Phe Lys
                405                 410                 415

Lys Ser Lys Ser Gly Lys Gln Lys Leu Val Val Glu Asn Gly Ile Tyr
            420                 425                 430

Thr Ser Leu Pro Glu Asn Glu Ala Pro Ala Leu Ala Asp Gln Leu Val
        435                 440                 445

Thr Val Phe Glu Asp Gly Glu Ile Lys Lys Ala Tyr Ser Phe Glu Gln
    450                 455                 460

Ile Arg Lys Asn Ala Thr Ile
465                 470

<210> 7 Niacin transporter niaP
<211> 1425
<212> DNA
<213> Burkholderia cenocepacia

<400> 7
atgcccgccg ccaccgctcc cgcctccgcc gccgcccggc tcgaacgcct gccgttctcc 60

ggctatcaca agcgcatctt cttcatcatc gcgatcgcgt tcttcttcga ttcggtcgac 120

ctcggcacga tgacgttcgt gctcggctcg attcgcaagg agttcgggct gtcgaccgcg 180

gccgccggcc tcgtcgcgag cgcgagcttc ttcgggatgg tgctcggcgc ggccgtcgcc 240

ggcctgctcg ccgaccgttt cggccgtcgg ccggtgttcc agtggagcat ggtgctgtgg 300

ggcgccgcgt cgtacctgtg ctcgaccgcg cagagcgtcg acgcgttgat cgtctatcgc 360

gtgttgctcg gcatcgggat ggggatggag tttccggtcg cgcagacgct gctgtccgaa 420

ttcgtgccga ccgagaaacg cggccgcctg atcgcgctga tggacggctt ctggccgctc 480

ggcttcatca cggccggcat cgtcgcgtat ttcgtgctgc cgcagttcgg ctggcgcacc 540

gtgttcgcgc tgctcgcgat tccggccgtg ttcgtgctcg tcgtacgccg catcgtgccg 600

gaatcgccgc gctggctcga acatgcgggc cggcacgcgg aagccgacac ggtgatgcac 660

acgatcgagg cgaaggtgat gcgctcggcc ggcgtcacga cgctgccgcc gccgtcgcgg 720

ctcgccgagc cggccgccgc acgcggtcgc ggcgcgctgc gcgagatctg gagcggcgtg 780

taccgtcgcc gcacggtgat ggtgtggctg ctgtggttct tcgcgctgct cggcttctac 840

ggcctcacgt cgtggctcgg cgcgctgctg cagcaggccg gcttcgaagt cacgaaatcg 900

gtgttctaca cggtgctgat ctcgctcggc ggcgtgccgg gcttcctgtg cgccgcgtgg 960

ctcgtcgaac gctggggccg caagccgacc tgcatcgcat cgctgatcgg cggcggtgcg 1020

atggcgtacg catacggcca gagcgcgctg tacggcggca gcacgacgct gctgatcgtc 1080

acgggcctcg cgatgcagtt cttcctgttc gggatgtggg cggcgctgta cacgtacacg 1140

cccgagctgt acggcaccgg cgcacgcgcg accggttcgg gcttcgcgtc ggcgatcggt 1200

cgcgtcggtt cgctgatcgg gccttacgtg gtcggcgtcg tgttgccggt gttcggccag 1260

ggcggcgtgt tcacgctcgg cgcgctgtcg ttcgtcgcgg cggccatcgc cgtgtggaca 1320

ctgggaatcg agacgaaggg cctcgcgctg gagcaactgg cggcaggcga cgacgcgggc 1380

ggcaacggcc ggtatccggc gacggcggcg gacaaggcgt cctga 1425

<210> 8 Niacin transporter niaP (optimized)
<211> 1425
<212> DNA
<213> Artificial Sequence

<220>
<223> Niacin transporter niaP (optimized)

<400> 8
atgcctgcag caaccgcacc ggcaagcgca gcagcacgtc tggaacgtct gccgtttagc 60

ggttatcata aacgcatctt tttcattatc gcgatcgcct ttttttcga tagcgttgat 120

ctgggcacca tgacctttgt tctgggtagc attcgtaaag aatttggtct gagcaccgca 180

gccgcaggtc tggttgcaag cgcaagcttt tttggtatgg ttctgggtgc agcagttgca 240

ggtctgctgg cagatcgttt tggtcgtcgt ccggtttttc agtggtcaat ggttctgtgg 300

ggtgcagcca gctatctgtg tagcaccgca cagagcgttg atgcactgat tgtttatcgt 360

gttctgttag gtattggtat gggtatggaa tttccggttg cacagaccct gctgagcgaa 420

```
tttgttccga ccgaaaaacg tggtcgtctg attgcactga tggatggttt ttggcctctg      480
ggttttatta ccgcaggtat tgttgcatat ttcgttctgc cgcagtttgg ttggcgtacc      540
gtttttgcac tgctggcaat tccggcagtt tttgtgctgg ttgttcgtcg tattgttccg      600
gaaagtccgc gttggctgga acatgcaggt cgtcatgccg aagcagatac cgttatgcat      660
accattgaag caaaagttat gcgtagtgcc ggtgttacaa ccctgcctcc gcctagccgt      720
ctggcagaac ctgcagcagc ccgtggtcgt ggtgcactgc gtgaaatttg gagcggtgtg      780
tatcgtcgtc gtaccgtgat ggtttggctg ctgtggtttt tcgccctgct gggcttctat      840
ggtctgacca gctggctggg tgccctgctg caacaggcag gttttgaagt taccaaaagc      900
gtgttttata ccgtcctgat tagcttaggt ggtgttccgg gttttctgtg tgcagcctgg      960
ctggttgaac gttggggtcg taaaccgacc tgtattgcaa gcctgattgg tggtggtgca     1020
atggcctatg catatggtca gagcgcactg tatggtggta gcaccacact gctgattgtt     1080
accggtctgg caatgcagtt ttttctgttt ggtatgtggg cagccctgta tacctataca     1140
ccggaactgt atggcacagg tgcacgtgcc accggtagcg gttttgccag cgcaattggt     1200
cgtgttggtt cactgattgg tccgtatgtt gttggtgttg ttctgccggt tttggtcaa      1260
ggtggtgtgt ttaccctggg tgcactgagc tttgttgcag ccgcaattgc agtttggacc     1320
ctgggtattg aaaccaaagg tctggcactg gaacagctgg cagccggtga tgatgccggt     1380
ggtaatggtc gttatccggc aacagcagca gataaagcaa gctaa                     1425
```

```
<210>  9   Niacin transporter niaP
<211>  474
<212>  PRT
<213>  Burkholderia cenocepacia

<400>  9

Met Pro Ala Ala Thr Ala Pro Ala Ser Ala Ala Ala Arg Leu Glu Arg
1               5                   10                  15


Leu Pro Phe Ser Gly Tyr His Lys Arg Ile Phe Phe Ile Ile Ala Ile
            20                  25                  30


Ala Phe Phe Phe Asp Ser Val Asp Leu Gly Thr Met Thr Phe Val Leu
        35                  40                  45


Gly Ser Ile Arg Lys Glu Phe Gly Leu Ser Thr Ala Ala Ala Gly Leu
    50                  55                  60


Val Ala Ser Ala Ser Phe Phe Gly Met Val Leu Gly Ala Ala Val Ala
65                  70                  75                  80
```

Gly Leu Leu Ala Asp Arg Phe Gly Arg Arg Pro Val Phe Gln Trp Ser
85 90 95

Met Val Leu Trp Gly Ala Ala Ser Tyr Leu Cys Ser Thr Ala Gln Ser
100 105 110

Val Asp Ala Leu Ile Val Tyr Arg Val Leu Leu Gly Ile Gly Met Gly
115 120 125

Met Glu Phe Pro Val Ala Gln Thr Leu Leu Ser Glu Phe Val Pro Thr
130 135 140

Glu Lys Arg Gly Arg Leu Ile Ala Leu Met Asp Gly Phe Trp Pro Leu
145 150 155 160

Gly Phe Ile Thr Ala Gly Ile Val Ala Tyr Phe Val Leu Pro Gln Phe
165 170 175

Gly Trp Arg Thr Val Phe Ala Leu Leu Ala Ile Pro Ala Val Phe Val
180 185 190

Leu Val Val Arg Arg Ile Val Pro Glu Ser Pro Arg Trp Leu Glu His
195 200 205

Ala Gly Arg His Ala Glu Ala Asp Thr Val Met His Thr Ile Glu Ala
210 215 220

Lys Val Met Arg Ser Ala Gly Val Thr Thr Leu Pro Pro Pro Ser Arg
225 230 235 240

Leu Ala Glu Pro Ala Ala Ala Arg Gly Arg Gly Ala Leu Arg Glu Ile
245 250 255

Trp Ser Gly Val Tyr Arg Arg Arg Thr Val Met Val Trp Leu Leu Trp
260 265 270

Phe Phe Ala Leu Leu Gly Phe Tyr Gly Leu Thr Ser Trp Leu Gly Ala
275 280 285

Leu Leu Gln Gln Ala Gly Phe Glu Val Thr Lys Ser Val Phe Tyr Thr
290 295 300

Val Leu Ile Ser Leu Gly Gly Val Pro Gly Phe Leu Cys Ala Ala Trp
305 310 315 320

Leu Val Glu Arg Trp Gly Arg Lys Pro Thr Cys Ile Ala Ser Leu Ile
325 330 335

Gly Gly Gly Ala Met Ala Tyr Ala Tyr Gly Gln Ser Ala Leu Tyr Gly
340 345 350

Gly Ser Thr Thr Leu Leu Ile Val Thr Gly Leu Ala Met Gln Phe Phe
355 360 365

Leu Phe Gly Met Trp Ala Ala Leu Tyr Thr Tyr Thr Pro Glu Leu Tyr
370 375 380

Gly Thr Gly Ala Arg Ala Thr Gly Ser Gly Phe Ala Ser Ala Ile Gly
385 390 395 400

Arg Val Gly Ser Leu Ile Gly Pro Tyr Val Val Gly Val Val Leu Pro
405 410 415

Val Phe Gly Gln Gly Gly Val Phe Thr Leu Gly Ala Leu Ser Phe Val
420 425 430

Ala Ala Ala Ile Ala Val Trp Thr Leu Gly Ile Glu Thr Lys Gly Leu
435 440 445

Ala Leu Glu Gln Leu Ala Ala Gly Asp Asp Ala Gly Gly Asn Gly Arg
450 455 460

Tyr Pro Ala Thr Ala Ala Asp Lys Ala Ser
465 470

<210> 10 Niacin transporter niaX
<211> 582
<212> DNA
<213> Streptococcus pneumoniae TIGR4

<400> 10
ttgtctggtt tattgtacca tactagtgta tatgcagtta aaaaggagat tcttgtgaat 60

acacggaaaa agacacaatt tatgacaatg acagcccttt taacggctat tgcgattttg 120

attccaattg ttatgccttt caagattgtc attccacctg cttcctatac tttggggagc 180

cacatcgcta tttttatagc catgttcttg tcgcccttga tggcagtttt tgtcatccta 240

gcctctagtt ttggattttt gatggctggc tatcccatgg ttatcgtttt tcgggctttt 300

tcccatatat cttttggtgc tttaggagct ctttacctac aaaaattccc cgatacccta 360

gataaaccaa aatcttcctg gattttcaac tttgttttgg ctgttgttca tgcccttgct 420

gaagtattgg cctgtgtcgt tttttacgca acttctggta ccaatgtaga aaatatgttt 480

tatgttctat ttgtactagt tggatttggt acaattatcc atagtatggt agactataca 540

ttagcactag ctgtctataa agtgcttcga aaacgccgtt aa 582

<210> 11 Niacin transporter niaX (optimized)
<211> 582
<212> DNA
<213> Artificial Sequence

<220>
<223> Niacin transporter niaX (optimized)

<400> 11
ttgagcggtc tgctgtatca caccagcgtt tatgcagtga aaaaagaaat tctggtgaac 60

acccgtaaaa aaacccagtt tatgaccatg accgcactgc tgaccgcaat tgccattctg 120

attccgattg ttatgccgtt caaaattgtt attccgcctg caagctatac cctgggtagc 180

catattgcaa tctttattgc aatgtttctg agtccgctga tggccgtttt tgttattctg 240

gcaagcagct ttggttttct gatggcaggt tatccgatgg ttattgtttt tcgtgcattt 300

agccacatta gctttggtgc actgggtgcc ctgtatctgc agaaatttcc ggatacactg 360

gataaaccga aaagcagctg gatctttaac tttgttctgg cagttgttca tgcactggcc 420

gaagttctgg catgtgttgt tttttatgca accagcggca ccaatgtgga aaatatgttt 480

tatgttctgt tcgtgctggt tggctttggc accattattc atagcatggt tgattataca 540

ctggccctgg cagtttataa agttctgcgt aaacgtcgct aa 582

<210> 12 Niacin transporter niaX
<211> 193
<212> PRT
<213> Streptococcus pneumoniae TIGR4

<400> 12

Leu Ser Gly Leu Leu Tyr His Thr Ser Val Tyr Ala Val Lys Lys Glu
1 5 10 15

Ile Leu Val Asn Thr Arg Lys Lys Thr Gln Phe Met Thr Met Thr Ala
20 25 30

Leu Leu Thr Ala Ile Ala Ile Leu Ile Pro Ile Val Met Pro Phe Lys
35 40 45

Ile Val Ile Pro Pro Ala Ser Tyr Thr Leu Gly Ser His Ile Ala Ile
50 55 60

Phe Ile Ala Met Phe Leu Ser Pro Leu Met Ala Val Phe Val Ile Leu
65 70 75 80

Ala Ser Ser Phe Gly Phe Leu Met Ala Gly Tyr Pro Met Val Ile Val
85 90 95

Phe Arg Ala Phe Ser His Ile Ser Phe Gly Ala Leu Gly Ala Leu Tyr

100 105 110

Leu Gln Lys Phe Pro Asp Thr Leu Asp Lys Pro Lys Ser Ser Trp Ile
115 120 125

Phe Asn Phe Val Leu Ala Val Val His Ala Leu Ala Glu Val Leu Ala
130 135 140

Cys Val Val Phe Tyr Ala Thr Ser Gly Thr Asn Val Glu Asn Met Phe
145 150 155 160

Tyr Val Leu Phe Val Leu Val Gly Phe Gly Thr Ile Ile His Ser Met
165 170 175

Val Asp Tyr Thr Leu Ala Leu Ala Val Tyr Lys Val Leu Arg Lys Arg
180 185 190

Arg

<210> 13 Nicotinamide mononucleotide transporter pnuC
<211> 651
<212> DNA
<213> Bacillus mycoides

<400> 13
atggttagaa gtccactttt tttactcatt tctagtattg tttgcatatt agttggattc 60

tatatccgat caagttatat tgaaattttc gcatcggtta tggggattat taatgtttgg 120

ttacttgcaa gggaaaaggt atcaaacttt ttatttggga tgattacagt tgcggtattt 180

ctgtatattt tcactacaca aggcttatac gcaatggcag tattagcagc cttccaattt 240

atatttaatg tgtacggttg gtattactgg attgcgcgta gtggggagga gaaggtaaaa 300

ccgacagttc gcttagattt gaaaggatgg attatttata tactttttat tttagttgct 360

tggattggtt ggggatatta tcaagtccgt tatttagaat cgacaagtcc atatttagat 420

gctttaaacg ctgtattagg attagtagct caatttatgc tgagccgaaa aatcttagaa 480

aattggcatt tatggatttt gtataacata gttagcattg tgatttatat ttcaacgggc 540

ttatacgtca tgttagtatt agctattatt aatctatttt tatgtatcga tggattgcta 600

gaatggaaga aaaaccataa agagcgagaa cgtgtaaata attatattta g 651

<210> 14 Nicotinamide mononucleotide transporter pnuC (optimized)
<211> 651
<212> DNA
<213> Artificial Sequence

<220>
<223> Nicotinamide mononucleotide transporter pnuC (optimized)

<400> 14
atggttcgta gtccgctgtt tctgctgatt agcagcattg tttgtattct ggtgggcttt 60

tatatccgca gcagctatat tgaaattttc gcaagcgtta tgggcatcat taatgtttgg 120

ctgctggcac gtgaaaaagt gagcaatttt ctgtttggta tgattaccgt tgccgtgttc 180

ctgtatatct ttaccacaca gggtctgtat gcaatggcag ttctggcagc atttcagttt 240

atctttaatg tgtatggctg gtattattgg attgcacgta gcggtgaaga aaaagttaaa 300

ccgaccgttc gtctggatct gaaaggttgg attatctata tcctgtttat tctggttgcc 360

tggattggtt ggggttatta tcaggttcgt tatctggaaa gcaccagtcc gtatctggat 420

gcactgaatg cagttttagg tctggttgca cagtttatgc tgagccgtaa aattctggaa 480

aattggcatc tgtggatcct gtataatatc gtgagcatcg tgatttatat cagcactggc 540

ctgtatgtta tgctggttct ggccattatt aacctgtttc tgtgtattga tggtctgctg 600

gaatggaaaa agaaccataa agaacgtgaa cgcgtgaaca actacatcta a 651

<210> 15 Nicotinamide mononucleotide transporter pnuC
<211> 216
<212> PRT
<213> Bacillus mycoides

<400> 15

Met Val Arg Ser Pro Leu Phe Leu Leu Ile Ser Ser Ile Ile Cys Ile
1 5 10 15

Leu Val Gly Phe Tyr Ile Arg Ser Ser Tyr Ile Glu Ile Phe Ala Ser
20 25 30

Val Met Gly Ile Ile Asn Val Trp Leu Leu Ala Arg Glu Lys Val Ser
35 40 45

Asn Phe Leu Phe Gly Met Ile Thr Val Ala Val Phe Leu Tyr Ile Phe
50 55 60

Thr Thr Gln Gly Leu Tyr Ala Met Ala Val Leu Ala Ala Phe Gln Phe
65 70 75 80

Ile Phe Asn Val Tyr Gly Trp Tyr Tyr Trp Ile Ala Arg Ser Gly Glu
85 90 95

Glu Lys Val Lys Pro Thr Val Arg Leu Asp Leu Lys Gly Trp Ile Ile
100 105 110

Tyr Ile Leu Phe Ile Leu Val Ala Trp Ile Gly Trp Gly Tyr Tyr Gln
115 120 125

Val Arg Tyr Leu Glu Ser Thr Asn Pro Tyr Leu Asp Ala Leu Asn Ala
130 135 140

Val Leu Gly Leu Val Ala Gln Phe Met Leu Ser Arg Lys Ile Leu Glu
145 150 155 160

Asn Trp His Leu Trp Ile Leu Tyr Asn Ile Val Ser Ile Val Ile Tyr
165 170 175

Ile Ser Thr Gly Leu Tyr Val Met Leu Val Leu Ala Ile Ile Asn Leu
180 185 190

Phe Leu Cys Ile Asp Gly Leu Leu Glu Trp Lys Lys Asn His Lys Glu
195 200 205

Arg Glu Arg Val Asn Asn Tyr Ile
210 215

<210> 16 Phosphoglucose isomerase pgi
<211> 1650
<212> DNA
<213> Escherichia coli

<400> 16
atgaaaaaca tcaatccaac gcagaccgct gcctggcagg cactacagaa acacttcgat 60

gaaatgaaag acgttacgat cgccgatctt tttgctaaag acggcgatcg tttttctaag 120

ttctccgcaa ccttcgacga tcagatgctg gtggattact ccaaaaaccg catcactgaa 180

gagacgctgg cgaaattaca ggatctggcg aaagagtgcg atctggcggg cgcgattaag 240

tcgatgttct ctggcgagaa gatcaaccgc actgaaaacc gcgccgtgct gcacgtagcg 300

ctgcgtaacc gtagcaatac cccgattttg gttgatggca aagacgtaat gccggaagtc 360

aacgcggtgc tggagaagat gaaaaccttc tcagaagcga ttatttccgg tgagtggaaa 420

ggttataccg gcaaagcaat cactgacgta gtgaacatcg ggatcggcgg ttctgacctc 480

ggcccataca tggtgaccga agctctgcgt ccgtacaaaa accacctgaa catgcacttt 540

gtttctaacg tcgatgggac tcacatcgcg gaagtgctga aaaaagtaaa cccggaaacc 600

acgctgttct tggtagcatc taaaaccttc accactcagg aaactatgac caacgcccat 660

agcgcgcgtg actggttcct gaaagcggca ggtgatgaaa aacacgttgc aaaacacttt 720

gcggcgcttt ccaccaatgc caaagccgtt ggcgagtttg gtattgatac tgccaacatg 780

ttcgagttct gggactgggt tggcggccgt tactctttgt ggtcagcgat tggcctgtcg 840

attgttctct ccatcggctt tgataacttc gttgaactgc tttccggcgc acacgcgatg 900

gacaagcatt tctccaccac gcctgccgag aaaaacctgc ctgtactgct ggcgctgatt 960

ggcatctggt acaacaattt ctttggtgcg gaaactgaag cgattctgcc gtatgaccag 1020

tatatgcacc gtttcgcggc gtacttccag cagggcaata tggagtccaa cggtaagtat 1080

gttgaccgta acggtaacgt tgtggattac cagactggcc cgattatctg gggtgaacca 1140

ggcactaacg gtcagcacgc gttctaccag ctgatccacc agggaaccaa aatggtaccg 1200

tgcgatttca tcgctccggc tatcacccat aacccgctct ctgatcatca ccagaaactg 1260

ctgtctaact tcttcgccca gaccgaagcg ctggcgtttg gtaaatcccg cgaagtggtt 1320

gagcaggaat atcgtgatca gggtaaagat ccggcaacgc ttgactacgt ggtgccgttc 1380

aaagtattcg aaggtaaccg cccgaccaac tccatcctgc tgcgtgaaat cactccgttc 1440

agcctgggtg cgttgattgc gctgtatgag cacaaaatct ttactcaggg cgtgatcctg 1500

aacatcttca ccttcgacca gtggggcgtg gaactgggta aacagctggc gaaccgtatt 1560

ctgccagagc tgaaagatga taaagaaatc agcagccacg atagctcgac caatggtctg 1620

attaaccgct ataaagcgtg gcgcggttaa 1650

<210> 17 Phosphoglucose isomerase pgi (optimized)
<211> 1650
<212> DNA
<213> Artificial Sequence

<220>
<223> Phosphoglucose isomerase pgi (optimized)

<400> 17
atgaagaaca ttaatccgac acagaccgca gcatggcagg cactgcagaa acattttgat 60

gaaatgaaag atgtgaccat tgcagacctg tttgcaaaag atggtgatcg ctttagcaaa 120

tttagcgcca cctttgatga tcagatgctg gttgattata gcaaaaaccg cattaccgaa 180

gaaaccctgg caaaactgca ggatctggca aaagaatgtg atctggcagg cgcaattaaa 240

agcatgttta gcggtgaaaa aatcaaccgt accgaaaatc gtgcagttct gcatgttgca 300

ctgcgtaatc gtagcaatac cccgattctg gttgatggta aagatgttat gccggaagtt 360

aatgccgttc tggaaaaaat gaaaaccttt agcgaagcca ttatcagcgg tgaatggaaa 420

ggttataccg gtaaagcaat taccgatgtg gtgaatattg gtattggtgg tagcgatctg 480

ggtccgtata tggttaccga agcactgcgt ccgtataaaa accatctgaa tatgcatttt 540

gtgagcaatg ttgatggcac ccatattgca gaagtgctga aaaaagttaa tccggaaacc 600

acactgtttc tggttgcaag caaaacattt accacacaag aaaccatgac caatgcacat 660

agcgcacgtg attggtttct gaaagcagcc ggtgatgaaa aacatgtggc aaaacacttt 720

gcagcactga gcaccaatgc aaaagcagtg ggtgaatttg gcattgatac cgccaatatg 780

tttgaattct gggattgggt tggtggtcgt tatagcctgt ggtcagcaat tggtctgagc 840

attgttctga gtattggctt tgataacttt gtggaactgc tgagcggtgc acatgcaatg 900

gataaacatt ttagcaccac accggcagaa aaaaatctgc cggttctgct ggcactgatt 960

ggtatttggt ataacaactt ttttggtgcc gaaaccgaag caattctgcc gtatgatcag 1020

tatatgcatc gttttgcagc atattttcag cagggtaata tggaaagcaa cggcaaatat 1080

gttgatcgca atggtaatgt ggtggattat cagaccggtc cgattatttg gggtgaaccg 1140

ggtacaaatg gtcagcatgc attttatcaa ctgattcatc agggtacaaa aatggtgccg 1200

tgtgatttta ttgcaccggc aattacccat aatccgctga gcgatcatca tcagaaactg 1260

ctgtcaaatt tctttgccca gaccgaagcg ctggcatttg gtaaaagccg tgaagttgtt 1320

gaacaagaat atcgcgatca gggtaaagat ccggcaacac tggattatgt tgttccgttt 1380

aaagtgtttg aaggtaatcg tccgaccaat agcattctgc tgcgtgaaat taccccgttt 1440

agcctgggtg ccctgattgc actgtatgaa cacaaaattt tcacccaggg tgtgatcctg 1500

aacattttta cctttgatca gtggggtgtt gaactgggta aacagctggc aaatcgtatt 1560

ctgccggaac tgaaagatga taaagaaatc agcagccatg atagcagtac caatggtctg 1620

attaatcgtt ataaagcctg gcgtggttaa 1650

<210> 18 Phosphoglucose isomerase pgi
<211> 549
<212> PRT
<213> Escherichia coli

<400> 18

Met Lys Asn Ile Asn Pro Thr Gln Thr Ala Ala Trp Gln Ala Leu Gln
1 5 10 15

Lys His Phe Asp Glu Met Lys Asp Val Thr Ile Ala Asp Leu Phe Ala
20 25 30

Lys Asp Gly Asp Arg Phe Ser Lys Phe Ser Ala Thr Phe Asp Asp Gln
35 40 45

Met Leu Val Asp Tyr Ser Lys Asn Arg Ile Thr Glu Glu Thr Leu Ala
50 55 60

Lys Leu Gln Asp Leu Ala Lys Glu Cys Asp Leu Ala Gly Ala Ile Lys
65 70 75 80

Ser Met Phe Ser Gly Glu Lys Ile Asn Arg Thr Glu Asn Arg Ala Val
85 90 95

Leu His Val Ala Leu Arg Asn Arg Ser Asn Thr Pro Ile Leu Val Asp
100 105 110

```
Gly Lys Asp Val Met Pro Glu Val Asn Ala Val Leu Glu Lys Met Lys
        115                 120                 125

Thr Phe Ser Glu Ala Ile Ile Ser Gly Glu Trp Lys Gly Tyr Thr Gly
    130                 135                 140

Lys Ala Ile Thr Asp Val Val Asn Ile Gly Ile Gly Gly Ser Asp Leu
145                 150                 155                 160

Gly Pro Tyr Met Val Thr Glu Ala Leu Arg Pro Tyr Lys Asn His Leu
                165                 170                 175

Asn Met His Phe Val Ser Asn Val Asp Gly Thr His Ile Ala Glu Val
            180                 185                 190

Leu Lys Lys Val Asn Pro Glu Thr Thr Leu Phe Leu Val Ala Ser Lys
        195                 200                 205

Thr Phe Thr Thr Gln Glu Thr Met Thr Asn Ala His Ser Ala Arg Asp
    210                 215                 220

Trp Phe Leu Lys Ala Ala Gly Asp Glu Lys His Val Ala Lys His Phe
225                 230                 235                 240

Ala Ala Leu Ser Thr Asn Ala Lys Ala Val Gly Glu Phe Gly Ile Asp
                245                 250                 255

Thr Ala Asn Met Phe Glu Phe Trp Asp Trp Val Gly Gly Arg Tyr Ser
            260                 265                 270

Leu Trp Ser Ala Ile Gly Leu Ser Ile Val Leu Ser Ile Gly Phe Asp
        275                 280                 285

Asn Phe Val Glu Leu Leu Ser Gly Ala His Ala Met Asp Lys His Phe
    290                 295                 300

Ser Thr Thr Pro Ala Glu Lys Asn Leu Pro Val Leu Leu Ala Leu Ile
305                 310                 315                 320

Gly Ile Trp Tyr Asn Asn Phe Phe Gly Ala Glu Thr Glu Ala Ile Leu
                325                 330                 335

Pro Tyr Asp Gln Tyr Met His Arg Phe Ala Ala Tyr Phe Gln Gln Gly
            340                 345                 350

Asn Met Glu Ser Asn Gly Lys Tyr Val Asp Arg Asn Gly Asn Val Val
        355                 360                 365
```

Asp Tyr Gln Thr Gly Pro Ile Ile Trp Gly Glu Pro Gly Thr Asn Gly
370 375 380

Gln His Ala Phe Tyr Gln Leu Ile His Gln Gly Thr Lys Met Val Pro
385 390 395 400

Cys Asp Phe Ile Ala Pro Ala Ile Thr His Asn Pro Leu Ser Asp His
405 410 415

His Gln Lys Leu Leu Ser Asn Phe Phe Ala Gln Thr Glu Ala Leu Ala
420 425 430

Phe Gly Lys Ser Arg Glu Val Val Glu Gln Glu Tyr Arg Asp Gln Gly
435 440 445

Lys Asp Pro Ala Thr Leu Asp Tyr Val Val Pro Phe Lys Val Phe Glu
450 455 460

Gly Asn Arg Pro Thr Asn Ser Ile Leu Leu Arg Glu Ile Thr Pro Phe
465 470 475 480

Ser Leu Gly Ala Leu Ile Ala Leu Tyr Glu His Lys Ile Phe Thr Gln
485 490 495

Gly Val Ile Leu Asn Ile Phe Thr Phe Asp Gln Trp Gly Val Glu Leu
500 505 510

Gly Lys Gln Leu Ala Asn Arg Ile Leu Pro Glu Leu Lys Asp Asp Lys
515 520 525

Glu Ile Ser Ser His Asp Ser Ser Thr Asn Gly Leu Ile Asn Arg Tyr
530 535 540

Lys Ala Trp Arg Gly
545

<210> 19 Glucose-6-phosphate dehydrogenase zwf
<211> 1476
<212> DNA
<213> Escherichia coli

<400> 19
atggcggtaa cgcaaacagc ccaggcctgt gacctggtca ttttcggcgc gaaaggcgac 60

cttgcgcgtc gtaaattgct gccttccctg tatcaactgg aaaaagccgg tcagctcaac 120

ccggacaccc ggattatcgg cgtagggcgt gctgactggg ataaagcggc atataccaaa 180

gttgtccgcg aggcgctcga aactttcatg aaagaaacca ttgatgaagg tttatgggac 240

```
accctgagtg cacgtctgga tttttgtaat ctcgatgtca atgacactgc tgcattcagc     300
cgtctcggcg cgatgctgga tcaaaaaaat cgtatcacca ttaactactt tgccatgccg     360
cccagcactt ttggcgcaat ttgcaaaggg cttggcgagg caaaactgaa tgctaaaccg     420
gcacgcgtag tcatggagaa accgctgggg acgtcgctgg cgacctcgca ggaaatcaat     480
gatcaggttg gcgaatactt cgaggagtgc caggtttacc gtatcgacca ctatcttggt     540
aaagaaacgg tgctgaacct gttggcgctg cgttttgcta actccctgtt tgtgaataac     600
tgggacaatc gcaccattga tcatgttgag attaccgtgg cagaagaagt ggggatcgaa     660
gggcgctggg gctattttga taaagccggt cagatgcgcg acatgatcca gaaccacctg     720
ctgcaaattc tttgcatgat tgcgatgtct ccgccgtctg acctgagcgc agacagcatc     780
cgcgatgaaa aagtgaaagt actgaagtct ctgcgccgca tcgaccgctc caacgtacgc     840
gaaaaaaccg tacgcgggca atatactgcg ggcttcgccc agggcaaaaa agtgccggga     900
tatctggaag aagagggcgc gaacaagagc agcaatacag aaactttcgt ggcgatccgc     960
gtcgacattg ataactggcg ctgggccggt gtgccattct acctgcgtac tggtaaacgt    1020
ctgccgacca aatgttctga agtcgtggtc tatttcaaaa cacctgaact gaatctgttt    1080
aaagaatcgt ggcaggatct gccgcagaat aaactgacta tccgtctgca acctgatgaa    1140
ggcgtggata tccaggtact gaataaagtt cctggccttg accacaaaca taacctgcaa    1200
atcaccaagc tggatctgag ctattcagaa acctttaatc agacgcatct ggcggatgcc    1260
tatgaacgtt tgctgctgga aaccatgcgt ggtattcagg cactgtttgt acgtcgcgac    1320
gaagtggaag aagcctggaa atgggtagac tccattactg aggcgtgggc gatggacaat    1380
gatgcgccga aaccgtatca ggccggaacc tggggacccg ttgcctcggt ggcgatgatt    1440
acccgtgatg gtcgttcctg gaatgagttt gagtaa                              1476
```

<210> 20 Glucose-6-phosphate dehydrogenase zwf (optimized)
<211> 1476
<212> DNA
<213> Artificial Sequence

<220>
<223> Glucose-6-phosphate dehydrogenase zwf (optimized)

<400> 20

```
atggcagtta cccagaccgc acaggcatgt gatctggtta tttttggtgc aaaaggtgat      60
ctggcacgtc gtaaactgct gccgagcctg tatcagctgg aaaaagcagg tcagctgaat     120
ccggatacac gtattattgg tgttggtcgt gcagattggg ataaagcagc atataccaaa     180
gttgttcgtg aagcactgga aacctttatg aaagaaacca ttgatgaagg tctgtgggat     240
accctgagcg cacgtctgga tttttgtaat ctggatgtta atgataccgc agcatttagc     300
cgtctgggtg caatgctgga tcagaaaaat cgtattacca tcaactattt tgcaatgcct     360
```

```
ccgagcacct ttggtgccat ttgtaaaggt ctgggtgaag caaaactgaa tgcaaaaccg      420
gcacgtgttg ttatggaaaa accgctgggc accagcctgg caaccagcca agaaattaat      480
gatcaggtgg gcgaatattt tgaagagtgt caggtttatc gcatcgatca ttatctgggt      540
aaagaaaccg ttctgaatct gctggcactg cgttttgcaa atagcctgtt tgtgaataac      600
tgggataatc gcaccattga tcatgtggaa attaccgttg cagaagaagt tggtattgaa      660
ggtcgttggg gctattttga taaagccggt cagatgcgtg atatgatcca gaatcatctg      720
ctgcagattc tgtgtatgat tgcaatgagc cctccgagcg atctgagcgc agatagcatt      780
cgtgatgaaa aagttaaagt gctgaaaagc ctgcgtcgta ttgatcgtag caatgtgcgt      840
gaaaaaaccg ttcgtggtca gtataccgca ggttttgcac agggtaaaaa agttccgggt      900
tatctggaag aagaaggcgc aaataaaagc agtaataccg aaacctttgt ggccattcgt      960
gtggatattg ataattggcg ttgggcaggc gttccgtttt atctgcgtac cggtaaacgt     1020
ctgccgacca aatgtagcga agttgttgtt tatttcaaaa caccggaact gaacctgttt     1080
aaagaaagct ggcaggatct gccgcagaat aaactgacca ttcgtctgca gccggatgaa     1140
ggtgttgata ttcaggttct gaataaagtt cctggcctgg atcacaaaca taacctgcag     1200
attaccaaac tggatctgag ctatagcgaa acgtttaatc agacccatct ggcagatgca     1260
tatgaacgtc tgctgctgga aaccatgcgt ggtattcagg cactgtttgt tcgccgtgat     1320
gaagttgaag aggcatggaa atgggttgat agcattaccg aagcatgggc aatggataat     1380
gatgcaccga aaccgtatca ggcaggcacc tggggtcctg ttgcaagcgt tgcaatgatt     1440
acccgtgatg gtcgtagctg gaatgaattt gaataa                               1476
```

<210> 21 Glucose-6-phosphate dehydrogenase zwf
<211> 491
<212> PRT
<213> Escherichia coli

<400> 21

```
Met Ala Val Thr Gln Thr Ala Gln Ala Cys Asp Leu Val Ile Phe Gly
1               5                   10                  15

Ala Lys Gly Asp Leu Ala Arg Arg Lys Leu Leu Pro Ser Leu Tyr Gln
            20                  25                  30

Leu Glu Lys Ala Gly Gln Leu Asn Pro Asp Thr Arg Ile Ile Gly Val
        35                  40                  45

Gly Arg Ala Asp Trp Asp Lys Ala Ala Tyr Thr Lys Val Val Arg Glu
    50                  55                  60
```

```
Ala Leu Glu Thr Phe Met Lys Glu Thr Ile Asp Glu Gly Leu Trp Asp
65                  70                  75                  80

Thr Leu Ser Ala Arg Leu Asp Phe Cys Asn Leu Asp Val Asn Asp Thr
                85                  90                  95

Ala Ala Phe Ser Arg Leu Gly Ala Met Leu Asp Gln Lys Asn Arg Ile
            100                 105                 110

Thr Ile Asn Tyr Phe Ala Met Pro Pro Ser Thr Phe Gly Ala Ile Cys
        115                 120                 125

Lys Gly Leu Gly Glu Ala Lys Leu Asn Ala Lys Pro Ala Arg Val Val
    130                 135                 140

Met Glu Lys Pro Leu Gly Thr Ser Leu Ala Thr Ser Gln Glu Ile Asn
145                 150                 155                 160

Asp Gln Val Gly Glu Tyr Phe Glu Glu Cys Gln Val Tyr Arg Ile Asp
                165                 170                 175

His Tyr Leu Gly Lys Glu Thr Val Leu Asn Leu Leu Ala Leu Arg Phe
            180                 185                 190

Ala Asn Ser Leu Phe Val Asn Asn Trp Asp Asn Arg Thr Ile Asp His
        195                 200                 205

Val Glu Ile Thr Val Ala Glu Glu Val Gly Ile Glu Gly Arg Trp Gly
    210                 215                 220

Tyr Phe Asp Lys Ala Gly Gln Met Arg Asp Met Ile Gln Asn His Leu
225                 230                 235                 240

Leu Gln Ile Leu Cys Met Ile Ala Met Ser Pro Pro Ser Asp Leu Ser
                245                 250                 255

Ala Asp Ser Ile Arg Asp Glu Lys Val Lys Val Leu Lys Ser Leu Arg
            260                 265                 270

Arg Ile Asp Arg Ser Asn Val Arg Glu Lys Thr Val Arg Gly Gln Tyr
        275                 280                 285

Thr Ala Gly Phe Ala Gln Gly Lys Lys Val Pro Gly Tyr Leu Glu Glu
    290                 295                 300

Glu Gly Ala Asn Lys Ser Ser Asn Thr Glu Thr Phe Val Ala Ile Arg
305                 310                 315                 320
```

```
Val Asp Ile Asp Asn Trp Arg Trp Ala Gly Val Pro Phe Tyr Leu Arg
                325                 330                 335

Thr Gly Lys Arg Leu Pro Thr Lys Cys Ser Glu Val Val Val Tyr Phe
            340                 345                 350

Lys Thr Pro Glu Leu Asn Leu Phe Lys Glu Ser Trp Gln Asp Leu Pro
        355                 360                 365

Gln Asn Lys Leu Thr Ile Arg Leu Gln Pro Asp Glu Gly Val Asp Ile
    370                 375                 380

Gln Val Leu Asn Lys Val Pro Gly Leu Asp His Lys His Asn Leu Gln
385                 390                 395                 400

Ile Thr Lys Leu Asp Leu Ser Tyr Ser Glu Thr Phe Asn Gln Thr His
                405                 410                 415

Leu Ala Asp Ala Tyr Glu Arg Leu Leu Leu Glu Thr Met Arg Gly Ile
            420                 425                 430

Gln Ala Leu Phe Val Arg Arg Asp Glu Val Glu Glu Ala Trp Lys Trp
        435                 440                 445

Val Asp Ser Ile Thr Glu Ala Trp Ala Met Asp Asn Asp Ala Pro Lys
    450                 455                 460

Pro Tyr Gln Ala Gly Thr Trp Gly Pro Val Ala Ser Val Ala Met Ile
465                 470                 475                 480

Thr Arg Asp Gly Arg Ser Trp Asn Glu Phe Glu
                485                 490


<210>  22   6-Phosphoglucono lactonase pgl
<211>  996
<212>  DNA
<213>  Escherichia coli

<400>  22
atgaagcaaa cagtttatat cgccagccct gagagccagc aaattcacgt ctggaatctg        60

aatcatgaag gcgcactgac gctgacacag gttgtcgatg tgccggggca ggtgcagccg       120

atggtggtca gcccggacaa acgttatctc tatgttggtg ttcgccctga gtttcgcgtc       180

ctggcgtatc gtatcgcccc ggacgatggc gcactgacct ttgccgcaga gtctgcgctg       240

ccgggtagtc cgacgcatat ttccaccgat caccaggggc agtttgtctt tgtaggttct       300

tacaatgcgg gtaacgtgag cgtaacgcgt ctggaagatg gcctgccagt gggcgtcgtc       360
```

gatgtggtcg aggggctgga cggttgccat tccgccaata tctcaccgga caaccgtacg 420

ctgtgggttc cggcattaaa gcaggatcgc atttgcctgt ttacggtcag cgatgatggt 480

catctcgtgg cgcaggaccc tgcggaagtg accaccgttg aaggggccgg cccgcgtcat 540

atggtattcc atccaaacga acaatatgcg tattgcgtca atgagttaaa cagctcagtg 600

gatgtctggg aactgaaaga tccgcacggt aatatcgaat gtgtccagac gctggatatg 660

atgccggaaa acttctccga cacccgttgg gcggctgata ttcatatcac cccggatggt 720

cgccatttat acgcctgcga ccgtaccgcc agcctgatta ccgttttcag cgtttcggaa 780

gatggcagcg tgttgagtaa agaaggcttc cagccaacgg aaacccagcc gcgcggcttc 840

aatgttgatc acagcggcaa gtatctgatt gccgccgggc aaaaatctca ccacatctcg 900

gtatacgaaa ttgttggcga gcaggggcta ctgcatgaaa aaggccgcta tgcggtcggg 960

cagggaccaa tgtgggtggt ggttaacgca cactaa 996

<210> 23 6-Phosphoglucono lactonase pgl (optimized)
<211> 996
<212> DNA
<213> Artificial Sequence

<220>
<223> 6-Phosphoglucono lactonase pgl (optimized)

<400> 23
atgaaacaga ccgtgtatat tgcaagtccg gaaagccagc agattcatgt ttggaatctg 60

aatcatgaag gtgcactgac cctgacacag gttgttgatg ttccaggtca ggttcagccg 120

atggttgtta gtccggataa acgttatctg tatgttggtg ttcgtccgga atttcgtgtt 180

ctggcatatc gtattgcacc ggatgatggt gccctgacct ttgcagcaga aagcgcactg 240

cctggtagcc cgacacatat ttcaaccgat catcagggcc agtttgtttt tgttggtagc 300

tataatgcag gtaatgttag cgttacccgt ctggaagatg gtctgccggt tggtgttgtg 360

gatgttgttg aaggtctgga tggttgtcat agcgcaaata tttcaccgga taatcgtacc 420

ctgtgggttc ctgcactgaa acaggatcgt atttgtctgt ttaccgttag tgatgatggt 480

catctggttg cacaggatcc ggcagaagtt accaccgttg aaggtgccgg tccgcgtcat 540

atggtttttc atccgaatga acagtatgcc tattgcgtga atgaactgaa tagcagcgtt 600

gatgtttggg aactgaaaga tccgcatggt aatattgaat gtgttcagac cctggatatg 660

atgccggaaa actttagtga tacccgttgg gcagcagata ttcatattac tccggatggt 720

cgtcatctgt atgcatgtga tcgtaccgca agcctgatta ccgtttttag cgttagcgaa 780

gatggtagcg ttctgagcaa agaaggtttt cagccgaccg aaacacagcc tcgtggtttt 840

aatgttgatc acagcggtaa atatctgatt gcagcaggtc agaaaagcca tcatatttcc 900

gtttatgaaa ttgtgggtga acagggtctg ctgcatgaaa aaggtcgtta tgcagttggt 960

cagggtccga tgtgggttgt tgttaatgca cattaa 996

<210> 24 6-Phosphoglucono lactonase pgl
<211> 331
<212> PRT
<213> Escherichia coli

<400> 24

Met Lys Gln Thr Val Tyr Ile Ala Ser Pro Glu Ser Gln Gln Ile His
1 5 10 15

Val Trp Asn Leu Asn His Glu Gly Ala Leu Thr Leu Thr Gln Val Val
20 25 30

Asp Val Pro Gly Gln Val Gln Pro Met Val Val Ser Pro Asp Lys Arg
35 40 45

Tyr Leu Tyr Val Gly Val Arg Pro Glu Phe Arg Val Leu Ala Tyr Arg
50 55 60

Ile Ala Pro Asp Asp Gly Ala Leu Thr Phe Ala Ala Glu Ser Ala Leu
65 70 75 80

Pro Gly Ser Pro Thr His Ile Ser Thr Asp His Gln Gly Gln Phe Val
85 90 95

Phe Val Gly Ser Tyr Asn Ala Gly Asn Val Ser Val Thr Arg Leu Glu
100 105 110

Asp Gly Leu Pro Val Gly Val Val Asp Val Val Glu Gly Leu Asp Gly
115 120 125

Cys His Ser Ala Asn Ile Ser Pro Asp Asn Arg Thr Leu Trp Val Pro
130 135 140

Ala Leu Lys Gln Asp Arg Ile Cys Leu Phe Thr Val Ser Asp Asp Gly
145 150 155 160

His Leu Val Ala Gln Asp Pro Ala Glu Val Thr Thr Val Glu Gly Ala
165 170 175

Gly Pro Arg His Met Val Phe His Pro Asn Glu Gln Tyr Ala Tyr Cys
180 185 190

Val Asn Glu Leu Asn Ser Ser Val Asp Val Trp Glu Leu Lys Asp Pro
195 200 205

```
His Gly Asn Ile Glu Cys Val Gln Thr Leu Asp Met Met Pro Glu Asn
    210                 215                 220

Phe Ser Asp Thr Arg Trp Ala Ala Asp Ile His Ile Thr Pro Asp Gly
225                 230                 235                 240

Arg His Leu Tyr Ala Cys Asp Arg Thr Ala Ser Leu Ile Thr Val Phe
                245                 250                 255

Ser Val Ser Glu Asp Gly Ser Val Leu Ser Lys Glu Gly Phe Gln Pro
            260                 265                 270

Thr Glu Thr Gln Pro Arg Gly Phe Asn Val Asp His Ser Gly Lys Tyr
        275                 280                 285

Leu Ile Ala Ala Gly Gln Lys Ser His His Ile Ser Val Tyr Glu Ile
    290                 295                 300

Val Gly Glu Gln Gly Leu Leu His Glu Lys Gly Arg Tyr Ala Val Gly
305                 310                 315                 320

Gln Gly Pro Met Trp Val Val Val Asn Ala His
                325                 330


<210>  25   6-Phosphogluconate dehydrogenase gnd
<211>  1407
<212>  DNA
<213>  Escherichia coli

<400>  25
atgtccaagc aacagatcgg cgtagtcggt atggcagtga tgggacgcaa ccttgcgctc        60

aacatcgaaa gccgtggtta taccgtctct attttcaacc gttcccgtga gaagacggaa       120

gaagtgattg ccgaaaatcc aggcaagaaa ctggttcctt actatacggt gaaagagttt       180

gtcgaatctc tggaaacgcc tcgtcgcatc ctgttaatgg tgaaagcagg tgcaggcacg       240

gatgctgcta ttgattccct caaaccatat ctcgataaag gagacatcat cattgatggt       300

ggtaacacct tcttccagga cactattcgt cgtaatcgtg agctttcagc agagggcttt       360

aacttcatcg gtaccggtgt ttctggcggt gaagaggggg cgctgaaagg tccttctatt       420

atgcctggtg gccagaaaga agcctatgaa ttggtagcac cgatcctgac caaaatcgcc       480

gccgtagctg aagacggtga accatgcgtt acctatattg gtgccgatgg cgcaggtcac       540

tatgtgaaga tggttcacaa cggtattgaa tacggcgata tgcagctgat tgctgaagcc       600

tattctctgc ttaaaggtgg cctgaacctc accaacgaag aactggcgca gacctttacc       660

gagtggaata acggtgaact gagcagttac ctgatcgaca tcaccaaaga tatcttcacc       720

aaaaaagatg aagacggtaa ctacctggtt gatgtgatcc tggatgaagc ggctaacaaa       780
```

ggtaccggta aatggaccag ccagagcgcg ctggatctcg gcgaaccgct gtcgctgatt 840

accgagtctg tgtttgcacg ttatatctct tctctgaaag atcagcgtgt tgccgcatct 900

aaagttctct ctggtccgca agcacagcca gcaggcgaca aggctgagtt catcgaaaaa 960

gttcgtcgtg cgctgtatct gggcaaaatc gtttcttacg cccagggctt ctctcagctg 1020

cgtgctgcgt ctgaagagta caactgggat ctgaactacg gcgaaatcgc gaagattttc 1080

cgtgctggct gcatcatccg tgcgcagttc ctgcagaaaa tcaccgatgc ttatgccgaa 1140

aatccacaga tcgctaacct gttgctggct ccgtacttca agcaaattgc cgatgactac 1200

cagcaggcgc tgcgtgatgt cgttgcttat gcagtacaga acggtattcc ggttccgacc 1260

ttctccgcag cggttgccta ttacgacagc taccgtgctg ctgttctgcc tgcgaacctg 1320

atccaggcac agcgtgacta ttttggtgcg catacttata agcgtattga taaagaaggt 1380

gtgttccata ccgaatggct ggattaa 1407

<210> 26 6-Phosphogluconate dehydrogenase gnd (optimized)
<211> 1407
<212> DNA
<213> Artificial Sequence

<220>
<223> 6-Phosphogluconate dehydrogenase gnd (optimized)

<400> 26
atgagcaaac agcagattgg tgttgttggt atggcagtta tgggtcgtaa tctggcactg 60

aatattgaaa gccgtggtta taccgtgagc atttttaacc gtagccgtga aaaaaccgaa 120

gaagtgattg cagaaaatcc gggtaaaaaa ctggttccgt attacaccgt taaagagttt 180

gttgaaagcc tggaaacacc gcgtcgtatt ctgctgatgg ttaaagccgg tgcaggcacc 240

gatgcagcaa ttgatagcct gaaaccgtat ctggataaag gcgatattat cattgatggt 300

ggcaacacct tttccagga taccattcgt cgtaatcgtg aactgagcgc agaaggcttt 360

aactttattg gcaccggtgt tagcggtggt gaagaaggtg cactgaaagg tccgagcatt 420

atgcctggtg gtcagaaaga agcatacgaa ctggttgcac cgattctgac caaaattgca 480

gcagttgccg aagatggtga accgtgtgtt acctatattg gtgcagatgg tgcaggtcat 540

tatgtgaaaa tggtgcataa cggtatcgag tatggtgata tgcagctgat tgcggaagca 600

tatagcctgc tgaaaggtgg tctgaatctg accaatgaag aactggcaca gacctttacc 660

gaatggaata atggtgaact gtccagctat ctgatcgata tcaccaaaga catcttcacc 720

aaaaaagatg aggatggcaa ttatctggtg gatgtgattc tggatgaagc agcaaataaa 780

ggcaccggta aatggaccag ccagagcgca ctggatctgg gtgaaccgct gagcctgatt 840

accgaaagcg tttttgcacg ttatatcagc agcctgaaag atcagcgtgt tgcagcaagc 900

aaagttctga gcggtccgca ggcacagcct gccggtgata aagcagaatt tattgaaaaa 960

gttcgccgtg cgctgtatct gggtaaaatt gttagctatg cacagggttt tagccagctg 1020

cgtgcagcca gcgaagaata caattgggat ctgaattatg gcgagatcgc caaaatcttt 1080

cgtgccggtt gtattattcg tgcacagttt ttacagaaaa tcaccgatgc ctatgccgaa 1140

aatccgcaga ttgcaaacct gctgctggca ccgtatttta agcagattgc cgatgattat 1200

cagcaggcac tgcgtgatgt tgttgcatat gccgttcaga atggtattcc ggttccgacc 1260

tttagcgcag ccgttgcata ttatgatagt tatcgtgcag ccgttctgcc tgcaaatctg 1320

attcaggccc agcgtgatta ttttggtgca catacctata aacgcatcga taaagaaggt 1380

gtgtttcata cagaatggct ggactaa 1407

<210> 27 6-Phosphogluconate dehydrogenase gnd
<211> 468
<212> PRT
<213> Escherichia coli

<400> 27

Met Ser Lys Gln Gln Ile Gly Val Val Gly Met Ala Val Met Gly Arg
1 5 10 15

Asn Leu Ala Leu Asn Ile Glu Ser Arg Gly Tyr Thr Val Ser Ile Phe
20 25 30

Asn Arg Ser Arg Glu Lys Thr Glu Glu Val Ile Ala Glu Asn Pro Gly
35 40 45

Lys Lys Leu Val Pro Tyr Tyr Thr Val Lys Glu Phe Val Glu Ser Leu
50 55 60

Glu Thr Pro Arg Arg Ile Leu Leu Met Val Lys Ala Gly Ala Gly Thr
65 70 75 80

Asp Ala Ala Ile Asp Ser Leu Lys Pro Tyr Leu Asp Lys Gly Asp Ile
85 90 95

Ile Ile Asp Gly Gly Asn Thr Phe Phe Gln Asp Thr Ile Arg Arg Asn
100 105 110

Arg Glu Leu Ser Ala Glu Gly Phe Asn Phe Ile Gly Thr Gly Val Ser
115 120 125

Gly Gly Glu Glu Gly Ala Leu Lys Gly Pro Ser Ile Met Pro Gly Gly
130 135 140

Gln Lys Glu Ala Tyr Glu Leu Val Ala Pro Ile Leu Thr Lys Ile Ala

145 150 155 160

Ala Val Ala Glu Asp Gly Glu Pro Cys Val Thr Tyr Ile Gly Ala Asp
165 170 175

Gly Ala Gly His Tyr Val Lys Met Val His Asn Gly Ile Glu Tyr Gly
180 185 190

Asp Met Gln Leu Ile Ala Glu Ala Tyr Ser Leu Leu Lys Gly Gly Leu
195 200 205

Asn Leu Thr Asn Glu Glu Leu Ala Gln Thr Phe Thr Glu Trp Asn Asn
210 215 220

Gly Glu Leu Ser Ser Tyr Leu Ile Asp Ile Thr Lys Asp Ile Phe Thr
225 230 235 240

Lys Lys Asp Glu Asp Gly Asn Tyr Leu Val Asp Val Ile Leu Asp Glu
245 250 255

Ala Ala Asn Lys Gly Thr Gly Lys Trp Thr Ser Gln Ser Ala Leu Asp
260 265 270

Leu Gly Glu Pro Leu Ser Leu Ile Thr Glu Ser Val Phe Ala Arg Tyr
275 280 285

Ile Ser Ser Leu Lys Asp Gln Arg Val Ala Ala Ser Lys Val Leu Ser
290 295 300

Gly Pro Gln Ala Gln Pro Ala Gly Asp Lys Ala Glu Phe Ile Glu Lys
305 310 315 320

Val Arg Arg Ala Leu Tyr Leu Gly Lys Ile Val Ser Tyr Ala Gln Gly
325 330 335

Phe Ser Gln Leu Arg Ala Ala Ser Glu Glu Tyr Asn Trp Asp Leu Asn
340 345 350

Tyr Gly Glu Ile Ala Lys Ile Phe Arg Ala Gly Cys Ile Ile Arg Ala
355 360 365

Gln Phe Leu Gln Lys Ile Thr Asp Ala Tyr Ala Glu Asn Pro Gln Ile
370 375 380

Ala Asn Leu Leu Leu Ala Pro Tyr Phe Lys Gln Ile Ala Asp Asp Tyr
385 390 395 400

Gln Gln Ala Leu Arg Asp Val Val Ala Tyr Ala Val Gln Asn Gly Ile
405 410 415

Pro Val Pro Thr Phe Ser Ala Ala Val Ala Tyr Tyr Asp Ser Tyr Arg
420 425 430

Ala Ala Val Leu Pro Ala Asn Leu Ile Gln Ala Gln Arg Asp Tyr Phe
435 440 445

Gly Ala His Thr Tyr Lys Arg Ile Asp Lys Glu Gly Val Phe His Thr
450 455 460

Glu Trp Leu Asp
465

<210> 28 Ribose-5-phosphate isomerase rpiA
<211> 660
<212> DNA
<213> Escherichia coli

<400> 28
atgacgcagg atgaattgaa aaaagcagta ggatgggcgg cacttcagta tgttcagccc 60

ggcaccattg ttggtgtagg tacaggttcc accgccgcac actttattga cgcgctcggt 120

acaatgaaag gccagattga aggggccgtt tccagttcag atgcttccac tgaaaaactg 180

aaaagcctcg gcattcacgt tttgatctc aacgaagtcg acagccttgg catctacgtt 240

gatggcgcag atgaaatcaa cggccacatg caaatgatca aaggcggcgg cgcggcgctg 300

acccgtgaaa aaatcattgc ttcggttgca gaaaaattta tctgtattgc agacgcttcc 360

aagcaggttg atattctggg taaattcccg ctgccagtag aagttatccc gatggcacgt 420

agtgcagtgg cgcgtcagct ggtgaaactg ggcggtcgtc cggaataccg tcagggcgtg 480

gtgaccgata atggcaacgt gatcctcgac gtccacggca tggaaatcct tgacccgata 540

gcgatggaaa acgccataaa tgcgattcct ggcgtggtga ctgttggctt gtttgctaac 600

cgtggcgcgg acgttgcgct gattggcaca cctgacggtg tcaaaaccat tgtgaaatga 660

<210> 29 Ribose-5-phosphate isomerase rpiA (optimized)
<211> 660
<212> DNA
<213> Artificial Sequence

<220>
<223> Ribose-5-phosphate isomerase rpiA (optimized)

<400> 29
atgacccagg atgaactgaa aaaagcagtt ggttgggcag cactgcagta tgttcagcct 60

ggcaccattg ttggtgttgg caccggtagc accgcagcac attttattga tgcactgggc 120

accatgaaag gtcagattga aggtgcagtt agcagcagtg atgcaagcac cgaaaaactg 180

aaaagcctgg gtattcatgt gtttgatctg aatgaagttg atagcctggg catttatgtt 240

gatggtgccg atgaaattaa tggccatatg cagatgatta aaggtggtgg tgcagcactg 300

acccgtgaaa aaatcattgc aagcgttgcc gaaaagttta tctgtattgc agatgccagc 360

aaacaggttg atattctggg taaatttccg ctgccggttg aagttattcc gatggcacgt 420

agcgcagttg cacgtcagct ggttaaactt ggtggtcgtc cggaatatcg tcagggtgtt 480

gttaccgata atggtaatgt tattctggat gtgcatggca tggaaattct ggatccgatt 540

gcaatggaaa atgccattaa tgcaattccg ggtgttgtga cagttggtct gtttgcaaat 600

cgtggtgcag atgttgcact gattggtaca ccggatggtg ttaaaaccat tgtgaaataa 660

<210> 30 Ribose-5-phosphate isomerase rpiA
<211> 219
<212> PRT
<213> Escherichia coli

<400> 30

Met Thr Gln Asp Glu Leu Lys Lys Ala Val Gly Trp Ala Ala Leu Gln
1 5 10 15

Tyr Val Gln Pro Gly Thr Ile Val Gly Val Gly Thr Gly Ser Thr Ala
20 25 30

Ala His Phe Ile Asp Ala Leu Gly Thr Met Lys Gly Gln Ile Glu Gly
35 40 45

Ala Val Ser Ser Ser Asp Ala Ser Thr Glu Lys Leu Lys Ser Leu Gly
50 55 60

Ile His Val Phe Asp Leu Asn Glu Val Asp Ser Leu Gly Ile Tyr Val
65 70 75 80

Asp Gly Ala Asp Glu Ile Asn Gly His Met Gln Met Ile Lys Gly Gly
85 90 95

Gly Ala Ala Leu Thr Arg Glu Lys Ile Ile Ala Ser Val Ala Glu Lys
100 105 110

Phe Ile Cys Ile Ala Asp Ala Ser Lys Gln Val Asp Ile Leu Gly Lys
115 120 125

Phe Pro Leu Pro Val Glu Val Ile Pro Met Ala Arg Ser Ala Val Ala
130 135 140

Arg Gln Leu Val Lys Leu Gly Gly Arg Pro Glu Tyr Arg Gln Gly Val
145 150 155 160

Val Thr Asp Asn Gly Asn Val Ile Leu Asp Val His Gly Met Glu Ile
165 170 175

Leu Asp Pro Ile Ala Met Glu Asn Ala Ile Asn Ala Ile Pro Gly Val
180 185 190

Val Thr Val Gly Leu Phe Ala Asn Arg Gly Ala Asp Val Ala Leu Ile
195 200 205

Gly Thr Pro Asp Gly Val Lys Thr Ile Val Lys
210 215

<210> 31 Ribose-5-phosphate isomerase rpiB
<211> 450
<212> DNA
<213> Escherichia coli

<400> 31
atgaaaaaga ttgcatttgg ctgtgatcat gtcggtttca ttttaaaaca tgaaatagtg 60

gcacatttag ttgagcgtgg cgttgaagtg attgataaag gaacctggtc gtcagagcgt 120

actgattatc cacattacgc cagtcaagtc gcactggctg ttgctggcgg agaggttgat 180

ggcgggattt tgatttgtgg tactggcgtc ggtatttcga tagcggcgaa caagtttgcc 240

ggaattcgcg cggtcgtctg tagcgaacct tattccgcgc aactttcgcg gcagcataac 300

gacaccaacg tgctggcttt tggttcacga gtggttggcc tcgaactggc aaaaatgatt 360

gtggatgcgt ggctgggcgc acagtacgaa ggcggtcgtc atcaacaacg cgtggaggcg 420

attacggcaa tagagcagcg gagaaattga 450

<210> 32 Ribose-5-phosphate isomerase rpiB (optimized)
<211> 450
<212> DNA
<213> Artificial Sequence

<220>
<223> Ribose-5-phosphate isomerase rpiB (optimized)

<400> 32
atgaaaaaaa tcgcctttgg ctgcgatcat gtgggcttta ttctgaaaca tgaaattgtt 60

gcccatctgg ttgaacgtgg tgttgaagtt attgataaag gcacctggtc aagcgaacgt 120

accgattatc cgcattatgc aagccaggtt gcactggcag ttgccggtgg tgaagttgat 180

ggtggtattc tgatttgtgg caccggtgtt ggtattagca ttgcagcaaa caaatttgca 240

ggtattcgtg cagttgtttg tagcgaaccg tatagcgcac agctgagccg tcagcataat 300

gataccaatg ttctggcatt tggtagccgt gttgttggtc tggaactggc aaaaatgatt 360

gttgatgcat ggctgggtgc acagtatgaa ggtggtcgtc atcagcagcg tgttgaagca 420

attaccgcaa ttgaacagcg tcgcaattaa 450

<210> 33 Ribose-5-phosphate isomerase rpiB
<211> 149
<212> PRT
<213> Escherichia coli

<400> 33

Met Lys Lys Ile Ala Phe Gly Cys Asp His Val Gly Phe Ile Leu Lys
1 5 10 15

His Glu Ile Val Ala His Leu Val Glu Arg Gly Val Glu Val Ile Asp
20 25 30

Lys Gly Thr Trp Ser Ser Glu Arg Thr Asp Tyr Pro His Tyr Ala Ser
35 40 45

Gln Val Ala Leu Ala Val Ala Gly Gly Glu Val Asp Gly Gly Ile Leu
50 55 60

Ile Cys Gly Thr Gly Val Gly Ile Ser Ile Ala Ala Asn Lys Phe Ala
65 70 75 80

Gly Ile Arg Ala Val Val Cys Ser Glu Pro Tyr Ser Ala Gln Leu Ser
85 90 95

Arg Gln His Asn Asp Thr Asn Val Leu Ala Phe Gly Ser Arg Val Val
100 105 110

Gly Leu Glu Leu Ala Lys Met Ile Val Asp Ala Trp Leu Gly Ala Gln
115 120 125

Tyr Glu Gly Gly Arg His Gln Gln Arg Val Glu Ala Ile Thr Ala Ile
130 135 140

Glu Gln Arg Arg Asn
145

<210> 34 Phosphoribosyl pyrophosphate synthetase prs
<211> 948
<212> DNA
<213> Escherichia coli

<400> 34
gtgcctgata tgaagctttt tgctggtaac gccaccccgg aactagcaca acgtattgcc 60

aaccgcctgt acacttcact cggcgacgcc gctgtaggtc gctttagcga tggcgaagtc 120

agcgtacaaa ttaatgaaaa tgtacgcggt ggtgatattt tcatcatcca gtccacttgt 180

gcccctacta acgacaacct gatggaatta gtcgttatgg ttgatgccct gcgtcgtgct 240

tccgcaggtc gtatcaccgc tgttatcccc tactttggct atgcgcgcca ggaccgtcgc 300

gtccgttccg ctcgtgtacc aatcactgcg aaagtggttg cagacttcct ctccagcgtc 360

ggtgttgacc gtgtgctgac agtggatctg cacgctgaac agattcaggg tttcttcgac 420

gttccggttg ataacgtatt tggtagcccg atcctgctgg aagacatgct gcagctgaat 480

ctggataacc caattgtggt ttctccggac atcggcggcg ttgtgcgtgc ccgcgctatc 540

gctaagctgc tgaacgatac cgatatggca atcatcgaca aacgtcgtcc gcgtgcgaac 600

gtttcacagg tgatgcatat catcggtgac gttgcaggtc gtgactgcgt actggtcgat 660

gatatgatcg acactggcgg tacgctgtgt aaagctgctg aagctctgaa agaacgtggt 720

gctaaacgtg tatttgcgta cgcgactcac ccgatcttct ctggcaacgc ggcgaacaac 780

ctgcgtaact ctgtaattga tgaagtcgtt gtctgcgata ccattccgct gagcgatgaa 840

atcaaatcac tgccgaacgt gcgtactctg accctgtcag gtatgctggc cgaagcgatt 900

cgtcgtatca gcaacgaaga atcgatctct gccatgttcg aacactaa 948

<210> 35 Phosphoribosyl pyrophosphate synthetase prs (optimized)
<211> 948
<212> DNA
<213> Artificial Sequence

<220>
<223> Phosphoribosyl pyrophosphate synthetase prs (optimized)

<400> 35
gtgccggata tgaaactgtt tgcaggtaat gcaacaccgg aactggcaca gcgtattgca 60

aatcgtctgt ataccagcct gggtgatgca gcagttggtc gttttagtga tggtgaagtt 120

agcgtgcaga ttaatgaaaa tgttcgcggt ggcgatatct ttattatcca gagcacctgt 180

gcaccgacca atgataatct gatggaactg gttgttatgg ttgatgcact gcgtcgtgca 240

agcgcaggtc gtattaccgc agttattccg tattttggtt atgcacgtca ggatcgtcgt 300

gttcgtagcg cacgtgttcc gattaccgca aaagttgttg cagattttct gagcagcgtt 360

ggtgttgatc gtgttctgac cgttgatctg catgccgagc agattcaggg ttttttttgat 420

gttccggtgg ataatgtttt tggtagcccg attctgctgg aagatatgct gcagctgaat 480

ctggataacc cgattgttgt tagtccggat attggtggtg ttgtgcgtgc acgtgccatt 540

gcaaaactgc tgaatgatac cgatatggcg attattgata aacgtcgtcc gcgtgcaaat 600

gttagccagg ttatgcatat aattggtgat gttgcaggtc gtgattgtgt tctggtggat 660

gatatgattg ataccggtgg caccctgtgt aaagcagccg aagcactgaa agaacgtggt 720

gcaaaacgtg tttttgccta tgcaacccat ccgattttta gcggtaatgc agcaaataat 780

ctgcgcaata gcgttattga tgaagttgtt gtttgtgaca ccattccgct gagtgatgaa 840

atcaaaagcc tgccgaatgt tcgtaccctg acactgagcg gtatgctggc agaagcaatt 900

cgtcgtatta gcaatgaaga aagcattagc gccatgtttg aacattaa 948

<210> 36 Phosphoribosyl pyrophosphate synthetase prs
<211> 315
<212> PRT
<213> Escherichia coli

<400> 36

Met Pro Asp Met Lys Leu Phe Ala Gly Asn Ala Thr Pro Glu Leu Ala
1 5 10 15

Gln Arg Ile Ala Asn Arg Leu Tyr Thr Ser Leu Gly Asp Ala Ala Val
20 25 30

Gly Arg Phe Ser Asp Gly Glu Val Ser Val Gln Ile Asn Glu Asn Val
35 40 45

Arg Gly Gly Asp Ile Phe Ile Ile Gln Ser Thr Cys Ala Pro Thr Asn
50 55 60

Asp Asn Leu Met Glu Leu Val Val Met Val Asp Ala Leu Arg Arg Ala
65 70 75 80

Ser Ala Gly Arg Ile Thr Ala Val Ile Pro Tyr Phe Gly Tyr Ala Arg
85 90 95

Gln Asp Arg Arg Val Arg Ser Ala Arg Val Pro Ile Thr Ala Lys Val
100 105 110

Val Ala Asp Phe Leu Ser Ser Val Gly Val Asp Arg Val Leu Thr Val
115 120 125

Asp Leu His Ala Glu Gln Ile Gln Gly Phe Phe Asp Val Pro Val Asp
130 135 140

Asn Val Phe Gly Ser Pro Ile Leu Leu Glu Asp Met Leu Gln Leu Asn
145 150 155 160

Leu Asp Asn Pro Ile Val Val Ser Pro Asp Ile Gly Gly Val Val Arg
165 170 175

Ala Arg Ala Ile Ala Lys Leu Leu Asn Asp Thr Asp Met Ala Ile Ile
180 185 190

Asp Lys Arg Arg Pro Arg Ala Asn Val Ser Gln Val Met His Ile Ile
195 200 205

Gly Asp Val Ala Gly Arg Asp Cys Val Leu Val Asp Asp Met Ile Asp
    210                 215                 220

Thr Gly Gly Thr Leu Cys Lys Ala Ala Glu Ala Leu Lys Glu Arg Gly
225                 230                 235                 240

Ala Lys Arg Val Phe Ala Tyr Ala Thr His Pro Ile Phe Ser Gly Asn
                245                 250                 255

Ala Ala Asn Asn Leu Arg Asn Ser Val Ile Asp Glu Val Val Val Cys
            260                 265                 270

Asp Thr Ile Pro Leu Ser Asp Glu Ile Lys Ser Leu Pro Asn Val Arg
        275                 280                 285

Thr Leu Thr Leu Ser Gly Met Leu Ala Glu Ala Ile Arg Arg Ile Ser
    290                 295                 300

Asn Glu Glu Ser Ile Ser Ala Met Phe Glu His
305                 310                 315

<210> 37 Nicotinamide phosphoribosyltransferase HS
<211> 1476
<212> DNA
<213> Homo sapiens

<400> 37
atgaatcctg cggcagaagc cgagttcaac atcctcctgg ccaccgactc ctacaaggtt 60

actcactata aacaatatcc acccaacaca agcaaagttt attcctactt tgaatgccgt 120

gaaaagaaga cagaaaactc caaattaagg aaggtgaaat atgaggaaac agtattttat 180

gggttgcagt acattcttaa taagtactta aaaggtaaag tagtaaccaa agagaaaatc 240

caggaagcca aagatgtcta caaagaacat ttccaagatg atgtctttaa tgaaaaggga 300

tggaactaca ttcttgagaa gtatgatggg catcttccaa tagaaataaa agctgttcct 360

gagggctttg tcattcccag aggaaatgtt ctcttcacgg tggaaaacac agatccagag 420

tgttactggc ttacaaattg gattgagact attcttgttc agtcctggta tccaatcaca 480

gtggccacaa attctagaga gcagaagaaa atattggcca aatatttgtt agaaacttct 540

ggtaacttag atggtctgga atacaagtta catgattttg gctacagagg agtctcttcc 600

caagagactg ctggcatagg agcatctgct cacttggtta acttcaaagg aacagataca 660

gtagcaggac ttgctctaat taaaaaatat tatggaacga aagatcctgt tccaggctat 720

tctgttccag cagcagaaca cagtaccata acagcttggg ggaaagacca tgaaaaagat 780

gcttttgaac atattgtaac acagttttca tcagtgcctg tatctgtggt cagcgatagc 840

```
tatgacattt ataatgcgtg tgagaaaata tggggtgaag atctaagaca tttaatagta      900

tcaagaagta cacaggcacc actaataatc agacctgatt ctggaaaccc tcttgacact      960

gtgttaaagg ttttggagat tttaggtaag aagtttcctg ttactgagaa ctcaaagggt     1020

tacaagttgc tgccacctta tcttagagtt attcaagggg atggagtaga tattaatacc     1080

ttacaagaga ttgtagaagg catgaaacaa aaaatgtgga gtattgaaaa tattgccttc     1140

ggttctggtg gaggtttgct acagaagttg acaagagatc tcttgaattg ttccttcaag     1200

tgtagctatg ttgtaactaa tggccttggg attaacgtct tcaaggaccc agttgctgat     1260

cccaacaaaa ggtccaaaaa gggccgatta tctttacata ggacgccagc agggaatttt     1320

gttacactgg aggaaggaaa aggagacctt gaggaatatg gtcaggatct tctccatact     1380

gtcttcaaga atggcaaggt gacaaaaagc tattcatttg atgaaataag aaaaaatgca     1440

cagctgaata ttgaactgga agcagcacat cattag                               1476
```

```
<210>  38   Nicotinamide phosphoribosyltransferase HS (optimized)
<211>  1476
<212>  DNA
<213>  Artificial Sequence

<220>
<223>  Nicotinamide phosphoribosyltransferase HS (optimized)

<400>  38
atgaatccgg cagcagaagc cgaatttaac attctgctgg caaccgatag ctataaagtg       60

acccattata aacagtatcc gcctaatacc agcaaagtgt atagctattt tgagtgccgt      120

gagaaaaaaa ccgaaaacag caaactgcgc aaagtgaaat atgaagaaac cgtgttttat      180

ggcctgcagt acatcctgaa caaatacctg aaaggtaaag tggtgaccaa agagaaaatt      240

caagaggcca aagatgtgta taaagaacac tttcaggatg acgtgttcaa cgaaaaaggc      300

tggaactata tcctggaaaa atatgatggt catctgccga ttgaaattaa agcagttccg      360

gaaggttttg ttattccgcg tggtaatgtt ctgtttaccg ttgaaaatac cgatccggaa      420

tgttattggc tgaccaattg gattgaaacc attctggttc agagctggta tccgattacc      480

gttgcaacca atagccgtga acagaaaaaa atcctggcca aatatctgct ggaaaccagc      540

ggtaatctgg atggtctgga atacaaactg catgattttg gttatcgtgg tgttagcagc      600

caagaaaccg caggtattgg tgcaagcgca catctggtta actttaaagg caccgatacc      660

gtggcaggtc tggcactgat taaaaagtat tatggcacca aagatccggt tccgggttat      720

agcgttccgg cagccgaaca ttcaaccatt accgcatggg gtaaagatca tgaaaaagat      780

gcctttgaac atatcgtgac ccagtttagc agcgtgccgg ttagcgttgt tagcgatagt      840

tatgatatct ataatgcctg cgagaagatc tggggtgaag atctgcgtca tctgattgtt      900

agccgtagca cccaggcacc gctgattatt cgtccggata gtggtaatcc gctggatacc      960
```

gttctgaaag ttctggaaat tctgggcaaa aaattcccgg ttacggaaaa tagcaaaggc 1020

tataaactgc tgcctccgta tctgcgtgtt attcaaggtg atggtgtgga tattaacacc 1080

ctgcaagaaa ttgtggaagg catgaaacag aaaatgtggt ccattgaaaa tatcgccttt 1140

ggtagcggtg gtggtctgct gcagaaactg acccgtgatc tgctgaattg tagctttaaa 1200

tgcagctatg ttgtgaccaa tggtctgggc attaacgttt ttaaagatcc tgttgccgat 1260

ccgaataaac gcagcaaaaa aggtcgtctg agcctgcatc gtacaccggc aggtaatttt 1320

gttaccctgg aagaaggtaa aggcgatctg gaagaatatg gtcaggatct gctgcatacc 1380

gttttcaaaa atggcaaagt gaccaaaagc tacagctttg atgaaattcg taaaaacgcc 1440

cagctgaaca ttgaactgga agcagcacat cattaa 1476

<210> 39 Nicotinamide phosphoribosyltransferase HS
<211> 491
<212> PRT
<213> Homo sapiens

<400> 39

Met Asn Pro Ala Ala Glu Ala Glu Phe Asn Ile Leu Leu Ala Thr Asp
1 5 10 15

Ser Tyr Lys Val Thr His Tyr Lys Gln Tyr Pro Pro Asn Thr Ser Lys
20 25 30

Val Tyr Ser Tyr Phe Glu Cys Arg Glu Lys Lys Thr Glu Asn Ser Lys
35 40 45

Leu Arg Lys Val Lys Tyr Glu Glu Thr Val Phe Tyr Gly Leu Gln Tyr
50 55 60

Ile Leu Asn Lys Tyr Leu Lys Gly Lys Val Val Thr Lys Glu Lys Ile
65 70 75 80

Gln Glu Ala Lys Asp Val Tyr Lys Glu His Phe Gln Asp Asp Val Phe
85 90 95

Asn Glu Lys Gly Trp Asn Tyr Ile Leu Glu Lys Tyr Asp Gly His Leu
100 105 110

Pro Ile Glu Ile Lys Ala Val Pro Glu Gly Phe Val Ile Pro Arg Gly
115 120 125

Asn Val Leu Phe Thr Val Glu Asn Thr Asp Pro Glu Cys Tyr Trp Leu
130 135 140

Thr Asn Trp Ile Glu Thr Ile Leu Val Gln Ser Trp Tyr Pro Ile Thr

145 150 155 160

Val Ala Thr Asn Ser Arg Glu Gln Lys Lys Ile Leu Ala Lys Tyr Leu
165 170 175

Leu Glu Thr Ser Gly Asn Leu Asp Gly Leu Glu Tyr Lys Leu His Asp
180 185 190

Phe Gly Tyr Arg Gly Val Ser Ser Gln Glu Thr Ala Gly Ile Gly Ala
195 200 205

Ser Ala His Leu Val Asn Phe Lys Gly Thr Asp Thr Val Ala Gly Leu
210 215 220

Ala Leu Ile Lys Lys Tyr Tyr Gly Thr Lys Asp Pro Val Pro Gly Tyr
225 230 235 240

Ser Val Pro Ala Ala Glu His Ser Thr Ile Thr Ala Trp Gly Lys Asp
245 250 255

His Glu Lys Asp Ala Phe Glu His Ile Val Thr Gln Phe Ser Ser Val
260 265 270

Pro Val Ser Val Val Ser Asp Ser Tyr Asp Ile Tyr Asn Ala Cys Glu
275 280 285

Lys Ile Trp Gly Glu Asp Leu Arg His Leu Ile Val Ser Arg Ser Thr
290 295 300

Gln Ala Pro Leu Ile Ile Arg Pro Asp Ser Gly Asn Pro Leu Asp Thr
305 310 315 320

Val Leu Lys Val Leu Glu Ile Leu Gly Lys Lys Phe Pro Val Thr Glu
325 330 335

Asn Ser Lys Gly Tyr Lys Leu Leu Pro Pro Tyr Leu Arg Val Ile Gln
340 345 350

Gly Asp Gly Val Asp Ile Asn Thr Leu Gln Glu Ile Val Glu Gly Met
355 360 365

Lys Gln Lys Met Trp Ser Ile Glu Asn Ile Ala Phe Gly Ser Gly Gly
370 375 380

Gly Leu Leu Gln Lys Leu Thr Arg Asp Leu Leu Asn Cys Ser Phe Lys
385 390 395 400

```
Cys Ser Tyr Val Val Thr Asn Gly Leu Gly Ile Asn Val Phe Lys Asp
                405                 410                 415

Pro Val Ala Asp Pro Asn Lys Arg Ser Lys Lys Gly Arg Leu Ser Leu
            420                 425                 430

His Arg Thr Pro Ala Gly Asn Phe Val Thr Leu Glu Glu Gly Lys Gly
        435                 440                 445

Asp Leu Glu Glu Tyr Gly Gln Asp Leu Leu His Thr Val Phe Lys Asn
    450                 455                 460

Gly Lys Val Thr Lys Ser Tyr Ser Phe Asp Glu Ile Arg Lys Asn Ala
465                 470                 475                 480

Gln Leu Asn Ile Glu Leu Glu Ala Ala His His
                485                 490

<210>  40   Primer for NAMPT CP (forward)
<211>  48
<212>  DNA
<213>  Artificial Sequence

<220>
<223>  Primer for NAMPT CP (forward)

<400>  40
aggagatata ccatgaccaa agaaaacctg attctgctgg cagatgca                    48


<210>  41   Primer for NAMPT CP (reverse)
<211>  48
<212>  DNA
<213>  Artificial Sequence

<220>
<223>  Primer for NAMPT CP (reverse)

<400>  41
gctcgaattc ggatcttaga tggttgcgtt tttacggatc tgctcaaa                    48


<210>  42   Primer for NAMPT SSC (forward)
<211>  48
<212>  DNA
<213>  Artificial Sequence

<220>
<223>  Primer for NAMPT SSC (forward)

<400>  42
aggagatata ccatgaagaa tctgattctg gccaccgata gctataaa                    48


<210>  43   Primer for NAMPT SSC (reverse)
<211>  48
<212>  DNA
<213>  Artificial Sequence
```

<220>
<223> Primer for NAMPT SSC (reverse)

<400> 43
gctcgaattc ggatcttaac gaccttcgct acgtttacga actgcatc 48

<210> 44 Primer for NAMPT HS (forward)
<211> 48
<212> DNA
<213> Artificial Sequence

<220>
<223> Primer for NAMPT HS (forward)

<400> 44
aggagatata ccatgaatcc ggcagcagaa gccgaattta acattctg 48

<210> 45 Primer for NAMPT SSC (reverse)
<211> 48
<212> DNA
<213> Artificial Sequence

<220>
<223> Primer for NAMPT HS (reverse)

<400> 45
gctcgaattc ggatcttaat gatgtgctgc ttccagttca atgttcag 48

<210> 46 Primer for pgi to pCDF (forward)
<211> 70
<212> DNA
<213> Artificial Sequence

<220>
<223> Primer for pgi to pCDF (forward)

<400> 46
cgtgatggtc gtagctggaa tgaatttgaa taaaaggaga tataccatga agaacattaa 60

tccgacacag 70

<210> 47 Primer for pgi to pCDF (reverse)
<211> 70
<212> DNA
<213> Artificial Sequence

<220>
<223> Primer for pgi to pCDF (reverse)

<400> 47
acttaagcat tatgcggccg caagcttgtc gacctgcagg cgcgccgtta accacgccag 60

gctttataac 70

<210> 48 Primer for zwf to pCDF (forward)
<211> 70
<212> DNA
<213> Artificial Sequence

<220>
<223> Primer for zwf to pCDF (forward)

<400> 48
gtcagggtcc gatgtgggtt gttgttaatg cacattaaaa ggagatatac catggcagtt 60

acccagaccg 70

<210> 49 Primer for zwf to pCDF (reverse)
<211> 24
<212> DNA
<213> Artificial Sequence

<220>
<223> Primer for zwf to pCDF (reverse)

<400> 49
ttattcaaat tcattccagc tacg 24

<210> 50 Primer for pgl to pCDF (forward)
<211> 70
<212> DNA
<213> Artificial Sequence

<220>
<223> Primer for pgl to pCDF (forward)

<400> 50
agaaggtgtg tttcatacag aatggctgga ctaaaaggag atataccatg aaacagaccg 60

tgtatattgc 70

<210> 51 Primer for pgl to pCDF (reverse)
<211> 23
<212> DNA
<213> Artificial Sequence

<220>
<223> Primer for pgl to pCDF (reverse)

<400> 51
ttaatgtgca ttaacaacaa ccc 23

<210> 52 Primer for gnd to pCDF (forward)
<211> 70
<212> DNA
<213> Artificial Sequence

<220>
<223> Primer for gnd to pCDF (forward)

<400> 52
tggtacaccg gatggtgtta aaaccattgt gaaataaaag gagatatacc atgagcaaac 60

agcagattgg 70

<210> 53 Primer for gnd to pCDF (reverse)

<211> 70
<212> DNA
<213> Artificial Sequence

<220>
<223> Primer for gnd to pCDF (reverse)

<400> 53
cattatgcgg ccgcaagctt gtcgacctgc aggcgcgccg agctcttagt ccagccattc 60

tgtatgaaac 70

<210> 54 Primer for rpiA to pCDF (forward)
<211> 70
<212> DNA
<213> Artificial Sequence

<220>
<223> Primer for rpiA to pCDF (forward)

<400> 54
caattaccgc aattgaacag cgtcgcaatt aaaaggagat ataccatgac ccaggatgaa 60

ctgaaaaaag 70

<210> 55 Primer for rpiA to pCDF (reverse)
<211> 27
<212> DNA
<213> Artificial Sequence

<220>
<223> Primer for rpiA to pCDF (reverse)

<400> 55
ttatttcaca atggttttaa caccatc 27

<210> 56 Primer for rpiB to pCDF (forward)
<211> 70
<212> DNA
<213> Artificial Sequence

<220>
<223> Primer for rpiB to pCDF (forward)

<400> 56
aatgaagaaa gcattagcgc catgtttgaa cattaaaagg agatatacca tgaaaaaaat 60

cgcctttggc 70

<210> 57 Primer for rpiB to pCDF (reverse)
<211> 18
<212> DNA
<213> Artificial Sequence

<220>
<223> Primer for rpiB to pCDF (reverse)

<400> 57
ttaattgcga cgctgttc 18

<210> 58 Primer for prs to pCDF (forward)
<211> 70
<212> DNA
<213> Artificial Sequence

<220>
<223> Primer for prs to pCDF (forward)

<400> 58
attcccctgt agaaataatt ttgtttaact ttaataagga gatataccgt gccggatatg 60

aaactgtttg 70

<210> 59 Primer for prs to pCDF (reverse)
<211> 20
<212> DNA
<213> Artificial Sequence

<220>
<223> Primer for prs to pCDF (reverse)

<400> 59
ttaatgttca aacatggcgc 20

<210> 60 Primer for pgi to pACYC (forward)
<211> 70
<212> DNA
<213> Artificial Sequence

<220>
<223> Primer for pgi to pACYC (forward)

<400> 60
tcccctgtag aaataatttt gtttaacttt aataaggaga tataccatga agaacattaa 60

tccgacacag 70

<210> 61 Primer for pgi to pACYC (reverse)
<211> 23
<212> DNA
<213> Artificial Sequence

<220>
<223> Primer for pgi to pACYC (reverse)

<400> 61
ttaaccacgc caggctttat aac 23

<210> 62 Primer for zwf to pACYC (forward)
<211> 70
<212> DNA
<213> Artificial Sequence

<220>
<223> Primer for zwf to pACYC (forward)

<400> 62

atggtctgat taatcgttat aaagcctggc gtggttaaaa ggagatatac catggcagtt 60

acccagaccg 70

<210> 63 Primer for zwf to pACYC (reverse)
<211> 24
<212> DNA
<213> Artificial Sequence

<220>
<223> Primer for zwf to pACYC (reverse)

<400> 63
ttattcaaat tcattccagc tacg 24

<210> 64 Primer for pgl to pACYC (forward)
<211> 70
<212> DNA
<213> Artificial Sequence

<220>
<223> Primer for pgl to pACYC (forward)

<400> 64
ccgtgatggt cgtagctgga atgaatttga ataaaaggag atataccatg aaacagaccg 60

tgtatattgc 70

<210> 65 Primer for pgl to pACYC (reverse)
<211> 23
<212> DNA
<213> Artificial Sequence

<220>
<223> Primer for pgl to pACYC (reverse)

<400> 65
ttaatgtgca ttaacaacaa ccc 23

<210> 66 Primer for gnd to pACYC (forward)
<211> 70
<212> DNA
<213> Artificial Sequence

<220>
<223> Primer for gnd to pACYC (forward)

<400> 66
tcagggtccg atgtgggttg ttgttaatgc acattaaaag gagatatacc atgagcaaac 60

agcagattgg 70

<210> 67 Primer for gnd to pACYC (reverse)
<211> 70
<212> DNA
<213> Artificial Sequence

<220>

<223> Primer for gnd to pACYC (reverse)

<400> 67
cattatgcgg ccgcaagctt gtcgacctgc aggcgcgccg agctcttagt ccagccattc 60

tgtatgaaac 70

<210> 68 Primer for rpiA to pACYC (forward)
<211> 70
<212> DNA
<213> Artificial Sequence

<220>
<223> Primer for rpiA to pACYC (forward)

<400> 68
aaggtgtgtt tcatacagaa tggctggact aaaaggagat ataccatgac ccaggatgaa 60

ctgaaaaaag 70

<210> 69 Primer for rpiA to pACYC (reverse)
<211> 27
<212> DNA
<213> Artificial Sequence

<220>
<223> Primer for rpiA to pACYC (reverse)

<400> 69
ttatttcaca atggttttaa caccatc 27

<210> 70 Primer for rpiB to pACYC (forward)
<211> 70
<212> DNA
<213> Artificial Sequence

<220>
<223> Primer for rpiB to pACYC (forward)

<400> 70
ggtacaccgg atggtgttaa aaccattgtg aaataaaagg agatatacca tgaaaaaaat 60

cgcctttggc 70

<210> 71 Primer for rpiB to pACYC (reverse)
<211> 18
<212> DNA
<213> Artificial Sequence

<220>
<223> Primer for rpiB to pACYC (reverse)

<400> 71
ttaattgcga cgctgttc 18

<210> 72 Primer for prs to pACYC (forward)
<211> 70
<212> DNA

<213> Artificial Sequence

<220>
<223> Primer for prs to pACYC (forward)

<400> 72
aagcaattac cgcaattgaa cagcgtcgca attaaaagga gatataccgt gccggatatg 60

aaactgtttg 70

<210> 73 Primer for prs to pACYC (reverse)
<211> 70
<212> DNA
<213> Artificial Sequence

<220>
<223> Primer for prs to pACYC (reverse)

<400> 73
tcgacttaag cattatgcgg ccgcaagctt gtcgacctgc aggcgcgccg ttaatgttca 60

aacatggcgc 70

<210> 74 Primer for niaP (forward)
<211> 32
<212> DNA
<213> Artificial Sequence

<220>
<223> Primer for niaP (forward)

<400> 74
aggagatata ccatgcctgc agcaaccgca cc 32

<210> 75 Primer for niaP (reverse)
<211> 50
<212> DNA
<213> Artificial Sequence

<220>
<223> Primer for niaP (reverse)

<400> 75
gctcgaattc ggatcttagc ttgctttatc tgctgctgtt gccggataac 50

<210> 76 Primer for niaX (forward)
<211> 49
<212> DNA
<213> Artificial Sequence

<220>
<223> Primer for niaX (forward)

<400> 76
aggagatata ccttgagcgg tctgctgtat cacaccagcg tttatgcag 49

<210> 77 Primer for niaX (reverse)
<211> 49

<212>   DNA
<213>   Artificial Sequence

<220>
<223>   Primer for niaX (reverse)

<400>   77
gctcgaattc ggatcttagc gacgtttacg cagaacttta taaactgcc   49


<210>   78   Primer for pnuC (forward)
<211>   48
<212>   DNA
<213>   Artificial Sequence

<220>
<223>   Primer for pnuC (forward)

<400>   78
aggagatata ccatggttcg tagtccgctg tttctgctga ttagcagc   48


<210>   79   Primer for pnuC (reverse)
<211>   51
<212>   DNA
<213>   Artificial Sequence

<220>
<223>   Primer for pnuC (reverse)

<400>   79
gctcgaattc ggatcttaga tgtagttgtt cacgcgttca cgttctttat g   51


<210>   80  Primer part2 for pnuC BM (forward)
<211>   66
<212>   DNA
<213>   Artificial Sequence

<220>
<223>   Primer part2 for pnuC BM (forward)

<400>   80
tattagttaa gtataagaag gagatataca atggttcgta gtccgctgtt tctgctgatt   60

agcagc   66


<210>   81  Primer part2 for pnuC BM (reverse)
<211>   66
<212>   DNA
<213>   Artificial Sequence

<220>
<223>   Primer part2 for pnuC BM (reverse)

<400>   81
atgctagtta ttgctcagcg gtggcagcag ttagatgtag ttgttcacgc gttcacgttc   60

tttatg   66


<210>   82  Primer part2 for niaP BC (forward)

```
<211>  50
<212>  DNA
<213>  Artificial Sequence

<220>
<223>  Primer part2 for niaP BC (forward)

<400>  82
tattagttaa gtataagaag gagatataca atgcctgcag caaccgcacc              50


<210>  83  Primer part2 for niaP BC (reverse)
<211>  65
<212>  DNA
<213>  Artificial Sequence

<220>
<223>  Primer part2 for niaP BC (reverse)

<400>  83
atgctagtta ttgctcagcg gtggcagcag ttagcttgct ttatctgctg ctgttgccgg   60

ataac                                                               65
```